(19) Europäisches Patentamt
European Patent Office
Office européen des brevets

(11) Veröffentlichungsnummer : **0 600 833 A1**

# (12) EUROPÄISCHE PATENTANMELDUNG

(21) Anmeldenummer : **93810803.2**

(22) Anmeldetag : **18.11.93**

(51) Int. Cl.[5] : **C07D 513/04,** A01N 43/90, // (C07D513/04, 285:00, 231:00)

(30) Priorität : **27.11.92 CH 3653/92**

(43) Veröffentlichungstag der Anmeldung : **08.06.94 Patentblatt 94/23**

(84) Benannte Vertragsstaaten : **AT BE CH DE DK ES FR GB GR IE IT LI LU MC NL PT SE**

(71) Anmelder : **CIBA-GEIGY AG**
**Klybeckstrasse 141**
**CH-4002 Basel (CH)**

(72) Erfinder : **Brunner, Hans-Georg, Dr.**
**Wannenstrasse 14**
**CH-4415 Lausen (CH)**
Erfinder : **Moser, Hans, Dr.**
**Hauptstrasse 67B**
**CH-4312 Magden (CH)**
Erfinder : **Pissiotas, Georg, Dr.**
**Am Sonnenrain 71**
**D-79539 Lörrach (DE)**

(54) **Herbizide Thiadiazabicyclooktane.**

(57) Thiadiazabicyclooktane der Formel I

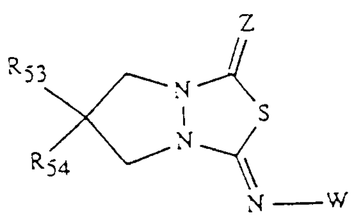

(I),

worin

| | |
|---|---|
| Z | Sauerstoff oder Schwefel ; |
| $R_{53}$ | Wasserstoff oder $C_1$-$C_6$-Alkyl ; |
| $R_{54}$ | $C_1$-$C_6$-Alkyl, $C_1$-$C_6$-Alkoxy, $C_3$-$C_6$-Alkenyloxy, $C_3$-$C_6$-Alkinyloxy, Hydroxy oder $C_1$-$C_6$-Haloalkoxy ist ; oder |
| $R_{53}$ und $R_{54}$ | zusammen mit dem C-Atom, an das sie gebunden sind, einen unsubstituierten 3-, 4-, 5- oder 6-gliedrigen gesättigten Ring bilden ; |
| W | eine Gruppe der Formel $W_1$ bis $W_{10}$ |

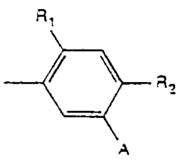

($W_1$);

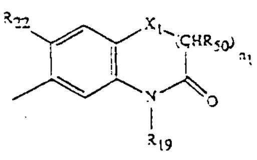

($W_2$);

EP 0 600 833 A1

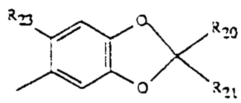 (W₃);

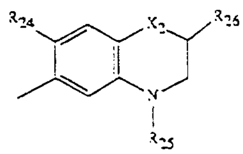 (W₄);

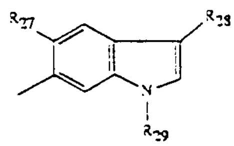 (W₅);

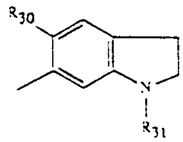 (W₆);

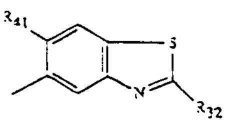 (W₇);

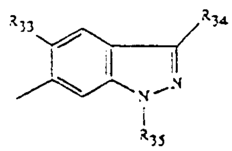 (W₈);

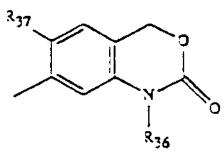 (W₉); oder

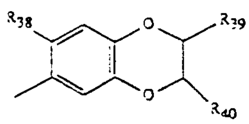 (W₁₀)

bedeutet ; und ihre Salze und Stereoisomere haben gute selektive pre- und post-emergente herbizide Eigenschaften.

Die vorliegende Erfindung betrifft neue, herbizid wirksame Thiadiazabicyclooktane, Verfahren zu ihrer Herstellung, Mittel, die diese Verbindungen als Wirkstoffe enthalten, sowie ihre Verwendung zum Bekämpfen von Unkräutern, vor allem selektiv in Nutzpflanzenkulturen wie z.B. Getreide, Mais, Soja, Raps, Reis und Baumwolle.

Thiadiazabicycloderivate mit herbizider Wirkung sind bereits bekannt. Derartige Verbindungen werden beispielsweise in EP-A-0 238 711, EP-A-0 304 920, US-A-4 885 023, US-A-4 684 397 und US-A-4 801 408 offenbart.

Es wurden nun neue Thiadiazabicyclooktane mit selektiver herbizider Wirkung gefunden.

Die erfindungsgemäßen Thiadiazabicyclooktane entsprechen der Formel I

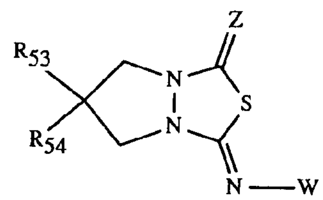

(I),

worin

| | |
|---|---|
| Z | Sauerstoff oder Schwefel; |
| $R_{53}$ | Wasserstoff oder $C_1$-$C_6$-Alkyl; |
| $R_{54}$ | $C_1$-$C_6$-Alkyl, $C_1$-$C_6$-Alkoxy, $C_3$-$C_6$-Alkenyloxy, $C_3$-$C_6$-Alkinyloxy, Hydroxy oder $C_1$-$C_6$-Haloalkoxy ist; oder |
| $R_{53}$ und $R_{54}$ | zusammen mit dem C-Atom, an das sie gebunden sind, einen unsubstituierten 3-, 4-, 5- oder 6-gliedrigen gesättigten Ring bilden; |
| W | eine Gruppe der Formel $W_1$ bis $W_{10}$ |

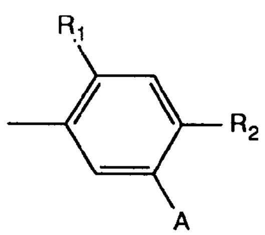

($W_1$);

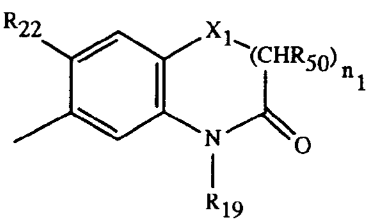

($W_2$);

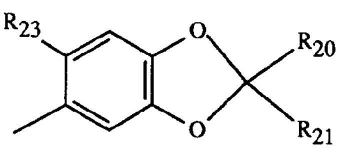

($W_3$);

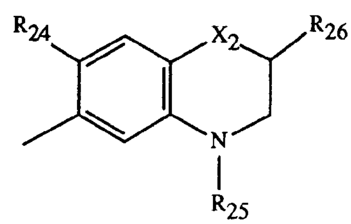

($W_4$);

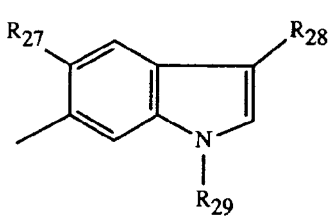

($W_5$);

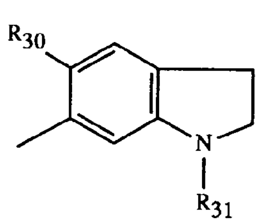

($W_6$);

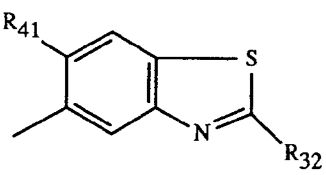 ($W_7$); 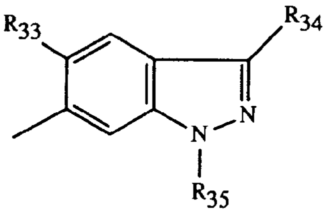 ($W_8$);

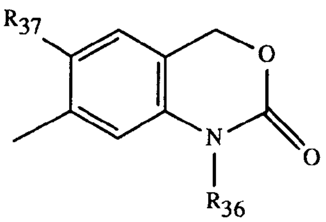 ($W_9$); oder 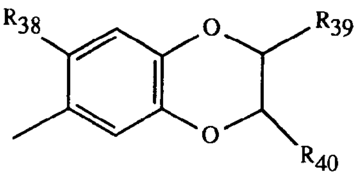 ($W_{10}$)

bedeutet;

$R_1$, $R_{22}$, $R_{23}$, $R_{24}$, $R_{27}$, $R_{30}$, $R_{33}$, $R_{37}$, $R_{38}$ und $R_{41}$ unabhängig voneinander Wasserstoff oder Halogen sind;

$R_2$ Wasserstoff, Cyano, Nitro, Halogen, $C_1$-$C_4$-Alkyl, $C_1$-$C_4$-Haloalkoxy oder $C_1$-$C_4$-Haloalkyl;

A Wasserstoff, Cyano, Nitro, $-COR_3$, $-X_3R_4$,

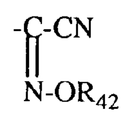 ,

$-COR_8$,

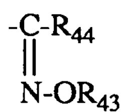 , 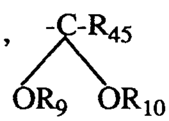 , 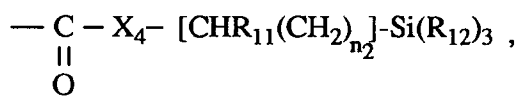 ,

$-N(R_{13})-SO_2-R_{14}$,

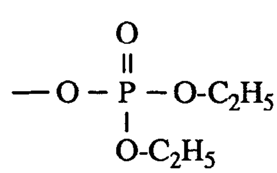 oder 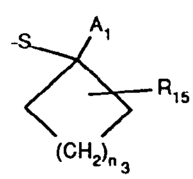 ;

$A_1$ Cyano oder $-COR_{16}$;

$R_3$ Halogen, $-X_4-R_5$, Amino, $C_1$-$C_4$-Alkylamino, Di-$C_1$-$C_4$-alkylamino, $C_2$-$C_4$-Haloalkylamino, Di-$C_2$-$C_4$-haloalkylamino, $C_1$-$C_4$-Alkoxyalkylamino, Di-$C_1$-$C_4$-alkoxyalkylamino, $C_3$- oder $C_4$-Alkenylamino, Diallylamino, -N-Pyrrolidino, -N-Piperidino, -N-Morpholino, -N-Thiomorpholino, -N-Piperazino, $-O-N=C(CH_3)-CH_3$ oder $-O-CH_2-CH_2-O-N=C(CH_3)-CH_3$;

$R_4$, $R_{42}$ und $R_{43}$ unabhängig voneinander Wasserstoff, $C_1$-$C_{10}$-Alkyl, $C_1$-$C_4$-Alkoxy-$C_1$-$C_4$-alkyl, $C_1$-$C_4$-Alkylthio-$C_1$-$C_4$-alkyl, Di-$C_1$-$C_4$-alkylamino-$C_1$-$C_4$-alkyl, $C_1$-$C_8$-Haloalkyl, $C_2$-$C_8$-Alkenyl, $C_2$-$C_8$-Haloalkenyl, $C_3$-$C_8$-Alkinyl, $C_3$-$C_7$-Cycloalkyl, Halogen-$C_3$-$C_7$-cycloalkyl, $C_1$-$C_8$-Alkylcarbonyl, Allylcarbonyl, $C_3$-$C_7$-Cycloalkylcarbonyl, Benzoyl, das unsubstituiert oder am Phenylring gleich oder verschieden bis zu dreifach durch Halogen, $C_1$-$C_4$-Alkyl, $C_1$-$C_4$-

Haloalkyl, $C_1$-$C_4$-Haloalkoxy oder $C_1$-$C_4$-Alkoxy substituiert ist; $C_1$-$C_6$-Alkyl substituiert durch Cyano, Nitro, Carboxyl, $C_1$-$C_8$-Alkylthio-$C_1$-$C_8$-alkoxycarbonyl, Phenyl, Halogenphenyl, $C_1$-$C_4$-Alkylphenyl, $C_1$-$C_4$-Alkoxyphenyl, $C_1$-$C_4$-Haloalkylphenyl, $C_1$-$C_4$-Haloalkoxyphenyl, $C_1$-$C_6$-Alkoxycarbonyl, $C_1$-$C_4$-Alkoxy-$C_1$-$C_8$-alkoxycarbonyl, $C_3$-$C_8$-Alkenyloxycarbonyl, $C_3$-$C_8$-Alkinyloxycarbonyl, $C_1$-$C_8$-Alkylthiocarbonyl, $C_3$-$C_8$-Alkenylthiocarbonyl, $C_3$-$C_8$-Alkinylthiocarbonyl, Carbamoyl, $C_1$-$C_4$-Alkylaminocarbonyl, Di-$C_1$-$C_4$-alkylaminocarbonyl; Phenylaminocarbonyl, das unsubstituiert oder am Phenyl gleich oder verschieden bis zu dreifach durch Halogen, $C_1$-$C_4$-Alkyl, $C_1$-$C_4$-Haloalkyl, $C_1$-$C_4$-Haloalkoxy oder $C_1$-$C_4$-Alkoxy oder einfach durch Cyano oder Nitro substituiert ist; Dioxolan-2-yl, das unsubstituiert oder durch ein oder zwei $C_1$-$C_4$-Alkylreste substituiert ist; oder Dioxanyl, das unsubstituiert oder durch ein oder zwei $C_1$-$C_4$-Alkylreste substituiert ist;

$R_5$ Wasserstoff, $C_1$-$C_{10}$-Alkyl, $C_1$-$C_4$-Alkoxy-$C_1$-$C_4$-alkyl, $C_2$-$C_8$-Haloalkyl, $C_1$-$C_{10}$-Alkylthio-$C_1$-$C_4$-alkyl, Di-$C_1$-$C_4$-alkylamino-$C_1$-$C_4$-alkyl, Cyano-$C_1$-$C_8$-alkyl, $C_3$-$C_8$-Alkenyl, $C_3$-$C_8$-Haloalkenyl, $C_3$-$C_8$-Alkinyl, $C_3$-$C_7$-Cycloalkyl, $C_3$-$C_7$-Cycloalkyl-$C_1$-$C_4$-alkyl, Halogen-$C_3$-$C_7$-cycloalkyl oder Benzyl, das unsubstituiert oder am Phenylring gleich oder verschieden bis zu dreifach durch Halogen, $C_1$-$C_4$-Alkyl, $C_1$-$C_4$-Haloalkyl, $C_1$-$C_4$-Haloalkoxy oder $C_1$-$C_4$-Alkoxy substituiert ist; Alkali-, Erdalkali- oder Ammoniumionen; oder die Gruppe $-[CHR_6(CH_2)_{n_4}]-COOR_7$;

$R_6$, $R_{20}$, $R_{21}$, $R_{26}$, $R_{28}$, $R_{32}$, $R_{34}$, $R_{39}$, $R_{40}$, $R_{46}$, $R_{47}$, $R_{49}$, $R_{50}$, $R_{51}$ und $R_{52}$ unabhängig voneinander Wasserstoff oder $C_1$-$C_4$-Alkyl;

$R_7$ und $R_{48}$ unabhängig voneinander Wasserstoff, $C_1$-$C_6$-Alkyl, $C_3$-$C_8$-Alkenyl, $C_3$-$C_8$-Alkinyl, $C_1$-$C_8$-Alkoxy-$C_2$-$C_8$-alkyl, $C_1$-$C_8$-Alkylthio-$C_1$-$C_8$-alkyl oder $C_3$-$C_7$-Cycloalkyl;

$R_8$ Wasserstoff oder $C_1$-$C_4$-Alkyl;

$R_{44}$ und $R_{45}$ unabhängig voneinander Wasserstoff, $C_1$-$C_4$-Alkyl, $C_1$-$C_4$-Haloalkyl oder $C_1$-$C_4$-Alkoxy-$C_1$-$C_4$-alkyl;

$R_9$ und $R_{10}$ unabhängig voneinander jeweils $C_1$-$C_4$-Alkyl, $C_2$-$C_4$-Haloalkyl oder $C_2$-$C_8$-Alkoxyalkyl; oder $R_9$ und $R_{10}$ zusammen eine Ethano-, Propano- oder eine Cyclohexan- 1 ,2-diylbrücke, wobei diese Gruppen entweder unsubstituiert oder durch ein bis zwei Reste aus der Gruppe $C_1$-$C_4$-Alkyl, $C_1$-$C_4$-Haloalkyl oder $C_1$-$C_4$-Hydroxyalkyl substituiert sein können;

$R_{11}$ Wasserstoff, $C_1$-$C_5$-Alkyl oder $C_3$-$C_7$-Alkenyl;

$R_{12}$ $C_1$-$C_8$-Alkyl;

$R_{13}$ Wasserstoff, $C_1$-$C_5$-Alkyl, Benzyl, $C_1$-$C_4$-Haloalkyl, $C_3$-$C_8$-Alkenyl oder $C_3$-$C_8$-Alkinyl;

$R_{14}$ $C_1$-$C_6$-Alkyl, $C_1$-$C_5$-Haloalkyl oder Di-$C_1$-$C_4$-alkylamino;

$R_{15}$ Wasserstoff, Fluor, Chlor, Brom, $C_1$-$C_4$-Alkyl oder Trifluormethyl;

$R_{16}$ Chlor, $-X_5-R_{17}$, Amino, $C_1$-$C_4$-Alkylamino, Di-$C_1$-$C_4$-alkylamino, $C_2$-$C_4$-Haloalkylamino, Di-$C_2$-$C_4$-haloalkylamino, $C_1$-$C_4$-Alkoxyalkylamino, Di-$C_1$-$C_4$-alkoxyalkylamino, $C_3$-$C_4$-Alkenylamino, Diallylamino, -N-Pyrrolidino, -N-Piperidino, -N-Morpholino, -N-Thiomorpholino, -N-Piperazino, oder die Gruppe $-O-N=C(CH_3)-CH_3$, $-O-CH_2-CH_2-O-N=C(CH_3)-CH_3$ oder $-N(OR_{46})-R_6$;

$R_{17}$ Wasserstoff, $C_1$-$C_{10}$-Alkyl, $C_1$-$C_4$-Alkoxy-$C_1$-$C_4$-alkyl, $C_2$-$C_8$-Haloalkyl, $C_1$-$C_{10}$-Alkylthio-$C_1$-$C_4$-alkyl, Di-$C_1$-$C_4$-alkylamino-$C_1$-$C_4$-alkyl, Cyano-$C_1$-$C_8$-alkyl, $C_3$-$C_8$-Alkenyl, $C_3$-$C_8$-Haloalkenyl, $C_3$-$C_8$-Alkinyl, $C_3$-$C_7$-Cycloalkyl, $C_3$-$C_7$-Cycloalkyl-$C_1$-$C_4$-alkyl, Halogen-$C_3$-$C_7$-cycloalkyl oder Benzyl, das unsubstituiert oder am Phenylring gleich oder verschieden bis zu dreifach durch Halogen, $C_1$-$C_4$-Alkyl, $C_1$-$C_4$-Haloalkyl, $C_1$-$C_4$-Haloalkoxy oder $C_1$-$C_4$-Alkoxy substituiert ist; Alkali-, Erdalkali- oder Ammoniumionen, oder die Gruppe $[CHR_{47}-(CH_2)_m]-COOR_{48}$ oder $-[CHR_{49}-(CH_2)_t-Si(R_{18})_3]$;

m 0, 1, 2, 3 oder 4;

t 0, 1, 2, 3 oder 4;

$R_{18}$ $C_1$-$C_4$-Alkyl;

$R_{19}$ Wasserstoff, $C_1$-$C_6$-Alkyl, $C_2$-$C_4$-Alkenyl, oder $C_2$-$C_6$-Alkinyl; durch Halogen substituiertes $C_1$-$C_6$-Alkyl, $C_2$-$C_4$-Alkenyl, oder $C_3$-$C_6$-Alkinyl; $C_1$-$C_4$-Alkoxy-$C_1$-$C_4$-alkyl, $C_1$-$C_4$-Alkoxy-$C_1$-$C_2$-alkoxy-$C_1$-$C_2$-alkyl, 1-Phenylpropen-3-yl, durch Cyano oder $C_3$-$C_6$-Cycloalkyl substituiertes $C_1$-$C_6$-Alkyl; Carboxy-$C_1$-$C_4$-alkyl, $C_1$-$C_6$-Alkoxycarbonyl-$C_1$-$C_4$-alkyl, $C_2$-$C_6$-Haloalkoxycarbonyl-$C_1$-$C_4$-alkyl, $C_1$-$C_4$-Alkoxy-$C_1$-$C_2$-alkoxycarbonyl-$C_1$-$C_4$-alkyl, $C_1$-$C_6$-Alkoxycarbonyl-$C_1$-$C_2$-alkoxycarbonyl-$C_1$-$C_4$-alkyl, $C_3$-$C_6$-Cycloalkyl-$C_1$-$C_2$-alkoxycarbonyl-$C_1$-$C_4$-alkyl, $C_1$-$C_5$-Alkylaminocarbonyl-$C_1$-$C_4$-alkyl, Di-$C_1$-$C_4$-alkylamino-$C_1$-$C_4$-alkyl, Di-$C_1$-$C_5$-alkylaminocarbonyl $C_1$-$C_4$-alkyl, $C_3$-$C_6$-Cycloalkyl, $C_1$-$C_4$-Alkylthio-$C_1$-$C_4$-alkyl, Benzyl oder durch Halogen substituiertes Benzyl, $C_1$-$C_4$-Alkylsulfonyl, $C_3$-$C_6$-Alkenyloxy-$C_1$-$C_4$-alkyl, $C_1$-$C_4$-Alkoxycarbonyl, $C_1$-$C_8$-Alkylcarbonyl,

$C_1$-$C_4$-Alkyl-COO 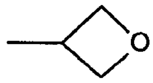, $C_1$-$C_4$-Alkyl-COO 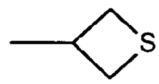, 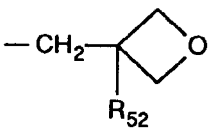,

$C_1$-$C_4$-Alkylthiocarbonyl-$C_1$-$C_4$-alkyl, oder die Gruppe $-[CHR_{47}-(CH_2)_m]COX_6-CHR_{47}-(CH_2)_m-COOR_{48}$;

$R_{25}$, $R_{29}$, $R_{31}$, $R_{35}$ und $R_{36}$ unabhängig voneiander Wasserstoff, $C_1$-$C_4$-Alkyl, $C_1$-$C_6$-Haloalkyl, $C_3$-$C_8$-Alkenyl, $C_3$-$C_8$-Haloalkenyl, $C_3$-$C_8$-Alkinyl, $C_1$-$C_4$-Alkoxy-$C_1$-$C_8$-alkyl, Cyano-$C_1$-$C_4$-alkyl, $C_1$-$C_8$-Alkoxycarbonyl-$C_1$-$C_4$-alkyl, $C_3$-$C_7$-Cycloalkyl, $C_3$-$C_7$-Cycloalkyl-$C_1$-$C_4$-alkyl, Benzyl, durch -N-Morpholino, -N-Thiomorpholino oder -N-Piperazino substituiertes $C_1$-$C_4$-Alkyl, Di-$C_1$-$C_4$-alkylamino-$C_1$-$C_4$-alkyl, $C_1$-$C_4$-Alkylaminocarbonyl-$C_1$-$C_4$-alkyl, Di-$C_1$-$C_4$-alkylaminocarbonyl-$C_1$-$C_4$-alkyl, $C_1$-$C_4$-Alkoxycarbonyl oder $C_1$-$C_4$-Alkylcarbonyl;

$X_1$, $X_2$, $X_3$, $X_4$, $X_5$ und $X_6$ unabhängig voneinander Sauerstoff oder Schwefel;

$n_1$, $n_2$, $n_3$ und $n_4$ unabhängig voneinander 0, 1, 2, 3 oder 4, bedeuten; mit den Massgaben, dass mindestens einer der Substituenten $R_1$, $R_2$ oder A verschieden von Wasserstoff ist; und dass wenn $R_{53}$ Wasserstoff ist, $R_{54}$ verschieden von $C_1$-$C_4$-Alkyl ist; und ihre Salze und Stereoisomere.

In den obigen Definitionen ist unter Halogen, Fluor, Chlor, Brom und Jod, vorzugsweise Fluor, Chlor und Brom zu verstehen.

Als Alkylgruppen können geradkettige oder verzweigte Alkylgruppen in Betracht kommen, beispielsweise Methyl, Ethyl, n-Propyl, iso-Propyl, n-Butyl iso-Butyl, sek.-Butyl, tert.-Butyl, sowie die verschiedenen isomeren Pentyl-, Hexyl-, Heptyl-, Octyl-, Nonyl- und Decylreste.

Als Haloalkyl kommen ein- oder mehrfach, insbesondere ein- bis dreifach durch Halogen substituierte Alkylgruppen in Betracht, wobei Halogen im einzelnen Brom oder Jod und insbesondere Fluor oder Chlor bedeutet, beispielsweise Fluormethyl, Difluormethyl, Trifluormethyl, Chlormethyl, Dichlormethyl, Trichlormethyl, 2,2,2-Trifluorethyl, 2-Fluorethyl, 2-Chlorethyl und 2,2,2-Trichlorethyl; vorzugsweise Difluorchlormethyl, Trifluormethyl, Dichlorfluormethyl und Trichlormethyl.

Alkoxy ist beispielsweise Methoxy, Ethoxy, n-Propyloxy, iso-Propyloxy, n-Butyloxy, iso-Butyloxy, sek.-Butyloxy und tert.-Butyloxy; vorzugsweise Methoxy, Ethoxy und iso-Propyloxy.

Haloalkoxy ist beispielsweise Fluormethoxy, Difluormethoxy, Trifluormethoxy, 2,2,2-Trifluorethoxy, 1,1,2,2-Tetrafluorethoxy, 2-Fluorethoxy, 2-Chlorethoxy und 2,2,2-Trichlorethoxy; vorzugsweise Difluormethoxy, Trifluormethoxy und 2-Chlorethoxy.

Alkylthio ist beispielsweise Methylthio, Ethylthio, n-Propylthio, iso-Propylthio, n-Butylthio, iso-Butylthio, sek.-Butylthio, tert.-Butylthio oder die isomeren Pentylthio, vorzugsweise Methylthio und Ethylthio.

Unter Alkenyl ist geradkettiges oder verzweigtes Alkenyl zu verstehen, wie z.B. Vinyl, Allyl, Methallyl, 1-Methylvinyl, But-2-en-1-yl, Pentenyl, 2-Hexenyl oder 3-Heptenyl. Bevorzugt sind Alkenylreste mit einer Kettenlänge von 2 bis 4 Kohlenstoffatomen.

Die in den Definitionen der Substituenten vorkommenden Alkinylreste können geradkettig oder verzweigt sein wie beispielsweise Ethinyl, Propargyl, 3-Butinyl, 1-Methylpropargyl, 1-Pentinyl oder 2-Hexinyl. Bevorzugt sind Ethinyl und Propargyl.

Cycloalkyl steht beispielsweise für Cyclopropyl, Dimethylcyclopropyl, Cyclobutyl, Cyclopentyl, Methylcyclopentyl, Cyclohexyl oder Cycloheptyl, vorzugsweise aber für Cyclopropyl, Cyclopentyl und Cyclohexyl.

Alkoxycarbonyl ist beispielsweise Methoxycarbonyl, Ethoxycarbonyl, n-Propyloxycarbonyl, iso-Propyloxycarbonyl oder n-Butyloxycarbonyl, vorzugsweise Methoxycarbonyl und Ethoxycarbonyl.

Alkoxyalkyl ist beispielsweise Methoxymethyl, Ethoxymethyl, Propyloxymethyl, Methoxyethyl, Ethoxyethyl, Propyloxyethyl, Methoxypropyl, Ethoxypropyl oder Propyloxypropyl.

Alkylthioalkyl ist beispielsweise Methylthiomethyl, Ethylthiomethyl, Methylthioethyl, Ethylthioethyl oder iso-Propylthioethyl.

Alkylaminoalkyl ist beispielsweise Methylaminoethyl, Dimethylaminoethyl, Ethylaminoethyl oder Diethylaminoethyl.

Cyanoalkyl ist beispielsweise Cyanomethyl, Cyanoethyl oder Cyanopropyl.

Halogencycloalkyl ist beispielsweise 2,2-Dichlorcyclopropyl oder Pentachlorcyclohexyl.

Alkylsulfonyl ist beispielsweise Methylsulfonyl, Ethylsulfonyl, Propylsulfonyl oder Butylsulfonyl, vorzugsweise Methyl- und Ethylsulfonyl.

Phenyl, auch als Teil eines Substituenten wie Phenoxy, Phenylthio, Phenoxycarbonyl, Phenylaminocarbonyl, Benzyl oder Benzoyl, kann im allgemeinen unsubstituiert oder substituiert vorliegen. Die Substituenten können dann in ortho-, meta- und/oder para-Stellung stehen. Bevorzugte Substituentenstellungen sind die ortho- und para-Position zur Ringverknüpfungsstelle. Bevorzugte Substituenten sind Halogenatome.

In den weiteren Substituenten, welche aus mehreren Teilelementen zusammengesetzt sind, haben diese Teilelemente die oben an Beispielen erläuterten Bedeutungen.

Bei den Salzen der Verbindungen der Formel I mit aciden Protonen, insbesondere der Derivate mit Carbonsäuregruppen (A= -C(O)-$X_4R_5$, wobei $X_4$ Sauerstoff, und $R_5$ Wasserstoff ist), handelt es sich beispielsweise um Alkalimetallsalze, wie z.B. Natrium- und Kaliumsalze; Erdalkalimetallsalze, wie z.B. Calcium- und Magnesiumsalze; Ammoniumsalze, d.h. unsubstituierte Ammoniumsalze und mono- oder mehrfach-substituierte Ammoniumsalze, wie z.B. Triethylammonium- und Methylammoniumsalze; oder um Salze mit anderen organischen Basen.

Beispiele für zur Salzbildung geeignete Amine sind primäre, sekundäre und tertiäre aliphatische und aromatische Amine wie Methylamin, Ethylamin, n-Propylamin, iso-Propylamin, die vier isomeren Butylamine, n-Amylamin, iso-Amylamin, Hexylamin, Heptylamin, Octylamin, Nonylamin, Decylamin, Pentadecylamin, Hexadecylamin, Heptadecylamin, Octadecylamin, Methyl-ethylamin, Methyl-isopropylamin, Methyl-hexylamin, Methyl-nonylamin, Methyl-pentadecylamin, Methyl-octadecylamin, Ethyl-butylamin, Ethyl-heptylamin, Ethyl-octylamin, Hexyl-heptylamin, Hexyl-octylamin, Dimethylamin, Diethylamin, Di-n-propylamin, Di-iso-propylamin, Di-n-butylamin, Di-n-amylamin, Di-iso-amylamin, Dihexylamin, Diheptylamin, Dioctylamin, Ethanolamin, n-Propanolamin, iso-Propanolamin, N,N-Diethylethanolamin, N-Ethylpropanolamin, N-Butylethanolamin, Allylamin, n-Butenyl-2-amin, n-Pentenyl-2-amin, 2,3-Dimethylbutenyl-2-amin, Di-butenyl-2-amin, n-Hexenyl-2-amin, Propylendiamin, Diethanolamin, Trimethylamin, Triethylamin, Tri-n-propylamin, Tri-iso-propylamin, Tri-n-butylamin, Tri-iso-butylamin, Tri-sek.-butylamin, Tri-n-amylamin; heterocyclische Amine wie z.B. Pyridin, Chinolin, iso-Chinolin, Morpholin, Thiomorpholin, N-Methylmorpholin, N-Methyl-thiomorpholin, Piperidin, Pyrrolidin, Indolin, Chinuclidin und Azepin; primäre Arylamine wie z.B. Aniline, Methoxyaniline, Ethoxyaniline, o,m,p-Toluidine, Phenylendiamine, Benzidine, Naphthylamine und o,m,p-Chloraniline.

Das mögliche Vorhandensein mindestens eines asymmetrischen Kohlenstoffatoms in den Verbindungen der Formel I, beispielsweise in den Ester- und Etherderivaten mit substituierten aliphatischen und alicyclischen Gruppen, hat zur Folge, dass die Verbindungen, sowohl in optisch aktiven Einzelisomeren, als auch in Form von racemischen Gemischen auftreten können. In der vorliegenden Erfindung sind unter den Wirkstoffen der Formel I sowohl die reinen optischen Antipoden als auch die Racemate zu verstehen. Sofern nicht speziell auf die einzelnen optischen Antipoden hingewiesen wird, sind diejenigen racemischen Gemische unter der gegebenen Formel zu verstehen, die beim angegebenen Herstellungsverfahren entstehen. Liegt eine aliphatische C=C- oder C=N-Doppelbindung vor, so kann auch geometrische Isomerie auftreten.

Bevorzugt sind Verbindungen der Formel I, worin Z Sauerstoff bedeutet.

Bevorzugt sind ebenfalls Verbindungen der Formel Ia

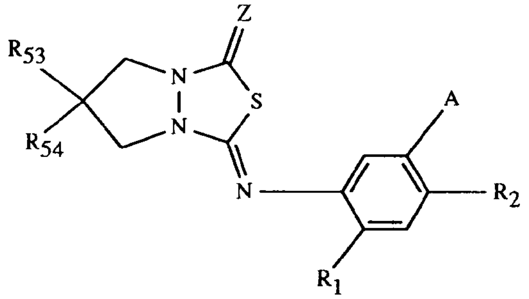

(Ia),

worin Z, A, $R_1$, $R_2$, $R_{53}$ und $R_{54}$ die unter Formel I angegebene Bedeutung haben.

Hiervon sind diejenigen Verbindungen besonders bevorzugt, worin A enweder für $-X_3R_4$, $-COR_8$, $-COR_3$,

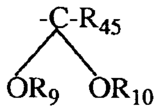 , 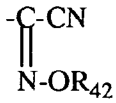 , 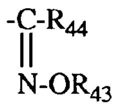

oder $-N(R_{13})-SO_2-R_{14}$ steht, wobei $X_3$ insbesondere Schwefel und $R_4$ durch $C_1$-$C_6$-Alkoxycarbonyl substituiertes $C_1$-$C_6$-Alkyl bedeutet.

In besonders bevorzugten Verbindungen der Formel Ia steht $R_1$ und $R_2$ für Halogen, insbesondere $R_1$ für Fluor und $R_2$ für Chlor.

Ebenfalls zu den bevorzugten Verbindungen gehören diejenigen der Formel Ib

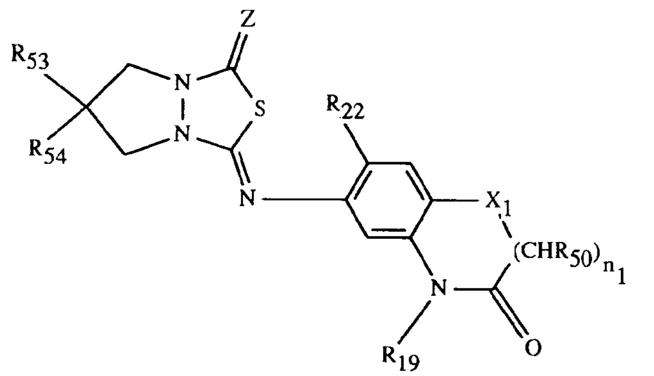

(Ib),

worin Z, $R_{19}$, $R_{22}$, $X_1$, $R_{50}$, $R_{53}$, $R_{54}$ und $n_1$ die unter Formel I angegebene Bedeutung haben.

Hiervon besonders bevorzugt sind diejenigen Verbindungen, worin $R_{19}$ $C_1$-$C_4$-Alkyl, $C_3$-oder $C_4$-Alkenyl, $C_3$- oder $C_4$-Haloalkenyl, $C_3$-$C_6$-Alkinyl, $C_1$-$C_4$-Alkoxy-$C_1$-$C_4$-alkyl, Cyano-$C_1$-$C_4$-alkyl, $C_1$-$C_6$-Alkoxycarbonyl-$C_1$-$C_4$-alkyl, $C_3$-$C_6$-Cycloalkyl, $C_3$-$C_6$-Cycloalkyl-$C_1$-$C_4$-alkyl, Benzyl, Di-$C_1$-$C_4$-alkylamino-$C_1$-$C_4$-alkyl, $C_1$-$C_4$-Alkylaminocarbonyl-$C_1$-$C_4$-alkyl, Di-$C_1$-$C_4$-alkylaminocarbonyl-$C_1$-$C_4$-alkyl, $C_1$-$C_4$-Alkoxycarbonyl oder $C_1$-$C_4$-Alkylcarbonyl bedeutet.

Ganz besonders bevorzugt sind hiervon Verbindungen, worin Z Sauerstoff; $R_{19}$ $C_1$-$C_3$-Alkyl, $C_1$-$C_3$-Alkoxycarbonyl-$C_1$- oder -$C_2$-alkyl oder $C_3$- oder $C_4$-Alkinyl; $R_{22}$ Wasserstoff oder Fluor, $R_{50}$ Wasserstoff; und $n_1$ 0 oder 1 ist.

Von besonderer Bedeutung sind Verbindungen der Formel I, worin $R_{53}$ und $R_{54}$ $C_1$-$C_4$-Alkyl bedeutet.

Ganz besonders wichtig sind hiervon Verbindungen der Formel I, worin $R_{53}$ und $R_{54}$ unabhängig voneinander Methyl oder Ethyl bedeuten.

Von besonderer Bedeutung sind auch Verbindungen der Formel I, worin $R_{53}$ und $R_{54}$ zusammen mit dem C-Atom, an das sie gebunden sind, einen unsubstituierten 3- oder 5-gliedrigen Ring bilden.

Von besonderer Bedeutung sind ebenfalls Verbindungen der Formel I, worin $R_{53}$ Wasserstoff; und $R_{54}$ $C_1$-$C_3$-Alkoxy, $C_3$-$C_5$-Alkenyloxy, $C_3$-$C_5$-Alkinyloxy oder $C_1$-$C_3$-Haloalkoxy bedeuten.

Ganz besonders bevorzugt sind hiervon diejenigen Verbindungen, worin $R_{54}$ $C_1$-$C_3$-Alkoxy oder $C_1$-$C_3$-Haloalkoxy bedeutet. Insbesondere sind von diesen Verbindungen diejenigen ganz besonders wichtig, worin $R_{54}$ Methoxy, iso-Propyloxy oder Difluormethoxy ist.

Als Einzelverbindungen aus dem Umfang der Formel I sind zu nennen:

8-(4-Chlor-2-fluor-5-iso-propyloxy-phenylimino)-3,3-dimethyl-7-thia-1,5-diazabicyclo[3.3.0]oktan-6-on;

8-(4-Chlor-2-fluor-5-iso-propyloxy-phenylimino)-3-methoxy-7-thia-1,5-diazabicyclo[3.3.0]oktan-6-on;

8-(4-Chlor-2-fluor-5-iso-propyloxy-phenylimino)-3-difluormethoxy-7-thia-1,5-diazabicyclo[3.3.0]oktan-6-on; und

8-(4-Chlor-2-fluor-5-iso-propyloxy-phenylimino)-3,3-ethandiyl-7-thia-1,5-diazabicyclo[3.3.0]oktan-6-on.

Das erfindungsgemässe Verfahren zur Herstellung der Verbindungen der Formel I

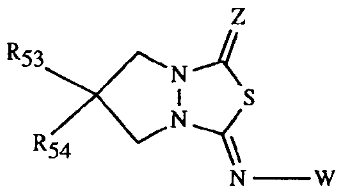

(I),

worin Z, W, $R_{53}$ und $R_{54}$ die unter Formel I angegebene Bedeutung haben, erfolgt in Analogie zu bekannten Verfahren und ist dadurch gekennzeichnet, dass man ein Isothiocyanat der Formel II

S=C=N-W (II),

worin W die unter Formel I angegebene Bedeutung hat, mit einer Verbindung der Formel III

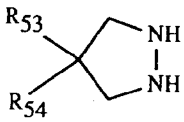

(III)

worin $R_{53}$ und $R_{54}$ die unter Formel I angegebene Bedeutung haben, in die Verbindung der Formel IV

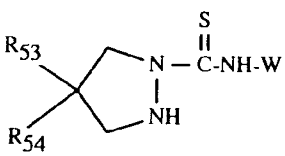

(IV)

überführt und diese anschließend mit einer Verbindung der Formel V

$CZCl_2$ (V),

worin Z für Sauerstoff oder Schwefel steht, umsetzt.

Die Umsetzung der Isothiocyanate der Formel II mit den Verbindungen der Formeln III wird mit Vorteil in einem inerten, organischen Lösungsmittel bei Temperaturen von -5°C bis zur Siedetemperatur des Lösungsmittels, insbesondere von 0 bis +50°C, besonders bevorzugt bei Raumtemperatur, durchgeführt. Geeignete Lösungsmittel für diese Reaktion sind beispielsweise Toluol, Xylole, Essigsäureethylester oder Acetonitril.

Die Umsetzung der Verbindung der Formeln IV mit der Verbindung der Formel V erfolgt vorteilhaft in einem inerten, organischen Lösungsmittel bei niedrigen Temperaturen, vorzugsweise bei 0 bis +50°C, besonders bevorzugt bei 0 bis +15°C.

Die für das erfindungsgemäße Herstellungsverfahren benötigten Ausgangsverbindungen der Formeln II und III sind entweder bekannt oder lassen sich analog zu literaturbekannten Verfahren herstellen. Die Herstellung derartiger Verbindungen aus 1,3-Dibrompropanen und Hydrazin ist beispielsweise in J. Am. Chem. Soc. 88, 3959-3963 (1966) beschrieben.

Verbindungen der Formel IIIa

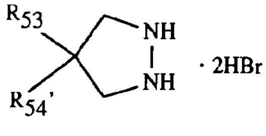

(IIIa),

worin $R_{53}$ Wasserstoff oder $C_1$-$C_6$-Alkyl; $R_{54}'$ $C_1$-$C_6$-Alkyl ist; oder $R_{53}$ und $R_{54}'$ zusammen mit dem C-Atom, an das sie gebunden sind, einen unsubstituierten 3-, 4-, 5- oder 6-gliedrigen gesättigten Ring bilden, können gemäss Reaktionsschema 1 hergestellt werden.

Reaktionsschema 1:

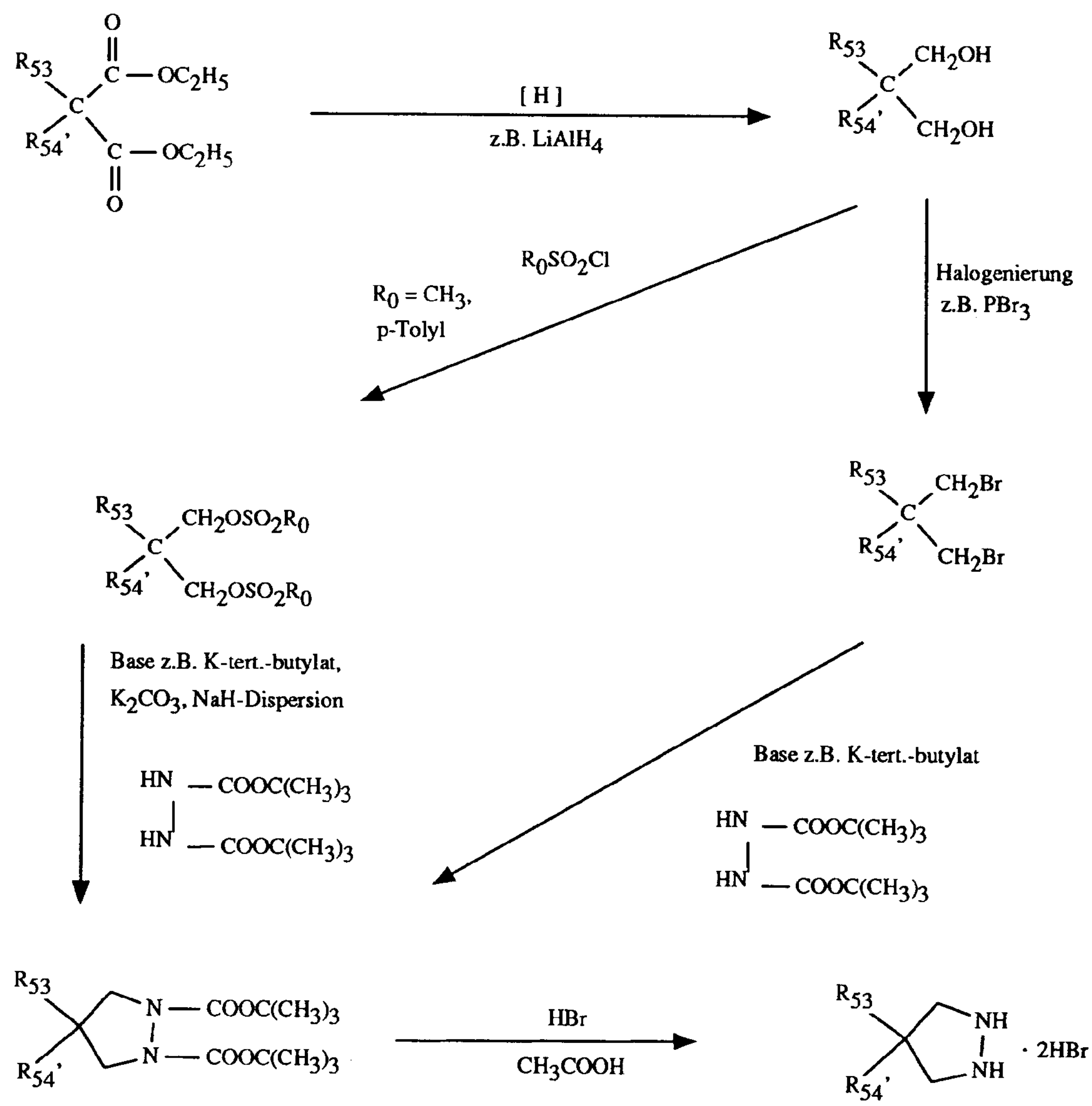


IIIa (Hydrobromid)

Verbindungen der Formel IIIb

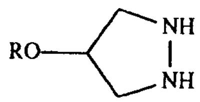

(IIIb),

worin R $C_1$-$C_6$-Alkyl, $C_3$-$C_6$-Alkenyl, $C_3$-$C_6$-Alkinyl oder $C_1$-$C_6$-Haloalkyl bedeutet, können gemäss Reaktionsschema 2 hergestellt werden.

Reaktionsschema 2:

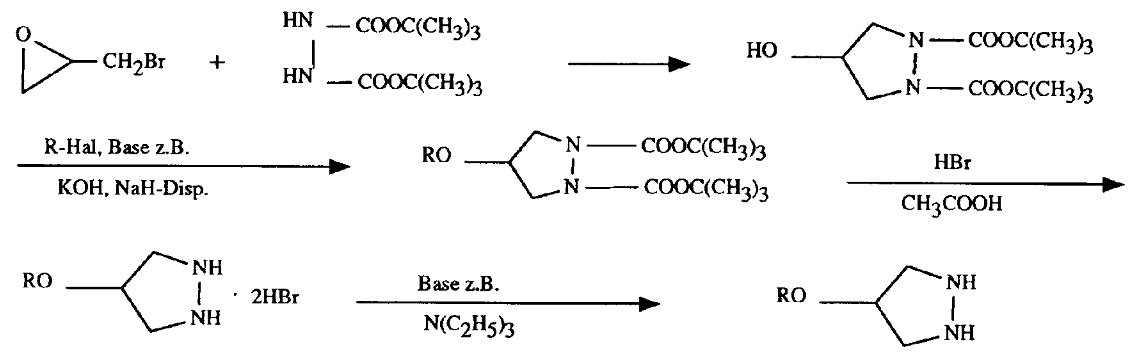


IIIb

Die Isothiocyanate der Formel II sind bekannt oder sind analog zu bekannten Verfahren herstellbar. Derartige Verbindungen sind beispielsweise in EP-A-0 304 920, EP-A-0 238 711, EP-A-0 409 025, EP-A-0 373 461, EP-A-0 311 135 und DE-OS-3 724 098 beschrieben.

Die Wirkstoffe der Formel I werden in der Regel mit Aufwandmengen von 0,001 bis 2 kg/ha, insbesondere 0,005 bis 1 kg/ha erfolgreich eingesetzt. Die für die erwünschte Wirkung erforderliche Dosierung kann durch Versuche ermittelt werden. Sie ist abhängig von der Art der Wirkung, dem Entwicklungsstadium der Kulturpflanze und des Unkrauts, sowie von der Applikation (Ort, Zeit, Verfahren) und kann, bedingt durch diese Parameter, innerhalb weiter Bereiche variieren.

Bei geringeren Aufwandmengen zeichnen sich die Verbindungen der Formel I durch wuchshemmende und herbizide Eigenschaften aus, die sie ausgezeichnet zum Einsatz in Kulturen von Nutzpflanzen, insbesondere in Getreide, Mais, Raps, Soja, Reis und Baumwolle, befähigen, wobei der Einsatz in Sojakultur ganz besonders bevorzugt ist.

Die Erfindung betrifft auch herbizide Mittel, welche einen neuen Wirkstoff der Formel I enthalten, sowie Verfahren zur Hemmung des Pflanzenwuchses.

Für die Verwendung der Verbindung der Formel I oder der Mittel, die diese Verbindungen als Wirkstoffe enthalten, zur Regulierung des Pflanzenwachstums kommen verschiedene Methoden und Techniken in Betracht, wie beispielsweise die folgenden:

i) Samenbeizung

a) Beizung der Samen mit einem als Spritzpulver formulierten Wirkstoff durch Schütteln in einem Gefäß bis zur gleichmäßigen Verteilung auf der Samenoberfläche (Trockenbeizung). Man verwendet dabei bis zu 4 g Wirkstoff der Formel I (bei einer 50 %igen Formulierung: bis zu 8,0 g Spritzpulver) pro 1 kg Saatgut.

b) Beizung der Samen mit einem Emulsionskonzentrat des Wirkstoffs oder mit einer wäßrigen Lösung des als Spritzpulver formulierten Wirkstoffs der Formel I nach Methode a) (Naßbeizung).

c) Beizung durch Tauchen des Saatguts in einer Brühe mit bis zu 1000 ppm Wirkstoff der Formel I während 1 bis 72 Stunden und gegebenenfalls nachfolgendes Trocknen der Samen (Tauchbeizung, Seed soaking).

Die Beizung des Saatguts oder die Behandlung des angekeimten Sämlings sind naturgemäß die bevorzugten Methoden der Applikation, weil die Wirkstoffbehandlung vollständig auf die Zielkultur gerichtet ist. Man verwendet in der Regel 0,001 g bis 4,0 g Aktivsubstanz pro 1 kg Saatgut, wobei man je nach Methodik, die auch den Zusatz anderer Wirkstoffe oder Mikronährstoffe ermöglicht, von den angegebenen Grenzkonzentrationen nach oben oder unten abweichen kann (Wiederholungsbeize).

ii) Kontrollierte Wirkstoffabgabe

Der Wirkstoff wird in Lösung auf mineralische Granulatträger oder polymerisierte Granulate (Harnstoff/Formaldehyd) aufgezogen und trocknen gelassen. Gegebenenfalls kann ein Überzug aufgebracht werden (Umhüllungsgranulate), der es erlaubt, den Wirkstoff über einen bestimmten Zeitraum dosiert abzugeben.

Die Verbindungen der Formel I werden in unveränderter Form, wie aus der Synthese erhältlich, oder vorzugsweise mit den in der Formulierungstechnik üblichen Hilfsmitteln eingesetzt und werden daher z.B. zu emulgierbaren Konzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Benetzen, Verstreuen oder Gießen werden gleich wie die Art der Mittel den angestrebten Zielen und den gegebenen Verhältnissen entsprechend gewählt.

Die Formulierungen, d.h. die den Wirkstoff der Formel I und gegebenenfalls einen oder mehrere feste oder flüssige Zusatzstoffe enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen und gegebenenfalls oberflächenaktiven Verbindungen (Tensiden).

Als Lösungsmittel können in Frage kommen: Aromatische Kohlenwasserstoffe, insbesondere die Fraktionen $C_8$ bis $C_{12}$, wie Mischungen von Alkylbenzolen, z.B. Xylolgemische oder alkylierte Naphthaline; aliphatische und cycloaliphatische Kohlenwasserstoffe wie Paraffine, Cyclohexan oder Tetrahydronaphthalin; Alkohole, wie Ethanol, Propanol oder Butanol; Glykole sowie deren Ether und Ester, wie Propylenglykol oder Dipropylenglykolether, Ketone wie Cyclohexanon, Isophoron oder Diacetonalkohol, starke polare Lösungsmittel wie N-Methyl-2-pyrrolidon, Dimethylsulfoxid oder Wasser; Pflanzenöle sowie deren Ester, wie Raps-, Ricinus- oder Sojaöl; gegebenenfalls auch Silikonöle.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen seien die Alkali-, Erdalkali oder gegebenenfalls substituierten Ammoniumsalze von höheren Fettsäuren ($C_{10}$-$C_{22}$), wie z.B. die Na- oder K-Salze der Öl- oder Stearinsäure, oder von natürlichen Fettsäuregemischen genannt, die z.B. aus Kokosnuß- oder Talgöl gewonnen werden können. Ferner sind auch die Fettsäure-methyl-taurinsalze zu erwähnen.

Häufiger werden jedoch sog. synthetische Tenside verwendet, insbesondere Fettalkoholsulfonate, Fettalkoholsulfate, sulfonierte Benzimidazolderivate oder Alkylarylsulfonate.

Die Fettalkoholsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschließt, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, die Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxid-Adduktes oder Phospholipide in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 Propylenglykolethergruppen, enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nicht ionischer Tenside seien Nonylphenolpolyethoxyethanole, Ricinusölpolyglykolether, Polypropylen-Polyethylenoxidaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi-(2-chlorethyl)-ethylammoniumbromid.

Die in der Formulierungstechnik gebräuchlichen Tenside sind u.a. in folgenden Publikationen beschrieben:

- "Mc Cutcheon's Detergents and Emulsifiers Annual", Mc Publishing Corp., Glen Rock, New Jersey, 1988.
- M. and J. Ash, "Encyclopedia of Surfactants", Vol. I-III, Chemical Publishing Co., New York, 1980-1981.
- Dr. Helmut Stache "Tensid-Taschenbuch", Carl Hanser Verlag, München/Wien 1981.

Die herbiziden Zubereitungen enthalten in der Regel 0,1 bis 99 %, insbesondere 0,1 bis 95 %, Wirkstoff der Formel I, 1 bis 99 % eines festen oder flüssigen Zusatzstoffes und 0 bis 25 %, insbesondere 0,1 bis 25 % eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren z.B. gegebenenfalls epoxydierte Pflanzenöle (epoxydiertes Kokosnußöl, Rapsöl oder Sojaöl), Entschäumer, z.B. Silikonöl, Konservierungsmittel, Viskositätsregulatoren, Bindemittel, Haftmittel sowie Dünger oder andere Wirkstoffe zur Erzielung spezieller Effekte enthalten.

Insbesondere setzen sich bevorzugte Formulierungen folgendermaßen zusammen:
(% = Gewichtsprozent)

| Emulgierbare Konzentrate: | |
|---|---|
| Aktiver Wirkstoff: | 1 bis 90 %, vorzugsweise 5 bis 50 % |
| oberflächenaktives Mittel: | 5 bis 30 %, vorzugsweise 10 bis 20 % |
| flüssiges Trägermittel: | 15 bis 94 %, vorzugsweise 70 bis 85 % |

| Stäube: | |
|---|---|
| Aktiver Wirkstoff: | 0,1 bis 10 %, vorzugsweise 0,1 bis 1 % |
| festes Trägermittel: | 99,9 bis 90 %, vorzugsweise 99,9 bis 99 % |

| Suspensions-Konzentrate: | |
|---|---|
| Aktiver Wirkstoff: | 5 bis 75 %, vorzugsweise 10 bis 50 % |
| Wasser: | 94 bis 24 %, vorzugsweise 88 bis 30 % |
| oberflächenaktives Mittel: | 1 bis 40 %; vorzugsweise 2 bis 30 % |

| Benetzbare Pulver: | |
|---|---|
| Aktiver Wirkstoff: | 0,5 bis 90 %, vorzugsweise 1 bis 80 % |
| oberflächenaktives Mittel: | 0,5 bis 20 %, vorzugsweise 1 bis 15 % |
| festes Trägermaterial: | 5 bis 95 %, vorzugsweise 15 bis 90 % |

| Granulate: | |
|---|---|
| Aktiver Wirkstoff: | 0,5 bis 30 %, vorzugsweise 3 bis 15 % |
| festes Trägermittel: | 99,5 bis 70 %, vorzugsweise 97 bis 85 % |

A. Herstellungsbeispiele

Beispiel H1: Herstellung von 2,3-Diazaspiro[4.2]cycloheptan-2,3-dicarbonsäure-tert.-butylester

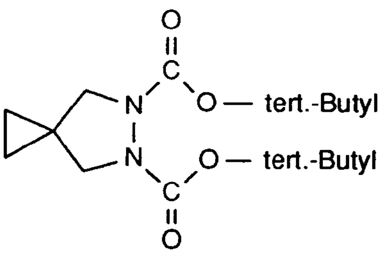

Zu einer Suspension von 3,1 g 60 %iger Natriumhydrid-Dispersion in 40 ml N,N-Dimethylformamid tropft man 9,1 g Hydrazin-N,N'-dicarbonsäure-di-tert.-butylester in 20 ml N,N-Dimethylformamid bei maximal 30°C. Nach 1-stündigem Rühren bei 22°C wird 10,0 g 1,1-Cyclopropan-dimethanol-dimethansulfonat in 20 ml N,N-Dimethylformamid als Suspension zur Reaktionsmischung zugetropft und anschliessend während 18 Stunden bei 22°C gerührt. Man giesst die entstandene gelbe Suspension auf 700 ml Eiswasser, nutscht das ausgefallene Produkt ab und trocknet es im Exsikkator. Man erhält 6,8 g des gewünschten Produktes 2,3-Diazaspiro[4.2]cycloheptan-2,3-dicarbonsäure-tert.-butylester als wachsartige Kristalle; Smp. 60-70°C.

Beispiel H2: Herstellung von 2,3-Diazaspiro[4.2]cycloheptan-dihydrobromid

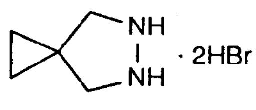

Zu einer Lösung von 29,8 g 2,3-Diazaspiro[4.2]cycloheptan-2,3-dicarbonsäure-tert.-butylester in 500 ml Diethylether wird bei 0-5°C 50 ml einer Lösung von Bromwasserstoff in Essigsäure (33 %) getropft. Nach 3 Stunden Rühren bei 0-5°C wird der ausgefallene Niederschlag abgenutscht, mit Diethylether gewaschen und im Vakuum getrocknet. Man erhält 23,3 g des gewünschten 2,3-Diazaspiro[4.2]cycloheptan-dihydrobromid als weisse, hygroskopische Kristalle.

Beispiel H3: Herstellung von 4-Hydroxypyrazolidin-N,N'-dicarbonsäure-di-tert.-butylester

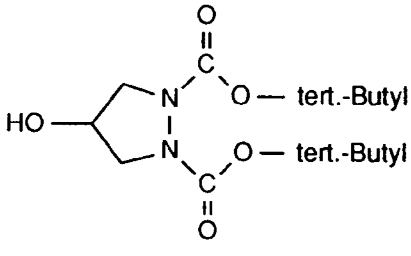

46,4 g Hydrazin-N,N'-dicarbonsäure-di-tert.-butylester, 69,0 g Kaliumcarbonat, 3,3 g Kaliumjodid, 20 ml Tetrabutylammoniumfluorid (1 molare Lösung in Tetrahydrofuran) und 21 ml Epibromhydrin werden in 400 ml

Ethylmethylketon während 48 Stunden am Rückfluss gekocht. Man engt die erhaltene Suspension ein, gibt 500 ml Diethylether dazu, filtriert ab und engt ein. Die Reindarstellung des Rohprodukts mittels Säulenchromatographie liefert 27,1 g des gewünschten 4-Hydroxy-pyrazolidin-N,N'-dicarbonsäure-di-tert.-butylester als weisse Kristalle; Smp. 100-102°C.

Beispiel H4: Herstellung von 4-Hydroxy-pyrazolidin-dihydrobromid

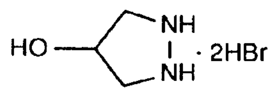

Zu einer Lösung von 11,5 g 4-Hydroxy-pyrazolidin-N,N'-dicarbonsaure-di-tert.-butylester in 160 ml Diethylether wird bei 22°C 24 ml einer Lösung von Bromwasserstoff in Essigsäure (33 %) getropft. Nach 3-stündigem Rühren bei 22°C wird 200 ml Diethylether zugegeben, das ausgefallene Produkt abfiltriert und am Hochvakuum bei 50°C getrocknet. Man erhält 9,4 g 4-Hydroxy-pyrazolidin-dihydrobromid als hellbraune, hygroskopische Kristalle.

Beispiel H5: Herstellung von 4-Methoxy-pyrazolidin-N,N'-dicarbonsäure-di-tert.-butylester

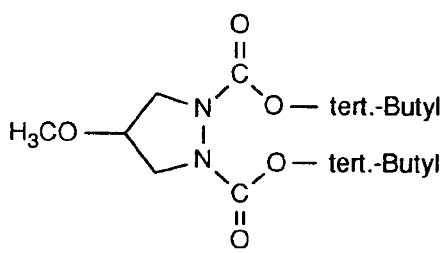

Zu einer Lösung von 8,6 g 4-Hydroxy-pyrazolidin-N,N'-dicarbonsäure-di-tert.-butylester in 90 ml N,N-Dimethylformamid gibt man bei 0-5°C portionenweise 1,2 g Natriumhydrid-Dispersion (80 %). Nach 30 Minuten Rühren bei 0-5°C tropft man eine Lösung von 2,5 ml Methyljodid in 10 ml N,N-Dimethylformamid dazu. Anschliessend wird während 3 Stunden bei 0-5°C weitergerührt und dann 500 ml Diethylether dazugegeben. Die organische Phase wäscht man mit Wasser, trocknet über Natriumsulfat und engt ein. Man erhält 8,0 g des gewünschten Produkts 4-Methoxy-pyrazolidin-N,N'-dicarbonsäure-di-tert.-butylester als gelbes Oel.

Beispiel H6: 4-Methoxy-pyrazolidin-dihydrobromid wird analog zu Beispiel H5 durch Verwendung von 4-Methoxy-pyrazolidin-N,N'-dicarbonsäure-di-tert.-butylester erhalten.

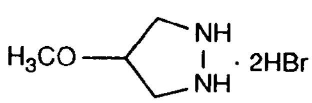

Beispiel H7: Herstellung von 4-Difluor-methoxy-pyrazolidin-N,N'-dicarbonsäure-di-tert.-butylester

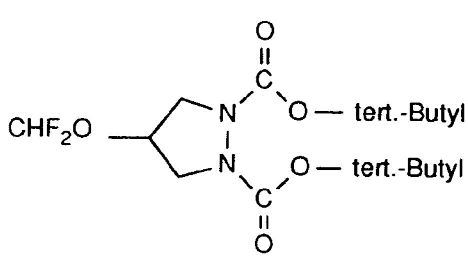

Zu einer Lösung von 6,9 g 4-Hydroxy-pyrazolidin-N,N'-dicarbonsäure-di-tert.-butylester in 60 ml Tetrahydrofuran gibt man 0,2 g Tetrabutylammoniumfluorid und 5,6 g Kaliumhydroxydpulver. Bei maximal 45°C wird 8,0 g Chlordifluormethan eingeleitet. Anschliessend wird die Reaktionslösung filtriert, eingeengt und mittels Säulenchromatographie gereinigt. Man erhält 2,7 g des gewünschten Produkts 4-Difluormethoxy-pyrazolidin-N,N'-dicarbonsäure-di-tert.-butylester als gelbes Oel.

Beispiel H8: 4-Difluormethoxy-pyrazolidin-dihydrobromid wird analog zu Beispiel H5 durch Verwendung von 4-Difluormethoxy-pyrazolidin-N,N'-dicarbonsäure-di-tert.-butylester erhalten.

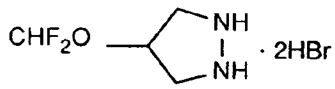

Beispiel H9: Herstellung von 4,4-Dimethyl-pyrazolidin-N,N'-dicarbonsäure-di-tert.-butylester

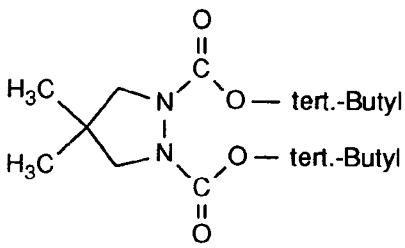

41,8 g Hydrazin-N,N'-dicarbonsäure-di-tert.-butylester, 74,5 g Kaliumcarbonat, 52,0 g 2,2-Dimethyl-1,3-propandiol-dimethansulfonat und 2,0 g Kaliumjodid werden in 360 ml N,N-Dimethylformamid während 48 Stunden bei 120-130°C gerührt. Darauf wird eingeengt, Diethylether zugegeben und die organische Phase mit Wasser gewaschen. Nach dem Trocknen über Natriumsulfat wird eingeengt und mittels Säulenchromatographie gereinigt. Man erhält 1,1 g des gewünschten Produkts 4,4-Dimethyl-pyrazolidin-N,N'-dicarbonsäure-di-tert.-butylester als weisse Kristalle.

Beispiel H10: 4,4-Dimethylpyrazolidin-dihydrobromid wird analog zu Beispiel H5 durch Verwendung von 4,4-Dimethyl-pyrazolidin-N,N'-dicarbonsäure-di-tert.-butylester erhalten.

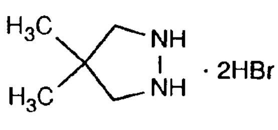

Beispiel H11: Herstellung von α-[2-Chlor-4-fluor-5-(4-methoxypyrazolidinylthio-carbonylamino)-phenylthio]-essigsäure-methylester

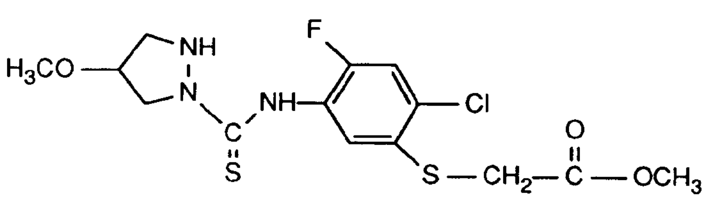

Zu einer Suspension von 2,65 g 4-Methoxypyrazolidin-dihydrobromid in 50 ml Essigsäureethylester gibt man 4,2 ml Triethylamin und rührt während 10 Minuten bei 22°C. Dann gibt man 2,35 g 4-Chlor-2-fluor-5-methoxycarbonylmethyl-thiophenylisothiocyanat dazu und rührt über Nacht bei 22°C. Die organische Phase wird mit Wasser gewaschen, getrocknet und eingeengt. Das kristalline Produkt α-[2-Chlor-4-fluor-5-(4-methoxypyrazolidinyl-thiocarbonylamino)-phenylthio]-essigsäuremethylester erhält man in 3,0 g Ausbeute; Smp. > 90°C (Zersetzung).

Beispiel H12: Herstellung von 8-(4-Chlor-2-fluor-5-methoxycarbonylmethylthio-phenylimino)-3-methoxy-7-thia- 1,5-diazabicyclo[3.3.0]oktan-6-on

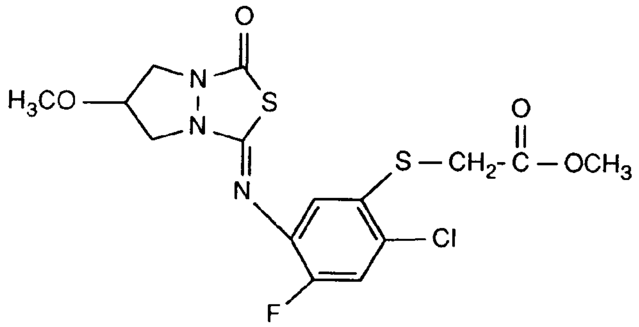

Zu einer Lösung von 2,8 g α-[2-Chlor-4-fluor-5-(4-methoxypyrazolidinyl-thiocarbonyl-amino)-phenylthio]-essigsäuremethylester in 20 ml Toluol gibt man bei 0-5°C 4,2 ml Phosgenlösung in Toluol (20 %). Nach 2-stündigem Rühren bei 22°C wird die organische Phase mit Wasser gewaschen, getrocknet und eingeengt. Die Reindarstellung des Produktes erfoglt mittels Säulenchromatographie. Man erhält 2,21 g des gewünschten Produktes 8-(4-Chlor-2-fluor-5-methoxy-carbonylmethylthio-phenylimino)-3-methoxy-7-thia-1,5-diazabicyclo[3.3.0]oktan-6-on als gelbes Oel.

Beispiel H13: Herstellung von 4-Hydroxy-N-[(2-fluor-4-chlor-5-isopropoxy)-phenyl]-pyrazolidin-2-thioamid

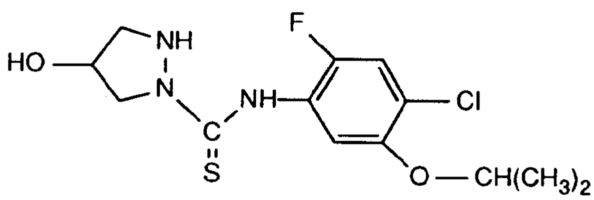

Zu einer Suspension von 2,0 g 4-Hydroxy-pyrazolidin-dihydrobromid in 25 ml Tetrahydrofuran wird bei 0-5°C 2,8 ml Triethylamin getropft. Nach 10 Minuten Rühren bei 22°C wird 2,0 g 4-Chlor-2-fluor-5-isopropoxy-phenylisothiocyanat in 5 ml Tetrahydrofuran dazugetropft und während 3 Stunden gerührt. Nach dem Einengen des Reaktionsgemisches wird Essigsäureethylester zugegeben, die organische Phase mit Wasser gewaschen, getrocknet und eingeengt. Die Reindarstellung des Produktes erfolgt durch Kristallisation aus Petrolether. Das gewünschte Produkt 4-Hydroxy-N-[(2-fluor-4-chlor-5-isopropoxy)-phenyl]-pyrazolidin-2-thioamid erhält man in 2,2 g Ausbeute als weisse Kristalle; Smp. 151-153°C (Zersetzung).

Beispiel H14: Herstellung von 8-(4-Chlor-2-fluor-5-isopropoxyphenylimino)-3-hydroxy-7-thia-1,5-diazabicyclo[3.3.0]oktan-6-on

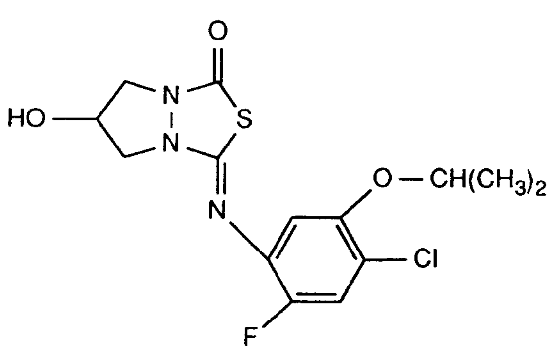

Zu einer Lösung von 2,1 g 4-Chlor-2-fluor-5-isopropoxy-N-(4-hydroxypyrazolidinylthiocarbonyl)-anilin in 50 ml Dichlormethan wird bei 0-5°C 3,4 ml Phosgen gelöst in Toluol (20 %) zugetropft. Nach 1-stündigem Rühren bei 22°C wird eingeengt und dann Essigsäureethylester zugegeben. Die organische Phase wird mit Wasser gewaschen, getrocknet, eingeengt und der erhaltene Rückstand mittels Säulenchromatographie gereinigt. Man erhält 1,95 g des gewünschten Produktes 8-(4-Chlor-2-fluor-5-isopropoxyphenylimino)-3-hydroxy-7-thia-1,5-diazabicyclo[3.3.0]oktan-6-on als Harz.

In analoger Weise werden die in den nachfolgenden Tabellen 1 bis 15 aufgeführten Verbindungen der Formel I hergestellt.

Tabelle 1: Verbindungen der Formel Ic:

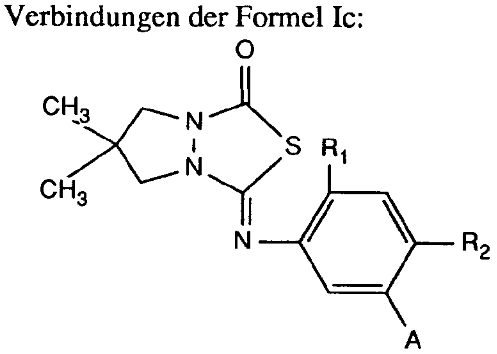

(Ic)

| Verb. Nr. | $R_1$ | $R_2$ | A | Phys. Daten |
|---|---|---|---|---|
| 1.001 | F | Cl | -H | |
| 1.002 | F | Cl | -CN | |
| 1.003 | F | Cl | $-NO_2$ | |
| 1.004 | F | Cl | -COOH | |
| 1.005 | F | Cl | $-COOCH_3$ | |
| 1.006 | F | Cl | $-COOC_2H_5$ | |
| 1.007 | F | Cl | $-COOC_3H_7$ | |
| 1.008 | F | Cl | $-COOCH(CH_3)_2$ | |
| 1.009 | F | Cl | $-COOC_4H_9$ | |
| 1.010 | F | Cl | $-COOCH-CH_2-CH_3$<br>$CH_3$ | |
| 1.011 | F | Cl | $CH_3$<br>$-COOCH_2-CH_2-CH$<br>$CH_3$ | |
| 1.012 | F | Cl | $-COOC_5H_{11}$ | |
| 1.013 | F | Cl | $-COOCH_2-CH_2-O-CH_3$ | |
| 1.014 | F | Cl | $-COOCH_2-CH_2-O-C_2H_5$ | |
| 1.015 | F | Cl | $-COOCH(CH_3)-CH_2-OCH_3$ | |
| 1.016 | F | Cl | $-COOCH_2-CH_2-S-CH_3$ | |
| 1.017 | F | Cl | $-COOCH(CH_3)-CH_2-S-CH_3$ | |
| 1.018 | F | Cl | $-COOCH(CH_3)-CH_2-S-C_2H_5$ | |
| 1.019 | F | Cl | $-COOCH(CH_3)-CH_2-S-C_3H_7$ | |

| Verb. Nr. | $R_1$ | $R_2$ | A | Phys. Daten |
|---|---|---|---|---|
| 1.020 | F | Cl | 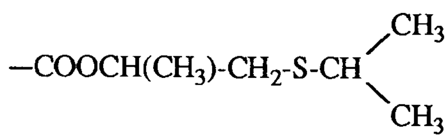 | |
| 1.021 | F | Cl | $-COOCH(CH_3)-CH_2-S-C_4H_9$ | |
| 1.022 | F | Cl | $-COOCH(CH_3)-CH_2-S-C_5H_{11}$ | |
| 1.023 | F | Cl | 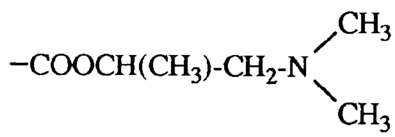 | |
| 1.024 | F | Cl | 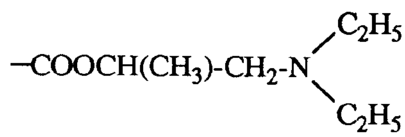 | |
| 1.025 | F | Cl | $-CONH_2$ | |
| 1.026 | F | Cl | $-CONH-CH_3$ | |
| 1.027 | F | Cl | 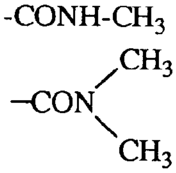 | |
| 1.028 | F | Cl | 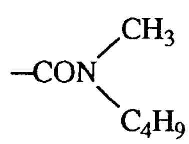 | |
| 1.029 | F | Cl | 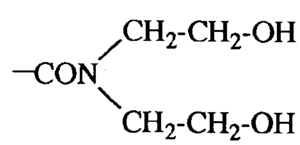 | |
| 1.030 | F | Cl | $-CONH-CH_2-CH=CH_2$ | |
| 1.031 | F | Cl | $-CON(CH_2-CH=CH_2)_2$ | |
| 1.032 | F | Cl | 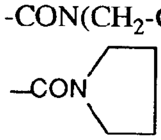 | |
| 1.033 | F | Cl | 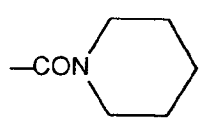 | |

| Verb. Nr. | $R_1$ | $R_2$ | A | Phys. Daten |
|---|---|---|---|---|
| 1.034 | F | Cl | 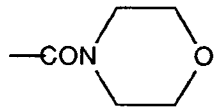 | |
| 1.035 | F | Cl | 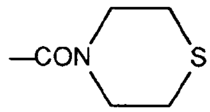 | |
| 1.036 | F | Cl | 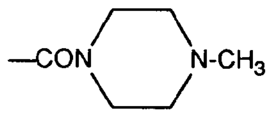 | |
| 1.037 | F | Cl | 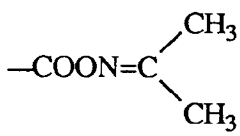 | |
| 1.038 | F | Cl | $-COOCH_2-CH_2-Cl$ | |
| 1.039 | F | Cl | $-COOCH_2-CN$ | |
| 1.040 | F | Cl | 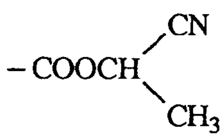 | |
| 1.041 | F | Cl | $-COOCH_2-CH=CH_2$ | |
| 1.042 | F | Cl | $-COOCH_2-CH=CHCl$ | |
| 1.043 | F | Cl | 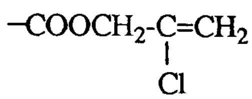 | |
| 1.044 | F | Cl | $-COOCH_2-C\equiv CH$ | |
| 1.045 | F | Cl | 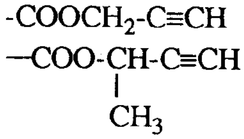 | |
| 1.046 | F | Cl | 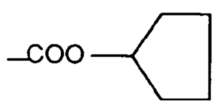 | |
| 1.047 | F | Cl | 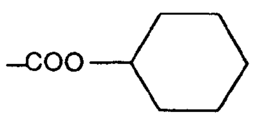 | |

| Verb. Nr. | $R_1$ | $R_2$ | A | Phys. Daten |
|---|---|---|---|---|
| 1.048 | F | Cl | $-COOCH_2-$ 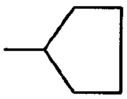 | |
| 1.049 | F | Cl | $-COOCH$ 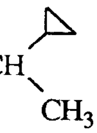 | |
| 1.050 | F | Cl | $-COOCH_2-$ 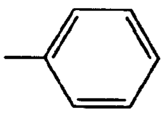 | |
| 1.051 | F | Cl | $-COOCH_2-$ 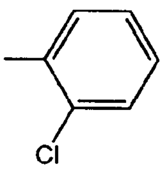 | |
| 1.052 | F | Cl | $COOCH_2-$ 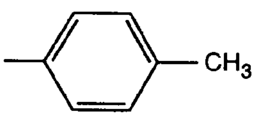 | |
| 1.053 | F | Cl | $-COSCH_3$ | |
| 1.054 | F | Cl | $-COSC_2H_5$ | |
| 1.055 | F | Cl | $-COSC_3H_7$ | |
| 1.056 | F | Cl | $-COS-CH_2-CH=CH_2$ | |
| 1.057 | F | Cl | $-COS-CH_2-COOCH_3$ | |
| 1.058 | F | Cl | $-COS-CH_2-COOC_2H_5$ | |
| 1.059 | F | Cl | $-COS-CH_2-COOC_5H_{11}$ | |
| 1.060 | F | Cl | $-COS-CH(CH_3)-COOCH_3$ | |
| 1.061 | F | Cl | $-COS-CH(CH_3)-COOC_2H_5$ | |
| 1.062 | F | Cl | $-COS-CH(CH_3)-COOC_3H_7$ | |

| Verb. Nr. | $R_1$ | $R_2$ | A | Phys. Daten |
|---|---|---|---|---|
| 1.063 | F | Cl | $-COS-CH_2-CH_2-COOCH_3$ | |
| 1.064 | F | Cl | $-COS-CH_2-COOCH_2-CH_2-O-CH_3$ | |
| 1.065 | F | Cl | $-COOCH_2-COOCH_3$ | |
| 1.066 | F | Cl | 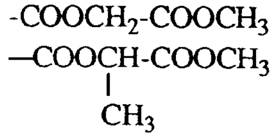 | |
| 1.067 | F | Cl | $-COOCH_2-COOC_5H_{11}$ | |
| 1.068 | F | Cl | $-COOCH_2-CH_2-Si(CH_3)_3$ | |
| 1.069 | F | Cl | -COONa | |
| 1.070 | F | Cl | 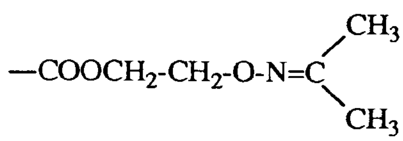 | |
| 1.071 | F | Cl | -OH | |
| 1.072 | F | Cl | $-OCH_3$ | |
| 1.073 | F | Cl | $-OC_2H_5$ | |
| 1.074 | F | Cl | $-OC_3H_7$ | |
| 1.075 | F | Cl | 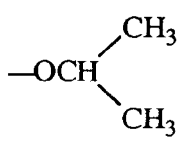 | Smp. 76-77°C |
| 1.076 | F | Cl | $-OC_4H_9$ | |
| 1.077 | F | Cl | 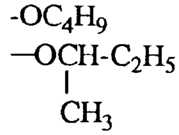 | |
| 1.078 | F | Cl | 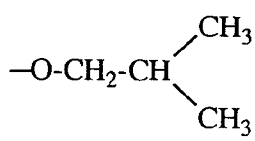 | |
| 1.079 | F | Cl | $-OCH_2CH=CH_2$ | |
| 1.080 | F | Cl | 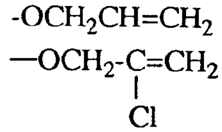 | |
| 1.081 | F | Cl | $-OCH_2CH=CHCl$ | |
| 1.082 | F | Cl | $-OCH_2C\equiv CH$ | |

| Verb. Nr. | $R_1$ | $R_2$ | A | Phys. Daten |
|---|---|---|---|---|
| 1.083 | F | Cl | $-OCH-C\equiv CH$<br>$\vert$<br>$CH_3$ | |
| 1.084 | F | Cl | $-OCH_2-COOCH_3$ | |
| 1.085 | F | Cl | $-O-CH_2-COOC_5H_{11}$ | |
| 1.086 | F | Cl | $-O-CH-COOCH_3$<br>$\vert$<br>$CH_3$ | |
| 1.087 | F | Cl | $-O-CH_2-COOC_2H_5$ | |
| 1.088 | F | Cl | $-O-CH-COOC_2H_5$<br>$\vert$<br>$CH_3$ | |
| 1.089 | F | Cl | $-O-CH_2-CH_2-O-CH_3$ | |
| 1.090 | F | Cl | $-O-CH-CH_2-S-CH_3$<br>$\vert$<br>$CH_3$ | |
| 1.091 | F | Cl | $-O-CH-CH_2-S-C_2H_5$<br>$\vert$<br>$CH_3$ | |
| 1.092 | F | Cl | $-O-CH-CH_2-S-C_3H_7$<br>$\vert$<br>$CH_3$ | |
| 1.093 | F | Cl | $-O-CH_2-CH_2-Cl$ | |
| 1.094 | F | Cl | $-O-CH_2-CN$ | |
| 1.095 | F | Cl | $-O-CH-CN$<br>$\vert$<br>$CH_3$ | |
| 1.096 | F | Cl | $-S-CH_3$ | |
| 1.097 | F | Cl | $-S-C_2H_5$ | |
| 1.098 | F | Cl | $-S-C_3H_7$ | |
| 1.099 | F | Cl | $CH_3$<br>$/$<br>$-S-CH$<br>$\backslash$<br>$CH_3$ | |
| 1.100 | F | Cl | $-S-CH_2-CH=CH_2$ | |

| Verb. Nr. | $R_1$ | $R_2$ | A | Phys. Daten |
|---|---|---|---|---|
| 1.101 | F | Cl | —S-CH$_2$-C=CH$_2$<br>│<br>Cl | |
| 1.102 | F | Cl | -S-CH$_2$-CH=CHCl | |
| 1.103 | F | Cl | -S-CH$_2$-C≡CH | |
| 1.104 | F | Cl | —S-CH-C≡CH<br>│<br>CH$_3$ | |
| 1.105 | F | Cl | -S-CH$_2$-COOCH$_3$ | |
| 1.106 | F | Cl | -S-CH$_2$-COOC$_2$H$_5$ | |
| 1.107 | F | Cl | -S-CH$_2$-COOC$_5$H$_{11}$ | |
| 1.108 | F | Cl | —S-CH-COOCH$_3$<br>│<br>CH$_3$ | |
| 1.109 | F | Cl | —S-CH-COOC$_2$H$_5$<br>│<br>CH$_3$ | |
| 1.110 | F | Cl | -S-CH$_2$-COOCH$_2$-CH$_2$-O-CH$_3$ | |
| 1.111 | F | Cl | —O-CH$_2$ — 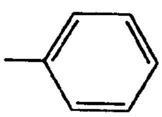 | |
| 1.112 | F | Cl | —S-CH$_2$ — 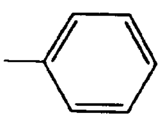 | |
| 1.113 | F | Cl | -C-CN<br>‖<br>N-O-CH$_3$ | |
| 1.114 | F | Cl | -C-CN<br>‖<br>N-O-CH$_2$-COOCH$_3$ | |
| 1.115 | F | Cl | -C-CN<br>‖<br>N-O-CH$_2$-C≡CH | |

| Verb. Nr. | $R_1$ | $R_2$ | A | Phys. Daten |
|---|---|---|---|---|
| 1.116 | F | Cl | 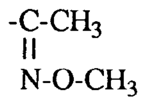 | |
| 1.117 | F | Cl | 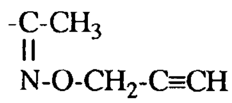 | |
| 1.118 | F | Cl | 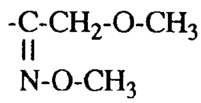 | |
| 1.119 | F | Cl | 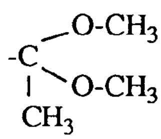 | |
| 1.120 | F | Cl | 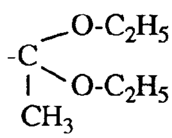 | |
| 1.121 | F | Cl | 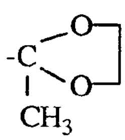 | |
| 1.122 | F | Cl | 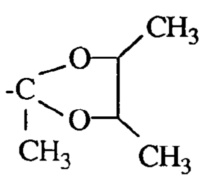 | |
| 1.123 | F | Cl | 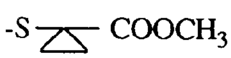 | |
| 1.124 | F | Cl | 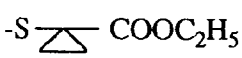 | |
| 1.125 | F | Cl | 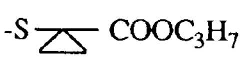 | |
| 1.126 | F | Cl | 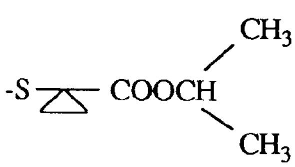 | |

| Verb. Nr. | $R_1$ | $R_2$ | A | Phys. Daten |
|---|---|---|---|---|
| 1.127 | F | Cl | 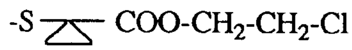 | |
| 1.128 | F | Cl | 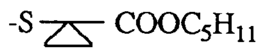 | |
| 1.129 | F | Cl | 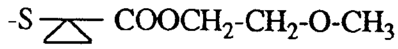 | |
| 1.130 | F | Cl | 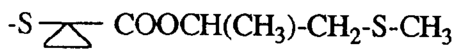 | |
| 1.131 | F | Cl | 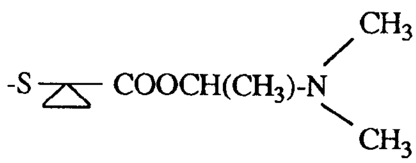 | |
| 1.132 | F | Cl | 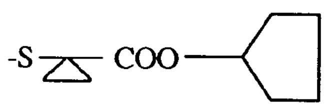 | |
| 1.133 | F | Cl | 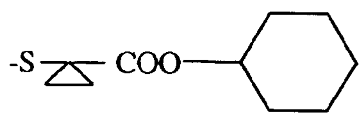 | |
| 1.134 | F | Cl | 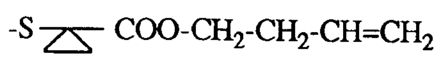 | |
| 1.135 | F | Cl | 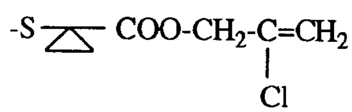 | |
| 1.136 | F | Cl | 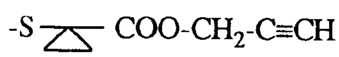 | |
| 1.137 | F | Cl | 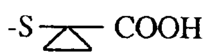 | |
| 1.138 | F | Cl | 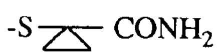 | |
| 1.139 | F | Cl | 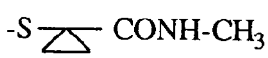 | |
| 1.140 | F | Cl | 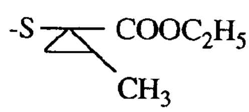 | |
| 1.141 | F | Cl | 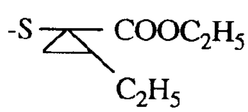 | |

| Verb. Nr. | $R_1$ | $R_2$ | A | Phys. Daten |
|---|---|---|---|---|
| 1.142 | F | Cl | 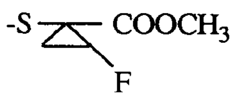 | |
| 1.143 | F | Cl | 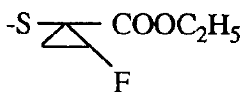 | |
| 1.144 | F | Cl | 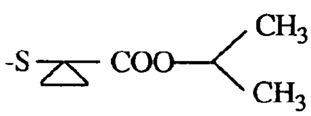 | |
| 1.145 | F | Cl | 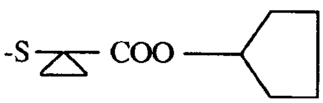 | |
| 1.146 | F | Cl | $-NH-SO_2-CH_3$ | |
| 1.147 | F | Cl | $-NH-SO_2-C_2H_5$ | |
| 1.148 | F | Cl | $-NH-SO_2-Cl$ | |
| 1.149 | F | Cl | 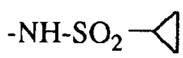 | |
| 1.150 | F | Cl | 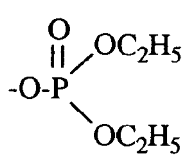 | |
| 1.151 | H | Cl | -COOH | Smp.> 230°C(Zers.) |
| 1.152 | H | Cl | $-COOCH_3$ | Smp. 104-106°C |
| 1.153 | H | Cl | 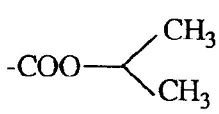 | Smp. 90-91°C |
| 1.154 | H | Cl | $-COO-C_5H_{11}$ | |
| 1.155 | H | Cl | $-COO-CH_2-CH_2-O-CH_3$ | |
| 1.156 | H | Cl | $-COOCH_2-S-CH_3$ | |
| 1.157 | H | Cl | $-COOCH(CH_3)-CH_2-S-CH_3$ | |
| 1.158 | H | Cl | 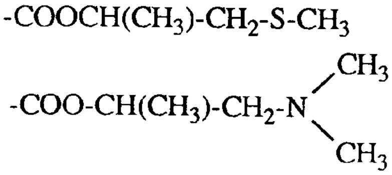 | |

| Verb. Nr. | $R_1$ | $R_2$ | A | Phys. Daten |
|---|---|---|---|---|
| 1.159 | H | Cl | 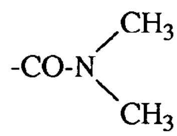 | |
| 1.160 | H | Cl | 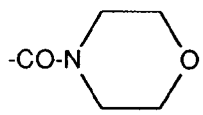 | |
| 1.161 | H | Cl | 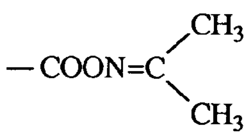 | |
| 1.162 | H | Cl | 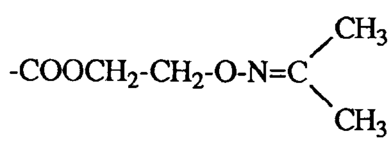 | |
| 1.163 | H | Cl | 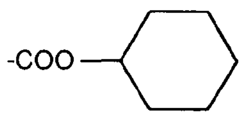 | |
| 1.164 | H | Cl | 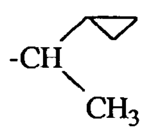 | |
| 1.165 | H | Cl | $-S-C_3H_7$ | |
| 1.166 | H | Cl | $-COOCH_2-COOCH_3$ | |
| 1.167 | H | Cl | $-COOCH(CH_3)-COOCH_3$ | |
| 1.168 | H | Cl | $-COS-CH_2-COOCH_3$ | |
| 1.169 | H | Cl | $-COS-CH(CH_3)-COOCH_3$ | |
| 1.170 | H | Cl | -OH | |
| 1.171 | H | Cl | $-OCH_3$ | |
| 1.172 | H | Cl | $-O-C_2H_5$ | |
| 1.173 | H | Cl | 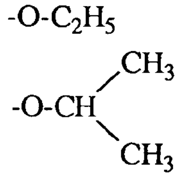 | |
| 1.174 | H | Cl | $-O-CH_2-C\equiv CH$ | |

| Verb. Nr. | $R_1$ | $R_2$ | A | Phys. Daten |
|---|---|---|---|---|
| 1.175 | H | Cl | $-O-CH_2-CH=CHCl$ | |
| 1.176 | H | Cl | 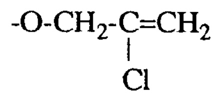 | |
| 1.177 | H | Cl | 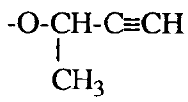 | |
| 1.178 | H | Cl | $-O-CH_2-COOCH_3$ | |
| 1.179 | H | Cl | $-O-CH_2-COOC_2H_5$ | |
| 1.180 | H | Cl | $-O-CH(CH_3)-COOCH_3$ | |
| 1.181 | H | Cl | $-SH$ | |
| 1.182 | H | Cl | $-SCH_3$ | |
| 1.183 | H | Cl | $-SC_2H_5$ | |
| 1.184 | H | Cl | 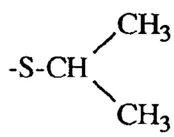 | |
| 1.185 | H | Cl | $-S-CH_2-COOCH_3$ | |
| 1.186 | H | Cl | $-S-CH(CH_3)-COOCH_3$ | |
| 1.187 | H | Cl | $-S-CH_2-COOC_2H_5$ | |
| 1.188 | H | Cl | 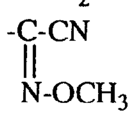 | |
| 1.189 | H | Cl | 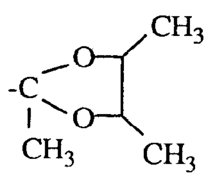 | |
| 1.190 | H | Cl | 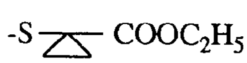 | |
| 1.191 | H | Cl | 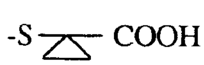 | |
| 1.192 | H | Cl | 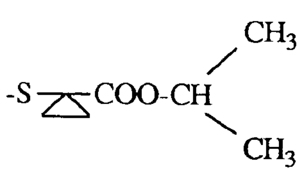 | |

| Verb. Nr. | $R_1$ | $R_2$ | A | Phys. Daten |
|---|---|---|---|---|
| 1.193 | H | Cl | 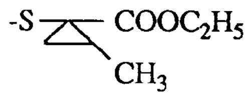 | |
| 1.194 | H | Cl | 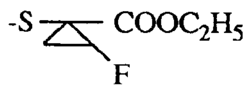 | |
| 1.195 | H | Cl | 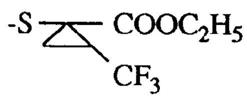 | |
| 1.196 | H | Cl | 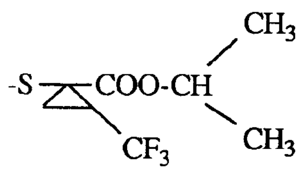 | |
| 1.197 | H | Cl | 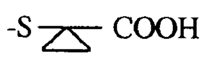 | |
| 1.198 | H | Cl | 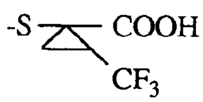 | |
| 1.199 | H | Cl | 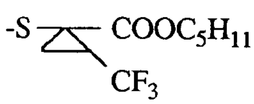 | |
| 1.200 | H | Cl | 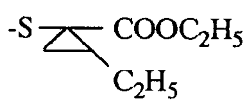 | |
| 1.201 | H | Cl | 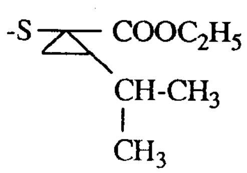 | |
| 1.202 | H | Cl | $-NH-SO_2-C_2H_5$ | |
| 1.203 | H | Cl | $-NH-SO_2-CH_2-Cl$ | |
| 1.204 | F | CN | -COOH | |
| 1.205 | F | CN | 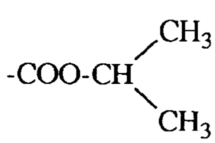 | |

| Verb. Nr. | $R_1$ | $R_2$ | A | Phys. Daten |
|---|---|---|---|---|
| 1.206 | F | CN | 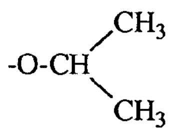 | |
| 1.207 | F | CN | $-O-CH_2-C{\equiv}CH$ | |
| 1.208 | F | CN | $-O-CH(CH_3)-C{\equiv}CH$ | |
| 1.209 | F | CN | $-S-CH_2-COOCH_3$ | |
| 1.210 | F | CN | $-S-CH(CH_3)-COOCH_3$ | |
| 1.211 | F | CN | $-O-CH_2-COOCH_3$ | |
| 1.212 | F | CN | $-O-CH_2-COOC_5H_{11}$ | |
| 1.213 | F | CN | $-O-CH(CH_3)-COOC_2H_5$ | |
| 1.214 | F | CN | 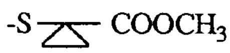 | |
| 1.215 | F | CN | 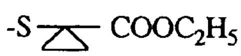 | |
| 1.216 | F | CN | 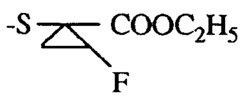 | |
| 1.217 | F | CN | 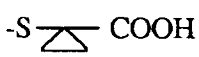 | |
| 1.218 | F | CN | 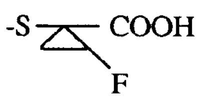 | |
| 1.219 | F | CN | 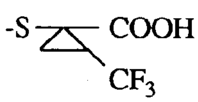 | |
| 1.220 | F | CN | 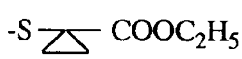 | |
| 1.221 | F | Br | -COOH | |
| 1.222 | F | Br | 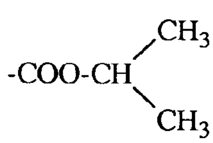 | |
| 1.223 | F | Br | -OH | |

| Verb. Nr. | $R_1$ | $R_2$ | A | Phys. Daten |
|---|---|---|---|---|
| 1.224 | F | Br | 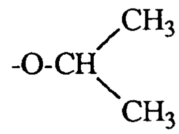 | |
| 1.225 | F | Br | $-O-CH_2-C\equiv CH$ | |
| 1.226 | F | Br | $-O-CH(CH_3)-C\equiv CH$ | |
| 1.227 | F | Br | $-O-CH_2COOCH_3$ | |
| 1.228 | F | Br | $-O-CH_2-COOC_5H_{11}$ | |
| 1.229 | F | Br | $-S-CH_2-COOCH_3$ | |
| 1.230 | F | Br | 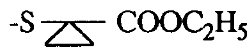 | |
| 1.231 | F | Br | 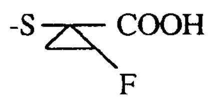 | |
| 1.232 | F | Br | 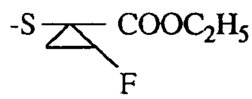 | |
| 1.233 | F | Cl | $-S-CH_2COOH$ | Smp.> 120°C (Zers.) |

Tabelle 2: Verbindungen der Formel Id:

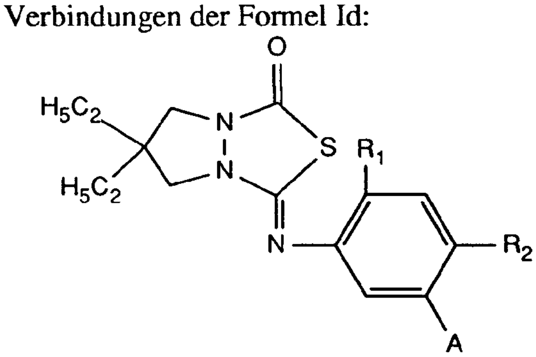

(Id)

| Verb. Nr. | $R_1$ | $R_2$ | A | Phys. Daten |
|---|---|---|---|---|
| 2.001 | F | Cl | -H | |
| 2.002 | F | Cl | -CN | |
| 2.003 | F | Cl | $-NO_2$ | |
| 2.004 | F | Cl | -COOH | |
| 2.005 | F | Cl | $-COOCH_3$ | |
| 2.006 | F | Cl | $-COOC_2H_5$ | |
| 2.007 | F | Cl | $-COOC_3H_7$ | |
| 2.008 | F | Cl | $-COOCH(CH_3)_2$ | |
| 2.009 | F | Cl | $-COOC_4H_9$ | |
| 2.010 | F | Cl | $-COOCH-CH_2-CH_3$<br>\|<br>$CH_3$ | |
| 2.011 | F | Cl | $CH_3$<br>/<br>$-COOCH_2-CH_2-CH$<br>\\<br>$CH_3$ | |
| 2.012 | F | Cl | $-COOC_5H_{11}$ | |
| 2.013 | F | Cl | $-COOCH_2-CH_2-O-CH_3$ | |
| 2.014 | F | Cl | $-COOCH_2-CH_2-O-C_2H_5$ | |
| 2.015 | F | Cl | $-COOCH(CH_3)-CH_2-OCH_3$ | |
| 2.016 | F | Cl | $-COOCH_2-CH_2-S-CH_3$ | |
| 2.017 | F | Cl | $-COOCH(CH_3)-CH_2-S-CH_3$ | |
| 2.018 | F | Cl | $-COOCH(CH_3)-CH_2-S-C_2H_5$ | |

| Verb. Nr. | $R_1$ | $R_2$ | A | Phys. Daten |
|---|---|---|---|---|
| 2.019 | F | Cl | $-COOCH(CH_3)-CH_2-S-C_3H_7$ | |
| 2.020 | F | Cl | 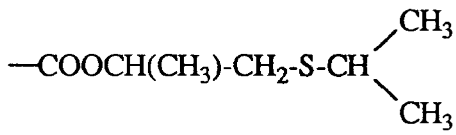 | |
| 2.021 | F | Cl | $-COOCH(CH_3)-CH_2-S-C_4H_9$ | |
| 2.022 | F | Cl | $-COOCH(CH_3)-CH_2-S-C_5H_{11}$ | |
| 2.023 | F | Cl | 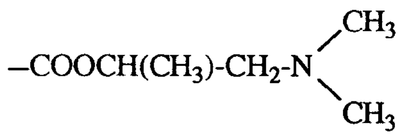 | |
| 2.024 | F | Cl | 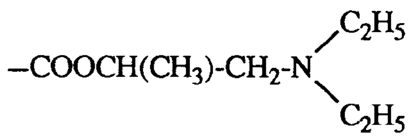 | |
| 2.025 | F | Cl | $-CONH_2$ | |
| 2.026 | F | Cl | $-CONH-CH_3$ | |
| 2.027 | F | Cl | 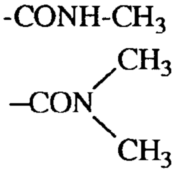 | |
| 2.028 | F | Cl | 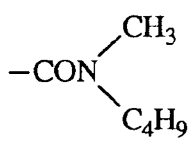 | |
| 2.029 | F | Cl | 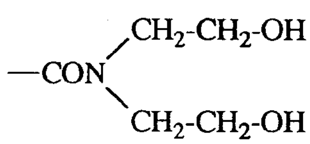 | |
| 2.030 | F | Cl | $-CONH-CH_2-CH=CH_2$ | |
| 2.031 | F | Cl | $-CON(CH_2-CH=CH_2)_2$ | |
| 2.032 | F | Cl | 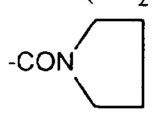 | |
| 2.033 | F | Cl | 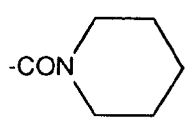 | |

| Verb. Nr. | $R_1$ | $R_2$ | A | Phys. Daten |
|---|---|---|---|---|
| 2.034 | F | Cl | 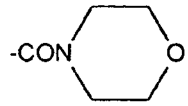 | |
| 2.035 | F | Cl | 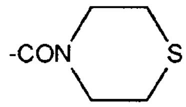 | |
| 2.036 | F | Cl | 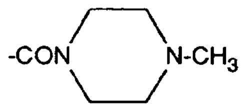 | |
| 2.037 | F | Cl | 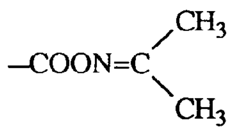 | |
| 2.038 | F | Cl | $-COOCH_2-CH_2-Cl$ | |
| 2.039 | F | Cl | $-COOCH_2-CN$ | |
| 2.040 | F | Cl | 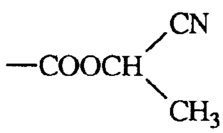 | |
| 2.041 | F | Cl | $-COOCH_2-CH=CH_2$ | |
| 2.042 | F | Cl | $-COOCH_2-CH=CHCl$ | |
| 2.043 | F | Cl | 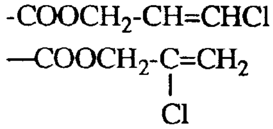 | |
| 2.044 | F | Cl | $-COOCH_2-C\equiv CH$ | |
| 2.045 | F | Cl | 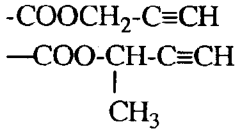 | |
| 2.046 | F | Cl | 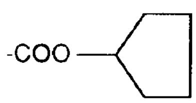 | |
| 2.047 | F | Cl | 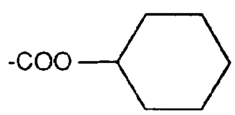 | |

| Verb. Nr. | $R_1$ | $R_2$ | A | Phys. Daten |
|---|---|---|---|---|
| 2.048 | F | Cl | 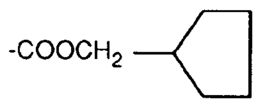 | |
| 2.049 | F | Cl | 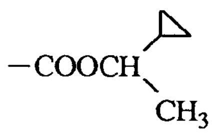 | |
| 2.050 | F | Cl | 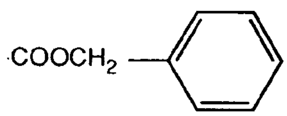 | |
| 2.051 | F | Cl | 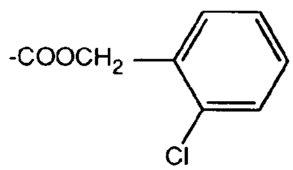 | |
| 2.052 | F | Cl | 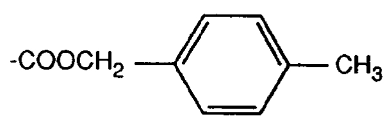 | |
| 2.053 | F | Cl | $-COSCH_3$ | |
| 2.054 | F | Cl | $-COSC_2H_5$ | |
| 2.055 | F | Cl | $-COSC_3H_7$ | |
| 2.056 | F | Cl | $-COS-CH_2-CH=CH_2$ | |
| 2.057 | F | Cl | $-COS-CH_2-COOCH_3$ | |
| 2.058 | F | Cl | $-COS-CH_2-COOC_2H_5$ | |
| 2.059 | F | Cl | $-COS-CH_2-COOC_5H_{11}$ | |
| 2.060 | F | Cl | $-COS-\underset{CH_3}{\underset{\vert}{CH}}-COOCH_3$ | |
| 2.061 | F | Cl | $-COS-\underset{CH_3}{\underset{\vert}{CH}}-COOC_2H_5$ | |
| 2.062 | F | Cl | $-COS-\underset{CH_3}{\underset{\vert}{CH}}-COOC_3H_7$ | |

| Verb. Nr. | $R_1$ | $R_2$ | A | Phys. Daten |
|---|---|---|---|---|
| 2.063 | F | Cl | $-COS-CH_2-CH_2-COOCH_3$ | |
| 2.064 | F | Cl | $-COS-CH_2-COOCH_2-CH_2-O-CH_3$ | |
| 2.065 | F | Cl | $-COOCH_2-COOCH_3$ | |
| 2.066 | F | Cl | 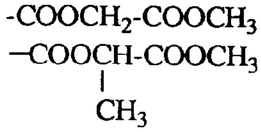 | |
| 2.067 | F | Cl | $-COOCH_2-COOC_5H_{11}$ | |
| 2.068 | F | Cl | $-COOCH_2-CH_2-Si(CH_3)_3$ | |
| 2.069 | F | Cl | -COONa | |
| 2.070 | F | Cl | 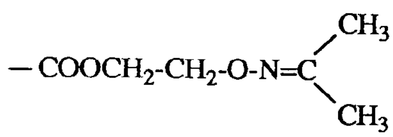 | |
| 2.071 | F | Cl | -OH | |
| 2.072 | F | Cl | $-OCH_3$ | |
| 2.073 | F | Cl | $-OC_2H_5$ | |
| 2.074 | F | Cl | $-OC_3H_7$ | |
| 2.075 | F | Cl | 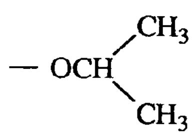 | |
| 2.076 | F | Cl | $-OC_4H_9$ | |
| 2.077 | F | Cl | 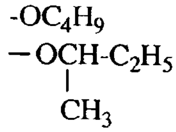 | |
| 2.078 | F | Cl | 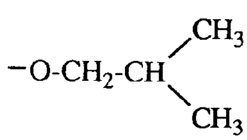 | |
| 2.079 | F | Cl | $-OCH_2CH=CH_2$ | |
| 2.080 | F | Cl | 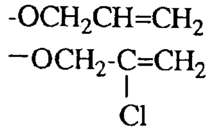 | |
| 2.081 | F | Cl | $-OCH_2CH=CHCl$ | |
| 2.082 | F | Cl | $-OCH_2C\equiv CH$ | |

| Verb. Nr. | $R_1$ | $R_2$ | A | Phys. Daten |
|---|---|---|---|---|
| 2.083 | F | Cl | $-OCH-C\equiv CH$<br>\|<br>$CH_3$ | |
| 2.084 | F | Cl | $-OCH_2-COOCH_3$ | |
| 2.085 | F | Cl | $-O-CH_2-COOC_5H_{11}$ | |
| 2.086 | F | Cl | $-O-CH-COOCH_3$<br>\|<br>$CH_3$ | |
| 2.087 | F | Cl | $-O-CH_2-COOC_2H_5$ | |
| 2.088 | F | Cl | $-O-CH-COOC_2H_5$<br>\|<br>$CH_3$ | |
| 2.089 | F | Cl | $O-CH_2-CH_2-O-CH_3$ | |
| 2.090 | F | Cl | $-O-CH-CH_2-S-CH_3$<br>\|<br>$CH_3$ | |
| 2.091 | F | Cl | $-O-CH-CH_2-S-C_2H_5$<br>\|<br>$CH_3$ | |
| 2.092 | F | Cl | $-O-CH-CH_2-S-C_3H_7$<br>\|<br>$CH_3$ | |
| 2.093 | F | Cl | $-O-CH_2-CH_2-Cl$ | |
| 2.094 | F | Cl | $-O-CH_2-CN$ | |
| 2.095 | F | Cl | $-O-CH-CN$<br>\|<br>$CH_3$ | |
| 2.096 | F | Cl | $-S-CH_3$ | |
| 2.097 | F | Cl | $-S-C_2H_5$ | |
| 2.098 | F | Cl | $-S-C_3H_7$ | |
| 2.099 | F | Cl | $CH_3$<br>/<br>$-S-CH$<br>\\<br>$CH_3$ | |
| 2.100 | F | Cl | $-S-CH_2-CH=CH_2$ | |

| Verb. Nr. | $R_1$ | $R_2$ | A | Phys. Daten |
|---|---|---|---|---|
| 2.101 | F | Cl | $-S-CH_2-C=CH_2$<br>            |<br>            Cl | |
| 2.102 | F | Cl | $-S-CH_2-CH=CHCl$ | |
| 2.103 | F | Cl | $-S-CH_2-C\equiv CH$ | |
| 2.104 | F | Cl | $-S-CH-C\equiv CH$<br>     |<br>    $CH_3$ | |
| 2.105 | F | Cl | $-S-CH_2-COOCH_3$ | |
| 2.106 | F | Cl | $-S-CH_2-COOC_2H_5$ | |
| 2.107 | F | Cl | $-S-CH_2-COOC_5H_{11}$ | |
| 2.108 | F | Cl | $-S-CH-COOCH_3$<br>     |<br>    $CH_3$ | |
| 2.109 | F | Cl | $-S-CH-COOC_2H_5$<br>     |<br>    $CH_3$ | |
| 2.110 | F | Cl | $-S-CH_2-COOCH_2-CH_2-O-CH_3$ | |
| 2.111 | F | Cl | 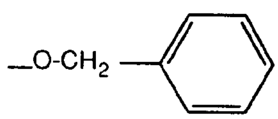 | |
| 2.112 | F | Cl | 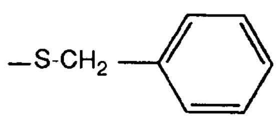 | |
| 2.113 | F | Cl | $-C-CN$<br>  ||<br>$N-O-CH_3$ | |
| 2.114 | F | Cl | $-C-CN$<br>  ||<br>$N-O-CH_2-COOCH_3$ | |
| 2.115 | F | Cl | $-C-CN$<br>  ||<br>$N-O-CH_2-C\equiv CH$ | |

| Verb. Nr. | $R_1$ | $R_2$ | A | Phys. Daten |
|---|---|---|---|---|
| 2.116 | F | Cl | 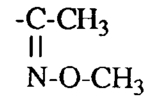 | |
| 2.117 | F | Cl | 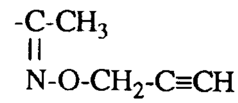 | |
| 2.118 | F | Cl | 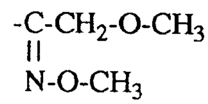 | |
| 2.119 | F | Cl | 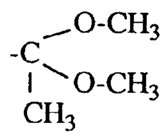 | |
| 2.120 | F | Cl | 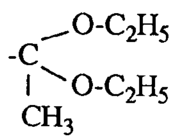 | |
| 2.121 | F | Cl | 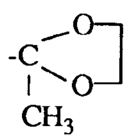 | |
| 2.122 | F | Cl | 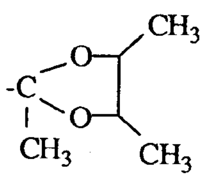 | |
| 2.123 | F | Cl | 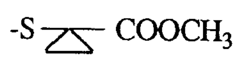 | |
| 2.124 | F | Cl | 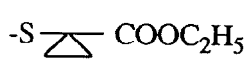 | |
| 2.125 | F | Cl | 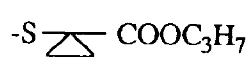 | |
| 2.126 | F | Cl | 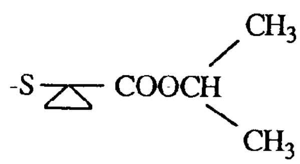 | |

| Verb. Nr. | $R_1$ | $R_2$ | A | Phys. Daten |
|---|---|---|---|---|
| 2.127 | F | Cl | 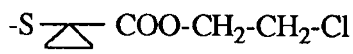 | |
| 2.128 | F | Cl | 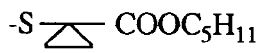 | |
| 2.129 | F | Cl | 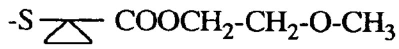 | |
| 2.130 | F | Cl | 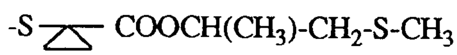 | |
| 2.131 | F | Cl | 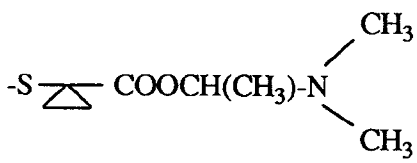 | |
| 2.132 | F | Cl | 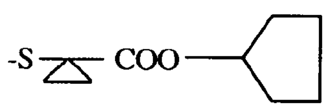 | |
| 2.133 | F | Cl | 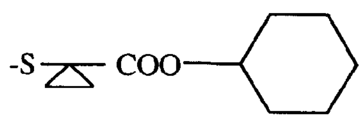 | |
| 2.134 | F | Cl | 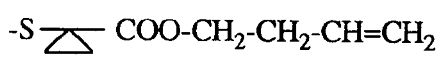 | |
| 2.135 | F | Cl | 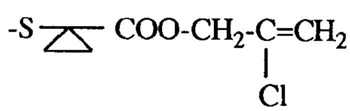 | |
| 2.136 | F | Cl | 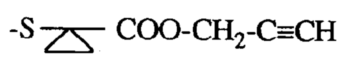 | |
| 2.137 | F | Cl | 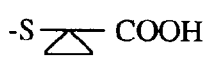 | |
| 2.138 | F | Cl | 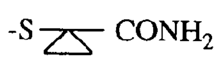 | |
| 2.139 | F | Cl | 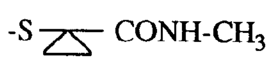 | |
| 2.140 | F | Cl | 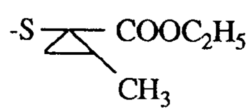 | |
| 2.141 | F | Cl | 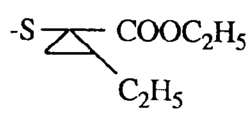 | |

| Verb. Nr. | $R_1$ | $R_2$ | A | Phys. Daten |
|---|---|---|---|---|
| 2.142 | F | Cl | 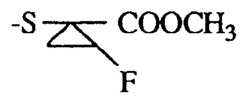 | |
| 2.143 | F | Cl | 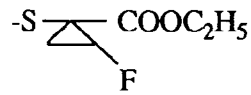 | |
| 2.144 | F | Cl | 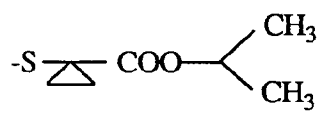 | |
| 2.145 | F | Cl | 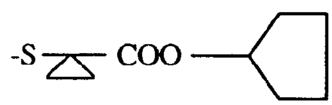 | |
| 2.146 | F | Cl | $-NH-SO_2-CH_3$ | |
| 2.147 | F | Cl | $-NH-SO_2-C_2H_5$ | |
| 2.148 | F | Cl | $-NH-SO_2-Cl$ | |
| 2.149 | F | Cl | 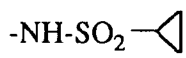 | |
| 2.150 | F | Cl | 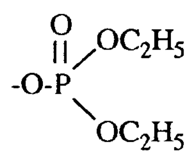 | |
| 2.151 | H | Cl | -COOH | |
| 2.152 | H | Cl | $-COOCH_3$ | |
| 2.153 | H | Cl | 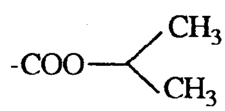 | |
| 2.154 | H | Cl | $-COO-C_5H_{11}$ | |
| 2.155 | H | Cl | $-COO-CH_2-CH_2-O-CH_3$ | |
| 2.156 | H | Cl | $-COOCH_2-S-CH_3$ | |
| 2.157 | H | Cl | $-COOCH(CH_3)-CH_2-S-CH_3$ | |
| 2.158 | H | Cl | 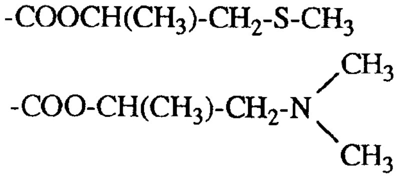 | |

| Verb. Nr. | $R_1$ | $R_2$ | A | Phys. Daten |
|---|---|---|---|---|
| 2.159 | H | Cl | 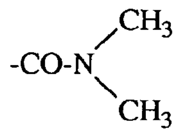 | |
| 2.160 | H | Cl | 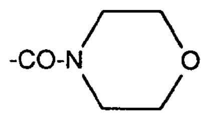 | |
| 2.161 | H | Cl | 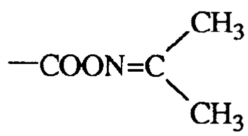 | |
| 2.162 | H | Cl | 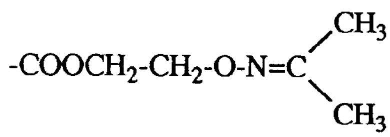 | |
| 2.163 | H | Cl | 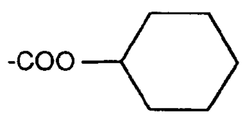 | |
| 2.164 | H | Cl | 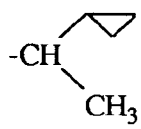 | |
| 2.165 | H | Cl | $-S-C_3H_7$ | |
| 2.166 | H | Cl | $-COOCH_2-COOCH_3$ | |
| 2.167 | H | Cl | $-COOCH(CH_3)-COOCH_3$ | |
| 2.168 | H | Cl | $-COS-CH_2-COOCH_3$ | |
| 2.169 | H | Cl | $-COS-CH(CH_3)-COOCH_3$ | |
| 2.170 | H | Cl | -OH | |
| 2.171 | H | Cl | $-OCH_3$ | |
| 2.172 | H | Cl | $-O-C_2H_5$ | |
| 2.173 | H | Cl | 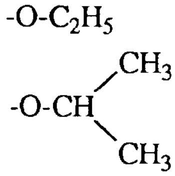 | |
| 2.174 | H | Cl | $-O-CH_2-C\equiv CH$ | |

| Verb. Nr. | $R_1$ | $R_2$ | A | Phys. Daten |
|---|---|---|---|---|
| 2.175 | H | Cl | $-O-CH_2-CH=CHCl$ | |
| 2.176 | H | Cl | 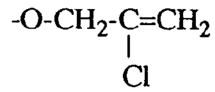 | |
| 2.177 | H | Cl | 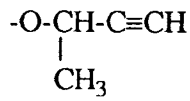 | |
| 2.178 | H | Cl | $-O-CH_2-COOCH_3$ | |
| 2.179 | H | Cl | $-O-CH_2-COOC_2H_5$ | |
| 2.180 | H | Cl | $-O-CH(CH_3)-COOCH_3$ | |
| 2.181 | H | Cl | $-SH$ | |
| 2.182 | H | Cl | $-SCH_3$ | |
| 2.183 | H | Cl | $-SC_2H_5$ | |
| 2.184 | H | Cl | 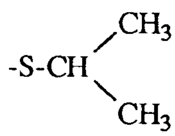 | |
| 2.185 | H | Cl | $-S-CH_2-COOCH_3$ | |
| 2.186 | H | Cl | $-S-CH(CH_3)-COOCH_3$ | |
| 2.187 | H | Cl | $-S-CH_2-COOC_2H_5$ | |
| 2.188 | H | Cl | 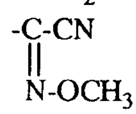 | |
| 2.189 | H | Cl | 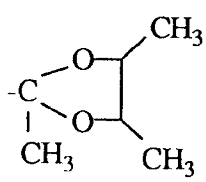 | |
| 2.190 | H | Cl | 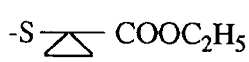 | |
| 2.191 | H | Cl | 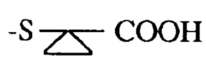 | |
| 2.192 | H | Cl | 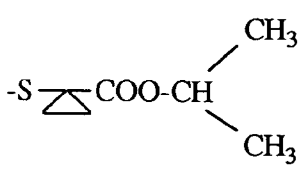 | |

| Verb. Nr. | $R_1$ | $R_2$ | A | Phys. Daten |
|---|---|---|---|---|
| 2.193 | H | Cl | 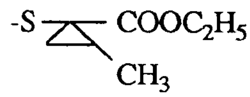 | |
| 2.194 | H | Cl | 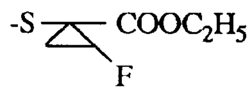 | |
| 2.195 | H | Cl | 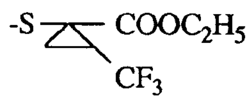 | |
| 2.196 | H | Cl | 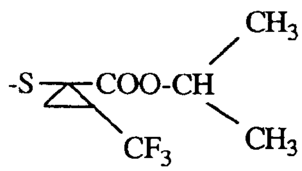 | |
| 2.197 | H | Cl | 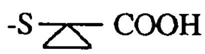 | |
| 2.198 | H | Cl | 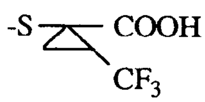 | |
| 2.199 | H | Cl | 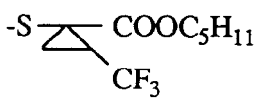 | |
| 2.200 | H | Cl | 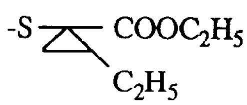 | |
| 2.201 | H | Cl | 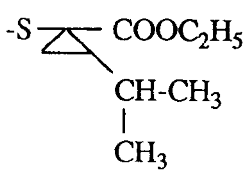 | |
| 2.202 | H | Cl | $-NH-SO_2-C_2H_5$ | |
| 2.203 | H | Cl | $-NH-SO_2-CH_2-Cl$ | |
| 2.204 | F | CN | -COOH | |
| 2.205 | F | CN | 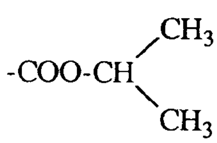 | |

| Verb. Nr. | $R_1$ | $R_2$ | A | Phys. Daten |
|---|---|---|---|---|
| 2.206 | F | CN | 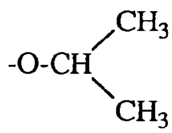 | |
| 2.207 | F | CN | $-O-CH_2-C{\equiv}CH$ | |
| 2.208 | F | CN | $-O-CH(CH_3)-C{\equiv}CH$ | |
| 2.209 | F | CN | $-S-CH_2-COOCH_3$ | |
| 2.210 | F | CN | $-S-CH(CH_3)-COOCH_3$ | |
| 2.211 | F | CN | $-O-CH_2-COOCH_3$ | |
| 2.212 | F | CN | $-O-CH_2-COOC_5H_{11}$ | |
| 2.213 | F | CN | $-O-CH(CH_3)-COOC_2H_5$ | |
| 2.214 | F | CN | 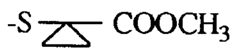 | |
| 2.215 | F | CN | 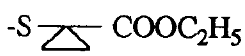 | |
| 2.216 | F | CN | 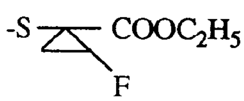 | |
| 2.217 | F | CN | 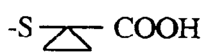 | |
| 2.218 | F | CN | 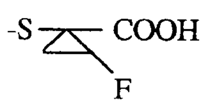 | |
| 2.219 | F | CN | 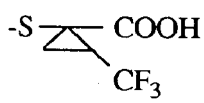 | |
| 2.220 | F | CN | 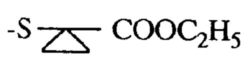 | |
| 2.221 | F | Br | -COOH | |
| 2.222 | F | Br | 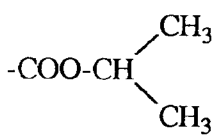 | |
| 2.223 | F | Br | -OH | |

| Verb. Nr. | $R_1$ | $R_2$ | A | Phys. Daten |
|---|---|---|---|---|
| 2.224 | F | Br | 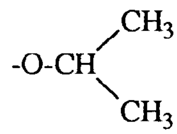 | |
| 2.225 | F | Br | $-O-CH_2-C\equiv CH$ | |
| 2.226 | F | Br | $-O-CH(CH_3)-C\equiv CH$ | |
| 2.227 | F | Br | $-O-CH_2COOCH_3$ | |
| 2.228 | F | Br | $-O-CH_2-COOC_5H_{11}$ | |
| 2.229 | F | Br | $-S-CH_2-COOCH_3$ | |
| 2.230 | F | Br | 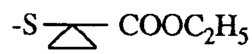 | |
| 2.231 | F | Br | 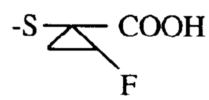 | |
| 2.232 | F | Br | 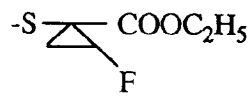 | |
| 2.233 | F | Cl | $-S-CH_2COOH$ | |

Tabelle 3: Verbindungen der Formel Ie:

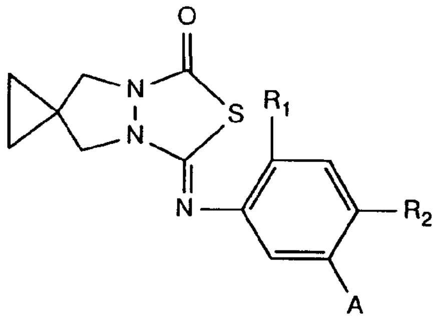

(Ie)

| Verb. Nr. | $R_1$ | $R_2$ | A | Phys. Daten |
|---|---|---|---|---|
| 3.001 | F | Cl | -H | |
| 3.002 | F | Cl | -CN | |
| 3.003 | F | Cl | $-NO_2$ | |
| 3.004 | F | Cl | -COOH | |
| 3.005 | F | Cl | $-COOCH_3$ | Smp. 118-119°C |
| 3.006 | F | Cl | $-COOC_2H_5$ | |
| 3.007 | F | Cl | $-COOC_3H_7$ | |
| 3.008 | F | Cl | $-COOCH(CH_3)_2$ | |
| 3.009 | F | Cl | $-COOC_4H_9$ | |
| 3.010 | F | Cl | $-COOCH-CH_2-CH_3$<br>$CH_3$ | |
| 3.011 | F | Cl | $CH_3$<br>$-COOCH_2-CH_2-CH$<br>$CH_3$ | |
| 3.012 | F | Cl | $-COOC_5H_{11}$ | |
| 3.013 | F | Cl | $-COOCH_2-CH_2-O-CH_3$ | |
| 3.014 | F | Cl | $-COOCH_2-CH_2-O-C_2H_5$ | |
| 3.015 | F | Cl | $-COOCH(CH_3)-CH_2-OCH_3$ | |
| 3.016 | F | Cl | $-COOCH_2-CH_2-S-CH_3$ | |
| 3.017 | F | Cl | $-COOCH(CH_3)-CH_2-S-CH_3$ | |
| 3.018 | F | Cl | $-COOCH(CH_3)-CH_2-S-C_2H_5$ | |

| Verb. Nr. | $R_1$ | $R_2$ | A | Phys. Daten |
|---|---|---|---|---|
| 3.019 | F | Cl | $-COOCH(CH_3)-CH_2-S-C_3H_7$ | |
| 3.020 | F | Cl | 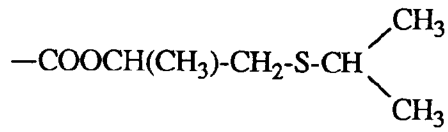 | |
| 3.021 | F | Cl | $-COOCH(CH_3)-CH_2-S-C_4H_9$ | |
| 3.022 | F | Cl | $-COOCH(CH_3)-CH_2-S-C_5H_{11}$ | |
| 3.023 | F | Cl | 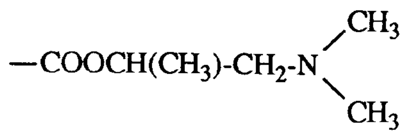 | |
| 3.024 | F | Cl | 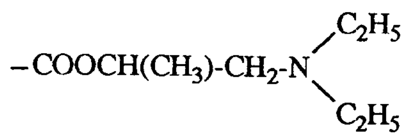 | |
| 3.025 | F | Cl | $-CONH_2$ | |
| 3.026 | F | Cl | $-CONH-CH_3$ | |
| 3.027 | F | Cl | 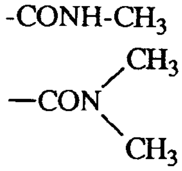 | |
| 3.028 | F | Cl | 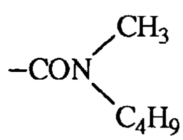 | |
| 3.029 | F | Cl | 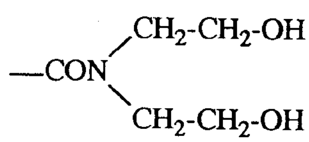 | |
| 3.030 | F | Cl | $-CONH-CH_2-CH=CH_2$ | |
| 3.031 | F | Cl | $-CON(CH_2-CH=CH_2)_2$ | |
| 3.032 | F | Cl | 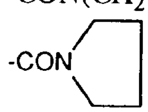 | |
| 3.033 | F | Cl | 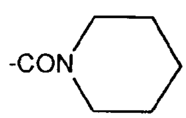 | |

| Verb. Nr. | $R_1$ | $R_2$ | A | Phys. Daten |
|---|---|---|---|---|
| 3.034 | F | Cl | 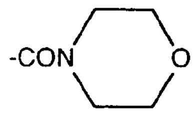 | |
| 3.035 | F | Cl | 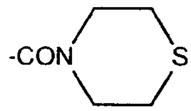 | |
| 3.036 | F | Cl | 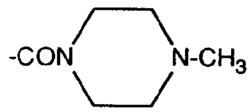 | |
| 3.037 | F | Cl | 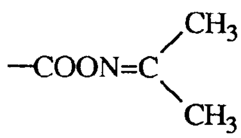 | |
| 3.038 | F | Cl | $-COOCH_2-CH_2-Cl$ | |
| 3.039 | F | Cl | $-COOCH_2-CN$ | |
| 3.040 | F | Cl | 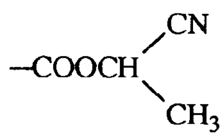 | |
| 3.041 | F | Cl | $-COOCH_2-CH=CH_2$ | |
| 3.042 | F | Cl | $-COOCH_2-CH=CHCl$ | |
| 3.043 | F | Cl | 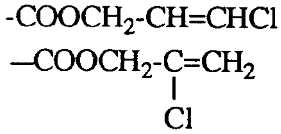 | |
| 3.044 | F | Cl | $-COOCH_2-C\equiv CH$ | |
| 3.045 | F | Cl | 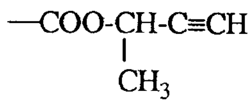 | |
| 3.046 | F | Cl | 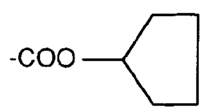 | |
| 3.047 | F | Cl | 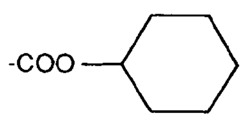 | |

| Verb. Nr. | $R_1$ | $R_2$ | A | Phys. Daten |
|---|---|---|---|---|
| 3.048 | F | Cl | 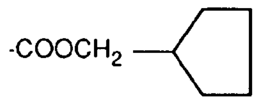 | |
| 3.049 | F | Cl | 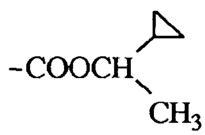 | |
| 3.050 | F | Cl | 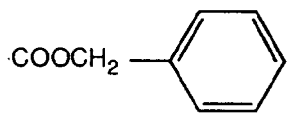 | |
| 3.051 | F | Cl | 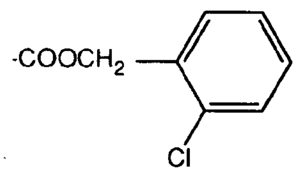 | |
| 3.052 | F | Cl | 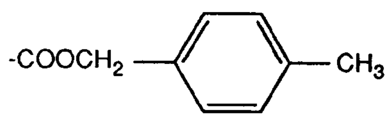 | |
| 3.053 | F | Cl | $-COSCH_3$ | |
| 3.054 | F | Cl | $-COSC_2H_5$ | |
| 3.055 | F | Cl | $-COSC_3H_7$ | |
| 3.056 | F | Cl | $-COS-CH_2-CH=CH_2$ | |
| 3.057 | F | Cl | $-COS-CH_2-COOCH_3$ | |
| 3.058 | F | Cl | $-COS-CH_2-COOC_2H_5$ | |
| 3.059 | F | Cl | $-COS-CH_2-COOC_5H_{11}$ | |
| 3.060 | F | Cl | $-COS-CH(CH_3)-COOCH_3$ | |
| 3.061 | F | Cl | $-COS-CH(CH_3)-COOC_2H_5$ | |
| 3.062 | F | Cl | $-COS-CH(CH_3)-COOC_3H_7$ | |

| Verb. Nr. | $R_1$ | $R_2$ | A | Phys. Daten |
|---|---|---|---|---|
| 3.063 | F | Cl | $-COS-CH_2-CH_2-COOCH_3$ | |
| 3.064 | F | Cl | $-COS-CH_2-COOCH_2-CH_2-O-CH_3$ | |
| 3.065 | F | Cl | $-COOCH_2-COOCH_3$ | |
| 3.066 | F | Cl | 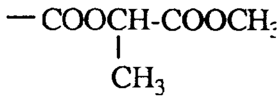 | |
| 3.067 | F | Cl | $-COOCH_2-COOC_5H_{11}$ | |
| 3.068 | F | Cl | $-COOCH_2-CH_2-Si(CH_3)_3$ | |
| 3.069 | F | Cl | -COONa | |
| 3.070 | F | Cl | 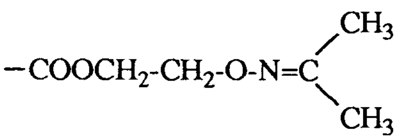 | |
| 3.071 | F | Cl | -OH | |
| 3.072 | F | Cl | $-OCH_3$ | |
| 3.073 | F | Cl | $-OC_2H_5$ | |
| 3.074 | F | Cl | $-OC_3H_7$ | |
| 3.075 | F | Cl | 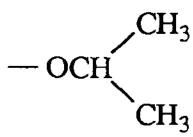 | Harz |
| 3.076 | F | Cl | $-OC_4H_9$ | |
| 3.077 | F | Cl | 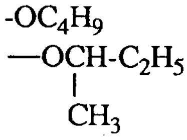 | |
| 3.078 | F | Cl | 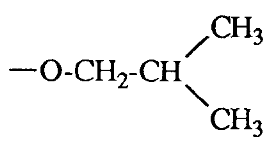 | |
| 3.079 | F | Cl | $-OCH_2CH=CH_2$ | |
| 3.080 | F | Cl | 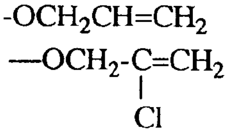 | |
| 3.081 | F | Cl | $-OCH_2CH=CHCl$ | |
| 3.082 | F | Cl | $-OCH_2C\equiv CH$ | |

| Verb. Nr. | $R_1$ | $R_2$ | A | Phys. Daten |
|---|---|---|---|---|
| 3.083 | F | Cl | $-OCH-C\equiv CH$<br>$CH_3$ | |
| 3.084 | F | Cl | $-OCH_2-COOCH_3$ | |
| 3.085 | F | Cl | $-O-CH_2-COOC_5H_{11}$ | |
| 3.086 | F | Cl | $-O-CH-COOCH_3$<br>$CH_3$ | |
| 3.087 | F | Cl | $-O-CH_2-COOC_2H_5$ | |
| 3.088 | F | Cl | $-O-CH-COOC_2H_5$<br>$CH_3$ | |
| 3.089 | F | Cl | $-O-CH_2-CH_2-O-CH_3$ | |
| 3.090 | F | Cl | $-O-CH-CH_2-S-CH_3$<br>$CH_3$ | |
| 3.091 | F | Cl | $-O-CH-CH_2-S-C_2H_5$<br>$CH_3$ | |
| 3.092 | F | Cl | $-O-CH-CH_2-S-C_3H_7$<br>$CH_3$ | |
| 3.093 | F | Cl | $-O-CH_2-CH_2-Cl$ | |
| 3.094 | F | Cl | $-O-CH_2-CN$ | |
| 3.095 | F | Cl | $-O-CH-CN$<br>$CH_3$ | |
| 3.096 | F | Cl | $-S-CH_3$ | |
| 3.097 | F | Cl | $-S-C_2H_5$ | |
| 3.098 | F | Cl | $-S-C_3H_7$ | |
| 3.099 | F | Cl | $CH_3$<br>$-S-CH$<br>$CH_3$ | |
| 3.100 | F | Cl | $-S-CH_2-CH=CH_2$ | |

| Verb. Nr. | $R_1$ | $R_2$ | A | Phys. Daten |
|---|---|---|---|---|
| 3.101 | F | Cl | $-S-CH_2-C=CH_2$<br>\|<br>Cl | |
| 3.102 | F | Cl | $-S-CH_2-CH=CHCl$ | |
| 3.103 | F | Cl | $-S-CH_2-C \equiv CH$ | |
| 3.104 | F | Cl | $-S-CH-C \equiv CH$<br>\|<br>$CH_3$ | |
| 3.105 | F | Cl | $-S-CH_2-COOCH_3$ | Smp. 63-65°C |
| 3.106 | F | Cl | $-S-CH_2-COOC_2H_5$ | |
| 3.107 | F | Cl | $-S-CH_2-COOC_5H_{11}$ | |
| 3.108 | F | Cl | $-S-CH-COOCH_3$<br>\|<br>$CH_3$ | |
| 3.109 | F | Cl | $-S-CH-COOC_2H_5$<br>\|<br>$CH_3$ | |
| 3.110 | F | Cl | $-S-CH_2-COOCH_2-CH_2-O-CH_3$ | |
| 3.111 | F | Cl | 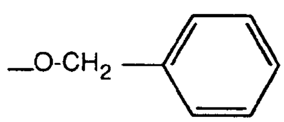 | |
| 3.112 | F | Cl | 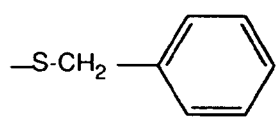 | |
| 3.113 | F | Cl | $-C-CN$<br>\|\|<br>$N-O-CH_3$ | |
| 3.114 | F | Cl | $-C-CN$<br>\|\|<br>$N-O-CH_2-COOCH_3$ | |
| 3.115 | F | Cl | $-C-CN$<br>\|\|<br>$N-O-CH_2-C \equiv CH$ | |

| Verb. Nr. | $R_1$ | $R_2$ | A | Phys. Daten |
|---|---|---|---|---|
| 3.116 | F | Cl | 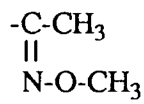 | |
| 3.117 | F | Cl | 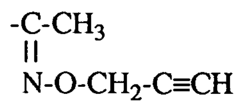 | |
| 3.118 | F | Cl | 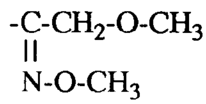 | |
| 3.119 | F | Cl | 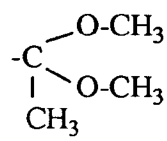 | |
| 3.120 | F | Cl | 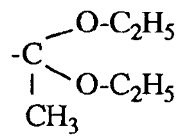 | |
| 3.121 | F | Cl | 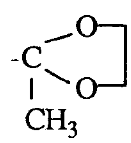 | |
| 3.122 | F | Cl | 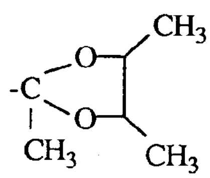 | |
| 3.123 | F | Cl | 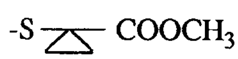 | |
| 3.124 | F | Cl | 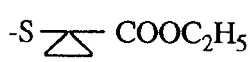 | |
| 3.125 | F | Cl | 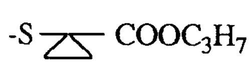 | |
| 3.126 | F | Cl | 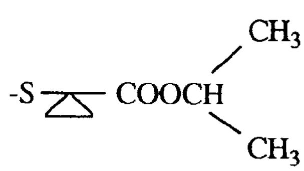 | |

| Verb. Nr. | $R_1$ | $R_2$ | A | Phys. Daten |
|---|---|---|---|---|
| 3.127 | F | Cl | 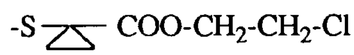 | |
| 3.128 | F | Cl | 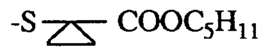 | |
| 3.129 | F | Cl | 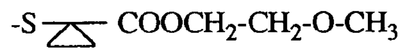 | |
| 3.130 | F | Cl | 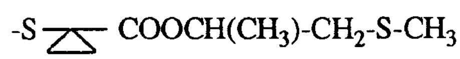 | |
| 3.131 | F | Cl | 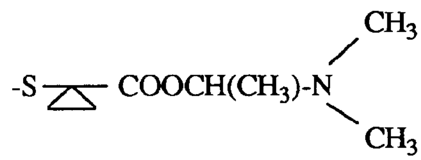 | |
| 3.132 | F | Cl | 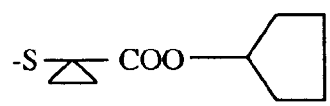 | |
| 3.133 | F | Cl | 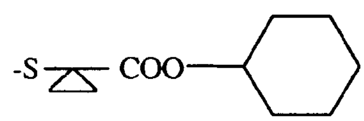 | |
| 3.134 | F | Cl | 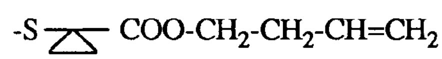 | |
| 3.135 | F | Cl | 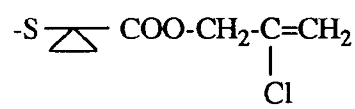 | |
| 3.136 | F | Cl | 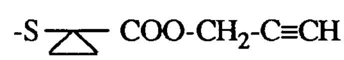 | |
| 3.137 | F | Cl | 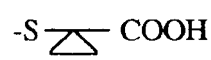 | |
| 3.138 | F | Cl | 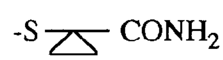 | |
| 3.139 | F | Cl | 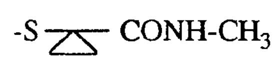 | |
| 3.140 | F | Cl | 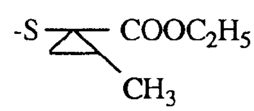 | |
| 3.141 | F | Cl | 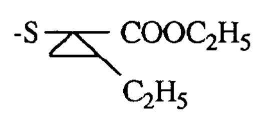 | |

| Verb. Nr. | $R_1$ | $R_2$ | A | Phys. Daten |
|---|---|---|---|---|
| 3.142 | F | Cl | 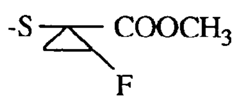 | |
| 3.143 | F | Cl | 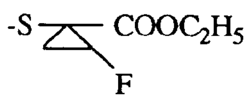 | |
| 3.144 | F | Cl | 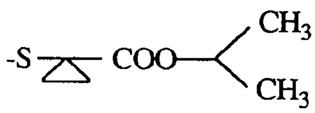 | |
| 3.145 | F | Cl | 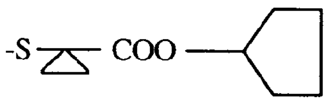 | |
| 3.146 | F | Cl | $-NH-SO_2-CH_3$ | |
| 3.147 | F | Cl | $-NH-SO_2-C_2H_5$ | |
| 3.148 | F | Cl | $-NH-SO_2-Cl$ | |
| 3.149 | F | Cl | 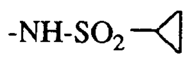 | |
| 3.150 | F | Cl | 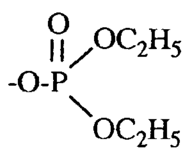 | |
| 3.151 | H | Cl | -COOH | |
| 3.152 | H | Cl | $-COOCH_3$ | Smp.> 85°C(Zers.) |
| 3.153 | H | Cl | 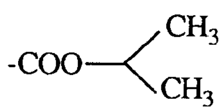 | Wachs |
| 3.154 | H | Cl | $-COO-C_5H_{11}$ | |
| 3.155 | H | Cl | $-COO-CH_2-CH_2-O-CH_3$ | |
| 3.156 | H | Cl | $-COOCH_2-S-CH_3$ | |
| 3.157 | H | Cl | $-COOCH(CH_3)-CH_2-S-CH_3$ | |
| 3.158 | H | Cl | 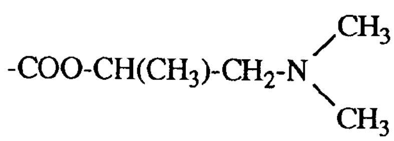 | |

| Verb. Nr. | $R_1$ | $R_2$ | A | Phys. Daten |
|---|---|---|---|---|
| 3.159 | H | Cl | 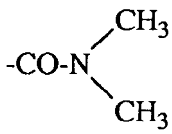 | |
| 3.160 | H | Cl | 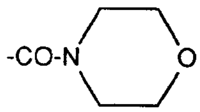 | |
| 3.161 | H | Cl | 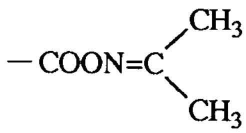 | |
| 3.162 | H | Cl | 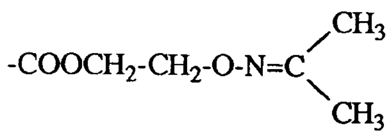 | |
| 3.163 | H | Cl | 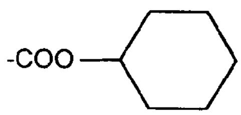 | |
| 3.164 | H | Cl | 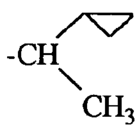 | |
| 3.165 | H | Cl | $-S-C_3H_7$ | |
| 3.166 | H | Cl | $-COOCH_2-COOCH_3$ | |
| 3.167 | H | Cl | $-COOCH(CH_3)-COOCH_3$ | |
| 3.168 | H | Cl | $-COS-CH_2-COOCH_3$ | |
| 3.169 | H | Cl | $-COS-CH(CH_3)-COOCH_3$ | |
| 3.170 | H | Cl | -OH | |
| 3.171 | H | Cl | $-OCH_3$ | |
| 3.172 | H | Cl | $-O-C_2H_5$ | |
| 3.173 | H | Cl | 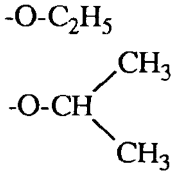 | |
| 3.174 | H | Cl | $-O-CH_2-C{\equiv}CH$ | |

| Verb. Nr. | $R_1$ | $R_2$ | A | Phys. Daten |
|---|---|---|---|---|
| 3.175 | H | Cl | $-O-CH_2-CH=CHCl$ | |
| 3.176 | H | Cl | 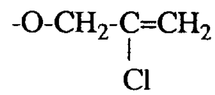 | |
| 3.177 | H | Cl | 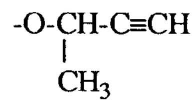 | |
| 3.178 | H | Cl | $-O-CH_2-COOCH_3$ | |
| 3.179 | H | Cl | $-O-CH_2-COOC_2H_5$ | |
| 3.180 | H | Cl | $-O-CH(CH_3)-COOCH_3$ | |
| 3.181 | H | Cl | -SH | |
| 3.182 | H | Cl | $-SCH_3$ | |
| 3.183 | H | Cl | $-SC_2H_5$ | |
| 3.184 | H | Cl | 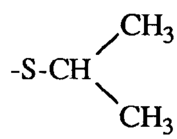 | |
| 3.185 | H | Cl | $-S-CH_2-COOCH_3$ | |
| 3.186 | H | Cl | $-S-CH(CH_3)-COOCH_3$ | |
| 3.187 | H | Cl | $-S-CH_2-COOC_2H_5$ | |
| 3.188 | H | Cl | 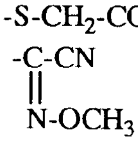 | |
| 3.189 | H | Cl | 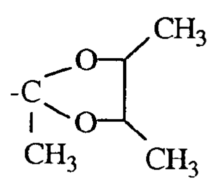 | |
| 3.190 | H | Cl | 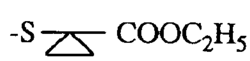 | |
| 3.191 | H | Cl | 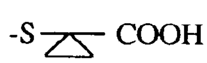 | |
| 3.192 | H | Cl | 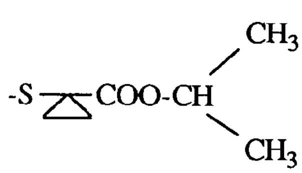 | |

| Verb. Nr. | $R_1$ | $R_2$ | A | Phys. Daten |
|---|---|---|---|---|
| 3.193 | H | Cl | 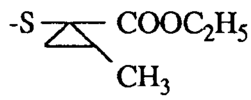 | |
| 3.194 | H | Cl | 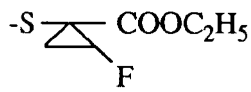 | |
| 3.195 | H | Cl | 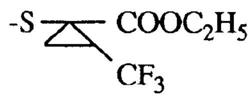 | |
| 3.196 | H | Cl | 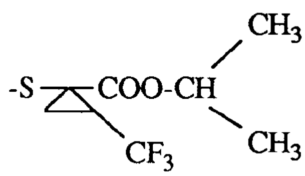 | |
| 3.197 | H | Cl | 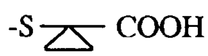 | |
| 3.198 | H | Cl | 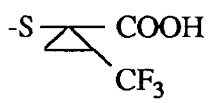 | |
| 3.199 | H | Cl | 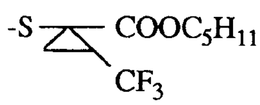 | |
| 3.200 | H | Cl | 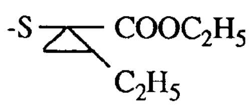 | |
| 3.201 | H | Cl | 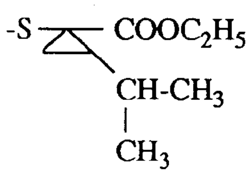 | |
| 3.202 | H | Cl | $-NH-SO_2-C_2H_5$ | |
| 3.203 | H | Cl | $-NH-SO_2-CH_2-Cl$ | |
| 3.204 | F | CN | -COOH | |
| 3.205 | F | CN | 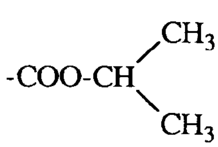 | |

| Verb. Nr. | $R_1$ | $R_2$ | A | Phys. Daten |
|---|---|---|---|---|
| 3.206 | F | CN | 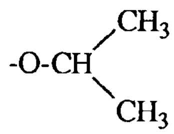 | |
| 3.207 | F | CN | $-O-CH_2-C{\equiv}CH$ | |
| 3.208 | F | CN | $-O-CH(CH_3)-C{\equiv}CH$ | |
| 3.209 | F | CN | $-S-CH_2-COOCH_3$ | |
| 3.210 | F | CN | $-S-CH(CH_3)-COOCH_3$ | |
| 3.211 | F | CN | $-O-CH_2-COOCH_3$ | |
| 3.212 | F | CN | $-O-CH_2-COOC_5H_{11}$ | |
| 3.213 | F | CN | $-O-CH(CH_3)-COOC_2H_5$ | |
| 3.214 | F | CN | 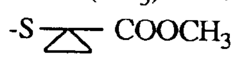 | |
| 3.215 | F | CN | 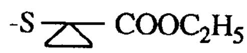 | |
| 3.216 | F | CN | 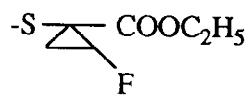 | |
| 3.217 | F | CN | 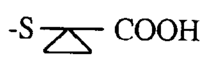 | |
| 3.218 | F | CN | 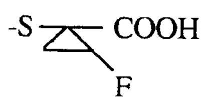 | |
| 3.219 | F | CN | 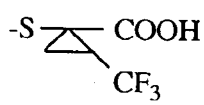 | |
| 3.220 | F | CN | 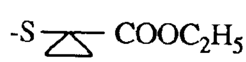 | |
| 3.221 | F | Br | -COOH | |
| 3.222 | F | Br | 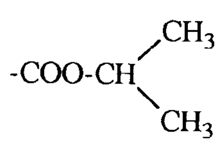 | |
| 3.223 | F | Br | -OH | |

| Verb. Nr. | $R_1$ | $R_2$ | A | Phys. Daten |
|---|---|---|---|---|
| 3.224 | F | Br | 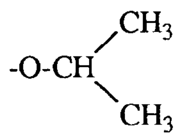 | |
| 3.225 | F | Br | $-O-CH_2-C\equiv CH$ | |
| 3.226 | F | Br | $-O-CH(CH_3)-C\equiv CH$ | |
| 3.227 | F | Br | $-O-CH_2COOCH_3$ | |
| 3.228 | F | Br | $-O-CH_2-COOC_5H_{11}$ | |
| 3.229 | F | Br | $S-CH_2-COOCH_3$ | |
| 3.230 | F | Br | 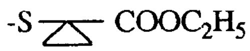 | |
| 3.231 | F | Br | 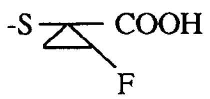 | |
| 3.232 | F | Br | 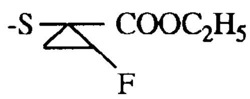 | |
| 3.233 | F | Cl | $-S-CH_2COOH$ | |

Tabelle 4: Verbindungen der Formel If

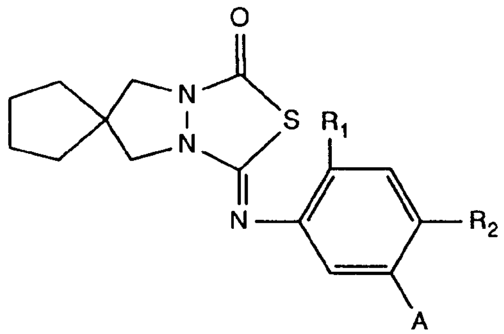

(If)

| Verb. Nr. | $R_1$ | $R_2$ | A | Phys. Daten |
|---|---|---|---|---|
| 4.001 | F | Cl | -H | |
| 4.002 | F | Cl | -CN | |
| 4.003 | F | Cl | $-NO_2$ | |
| 4.004 | F | Cl | -COOH | |
| 4.005 | F | Cl | $-COOCH_3$ | |
| 4.006 | F | Cl | $-COOC_2H_5$ | |
| 4.007 | F | Cl | $-COOC_3H_7$ | |
| 4.008 | F | Cl | $-COOCH(CH_3)_2$ | |
| 4.009 | F | Cl | $-COOC_4H_9$ | |
| 4.010 | F | Cl | $-COOCH-CH_2-CH_3$<br>$\vert$<br>$CH_3$ | |
| 4.011 | F | Cl | $CH_3$<br>/<br>$-COOCH_2-CH_2-CH$<br>\\<br>$CH_3$ | |
| 4.012 | F | Cl | $-COOC_5H_{11}$ | |
| 4.013 | F | Cl | $-COOCH_2-CH_2-O-CH_3$ | |
| 4.014 | F | Cl | $-COOCH_2-CH_2-O-C_2H_5$ | |
| 4.015 | F | Cl | $-COOCH(CH_3)-CH_2-OCH_3$ | |
| 4.016 | F | Cl | $-COOCH_2-CH_2-S-CH_3$ | |
| 4.017 | F | Cl | $-COOCH(CH_3)-CH_2-S-CH_3$ | |

| Verb. Nr. | $R_1$ | $R_2$ | A | Phys. Daten |
|---|---|---|---|---|
| 4.018 | F | Cl | $-COOCH(CH_3)-CH_2-S-C_2H_5$ | |
| 4.019 | F | Cl | $-COOCH(CH_3)-CH_2-S-C_3H_7$ | |
| 4.020 | F | Cl | 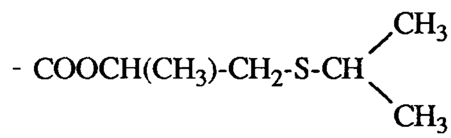 | |
| 4.021 | F | Cl | $-COOCH(CH_3)-CH_2-S-C_4H_9$ | |
| 4.022 | F | Cl | $-COOCH(CH_3)-CH_2-S-C_5H_{11}$ | |
| 4.023 | F | Cl | 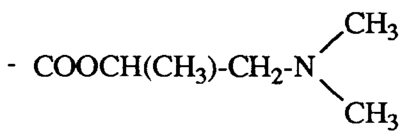 | |
| 4.024 | F | Cl | 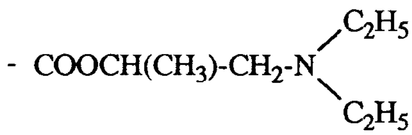 | |
| 4.025 | F | Cl | $-CONH_2$ | |
| 4.026 | F | Cl | $-CONH-CH_3$ | |
| 4.027 | F | Cl | 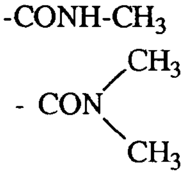 | |
| 4.028 | F | Cl | 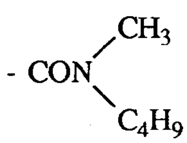 | |
| 4.029 | F | Cl | 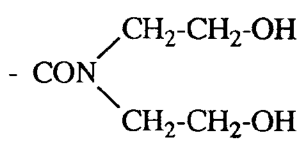 | |
| 4.030 | F | Cl | $-CONH-CH_2-CH=CH_2$ | |
| 4.031 | F | Cl | $-CON(CH_2-CH=CH_2)_2$ | |
| 4.032 | F | Cl | 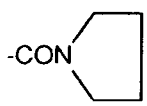 | |

| Verb. Nr. | $R_1$ | $R_2$ | A | Phys. Daten |
|---|---|---|---|---|
| 4.033 | F | Cl | 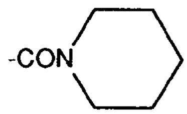 | |
| 4.034 | F | Cl | 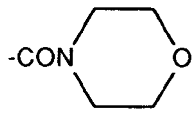 | |
| 4.035 | F | Cl | 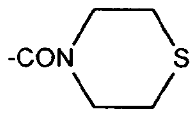 | |
| 4.036 | F | Cl | 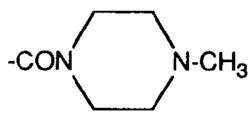 | |
| 4.037 | F | Cl | 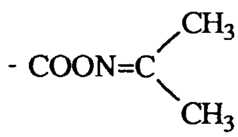 | |
| 4.038 | F | Cl | $-COOCH_2-CH_2-Cl$ | |
| 4.039 | F | Cl | $-COOCH_2-CN$ | |
| 4.040 | F | Cl | 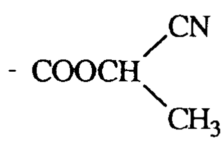 | |
| 4.041 | F | Cl | $-COOCH_2-CH=CH_2$ | |
| 4.042 | F | Cl | $-COOCH_2-CH=CHCl$ | |
| 4.043 | F | Cl | 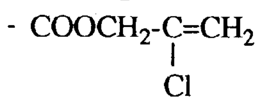 | |
| 4.044 | F | Cl | $-COOCH_2-C\equiv CH$ | |
| 4.045 | F | Cl | 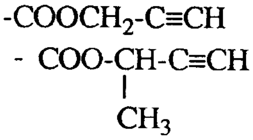 | |
| 4.046 | F | Cl | 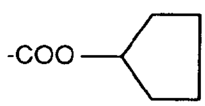 | |

| Verb. Nr. | $R_1$ | $R_2$ | A | Phys. Daten |
|---|---|---|---|---|
| 4.047 | F | Cl | 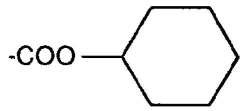 | |
| 4.048 | F | Cl | 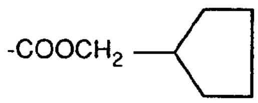 | |
| 4.049 | F | Cl | 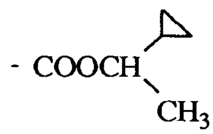 | |
| 4.050 | F | Cl | 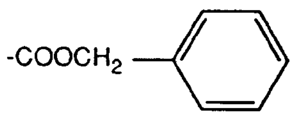 | |
| 4.051 | F | Cl | 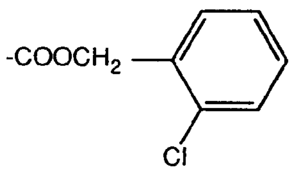 | |
| 4.052 | F | Cl | 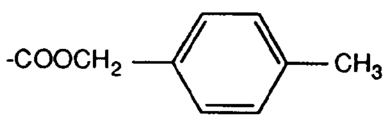 | |
| 4.053 | F | Cl | $-COSCH_3$ | |
| 4.054 | F | Cl | $-COSC_2H_5$ | |
| 4.055 | F | Cl | $-COSC_3H_7$ | |
| 4.056 | F | Cl | $-COS-CH_2-CH=CH_2$ | |
| 4.057 | F | Cl | $-COS-CH_2-COOCH_3$ | |
| 4.058 | F | Cl | $-COS-CH_2-COOC_2H_5$ | |
| 4.059 | F | Cl | $-COS-CH_2-COOC_5H_{11}$ | |
| 4.060 | F | Cl | $-\ COS-CH-COOCH_3$<br>\|<br>$CH_3$ | |
| 4.061 | F | Cl | $-\ COS-CH-COOC_2H_5$<br>\|<br>$CH_3$ | |

| Verb. Nr. | $R_1$ | $R_2$ | A | Phys. Daten |
|---|---|---|---|---|
| 4.062 | F | Cl | 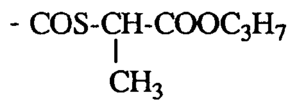 | |
| 4.063 | F | Cl | $-COS-CH_2-CH_2-COOCH_3$ | |
| 4.064 | F | Cl | $-COS-CH_2-COOCH_2-CH_2-O-CH_3$ | |
| 4.065 | F | Cl | $-COOCH_2-COOCH_3$ | |
| 4.066 | F | Cl | 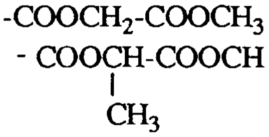 | |
| 4.067 | F | Cl | $-COOCH_2-COOC_5H_{11}$ | |
| 4.068 | F | Cl | $-COOCH_2-CH_2-Si(CH_3)_3$ | |
| 4.069 | F | Cl | -COONa | |
| 4.070 | F | Cl | 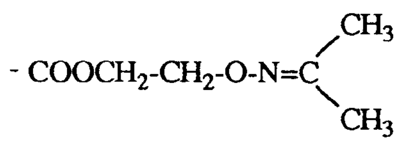 | |
| 4.071 | F | Cl | -OH | |
| 4.072 | F | Cl | $-OCH_3$ | |
| 4.073 | F | Cl | $-OC_2H_5$ | |
| 4.074 | F | Cl | $-OC_3H_7$ | |
| 4.075 | F | Cl | 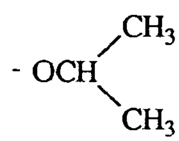 | |
| 4.076 | F | Cl | $-OC_4H_9$ | |
| 4.077 | F | Cl | 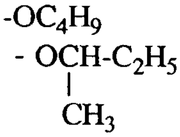 | |
| 4.078 | F | Cl | 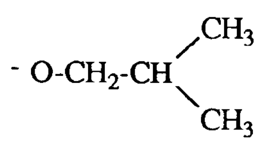 | |
| 4.079 | F | Cl | $-OCH_2CH=CH_2$ | |
| 4.080 | F | Cl | 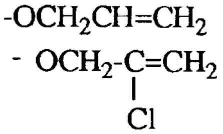 | |

| Verb. Nr. | $R_1$ | $R_2$ | A | Phys. Daten |
|---|---|---|---|---|
| 4.081 | F | Cl | $-OCH_2CH=CHCl$ | |
| 4.082 | F | Cl | $-OCH_2C\equiv CH$ | |
| 4.083 | F | Cl | $-OCH-C\equiv CH$<br>$CH_3$ | |
| 4.084 | F | Cl | $-OCH_2-COOCH_3$ | |
| 4.085 | F | Cl | $-O-CH_2-COOC_5H_{11}$ | |
| 4.086 | F | Cl | $-O-CH-COOCH_3$<br>$CH_3$ | |
| 4.087 | F | Cl | $-O-CH_2-COOC_2H_5$ | |
| 4.088 | F | Cl | $-O-CH-COOC_2H_5$<br>$CH_3$ | |
| 4.089 | F | Cl | $-O-CH_2-CH_2-O-CH_3$ | |
| 4.090 | F | Cl | $-O-CH-CH_2-S-CH_3$<br>$CH_3$ | |
| 4.091 | F | Cl | $-O-CH-CH_2-S-C_2H_5$<br>$CH_3$ | |
| 4.092 | F | Cl | $-O-CH-CH_2-S-C_3H_7$<br>$CH_3$ | |
| 4.093 | F | Cl | $-O-CH_2-CH_2-Cl$ | |
| 4.094 | F | Cl | $-O-CH_2-CN$ | |
| 4.095 | F | Cl | $-O-CH-CN$<br>$CH_3$ | |
| 4.096 | F | Cl | $-S-CH_3$ | |
| 4.097 | F | Cl | $-S-C_2H_5$ | |
| 4.098 | F | Cl | $-S-C_3H_7$ | |
| 4.099 | F | Cl | $CH_3$<br>$-S-CH$<br>$CH_3$ | |

| Verb. Nr. | $R_1$ | $R_2$ | A | Phys. Daten |
|---|---|---|---|---|
| 4.100 | F | Cl | $-S-CH_2-CH=CH_2$ | |
| 4.101 | F | Cl | $-S-CH_2-C=CH_2$<br>I<br>Cl | |
| 4.102 | F | Cl | $-S-CH_2-CH=CHCl$ | |
| 4.103 | F | Cl | $-S-CH_2-C{\equiv}CH$ | |
| 4.104 | F | Cl | $-S-CH-C{\equiv}CH$<br>I<br>$CH_3$ | |
| 4.105 | F | Cl | $-S-CH_2-COOCH_3$ | |
| 4.106 | F | Cl | $-S-CH_2-COOC_2H_5$ | |
| 4.107 | F | Cl | $-S-CH_2-COOC_5H_{11}$ | |
| 4.108 | F | Cl | $-S-CH-COOCH_3$<br>I<br>$CH_3$ | |
| 4.109 | F | Cl | $-S-CH-COOC_2H_5$<br>I<br>$CH_3$ | |
| 4.110 | F | Cl | $-S-CH_2-COOCH_2-CH_2-O-CH_3$ | |
| 4.111 | F | Cl | 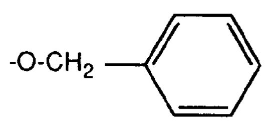 | |
| 4.112 | F | Cl | 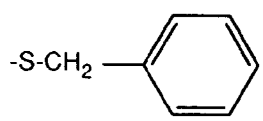 | |
| 4.113 | F | Cl | -C-CN<br>II<br>$N-O-CH_3$ | |
| 4.114 | F | Cl | -C-CN<br>II<br>$N-O-CH_2-COOCH_3$ | |
| 4.115 | F | Cl | -C-CN<br>II<br>$N-O-CH_2-C{\equiv}CH$ | |

| Verb. Nr. | $R_1$ | $R_2$ | A | Phys. Daten |
|---|---|---|---|---|
| 4.116 | F | Cl | 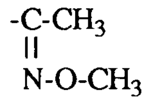 | |
| 4.117 | F | Cl | 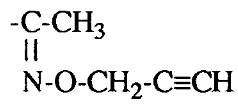 | |
| 4.118 | F | Cl | 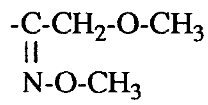 | |
| 4.119 | F | Cl | 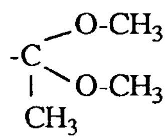 | |
| 4.120 | F | Cl | 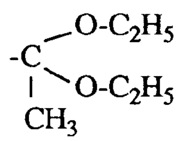 | |
| 4.121 | F | Cl | 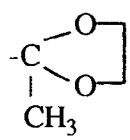 | |
| 4.122 | F | Cl | 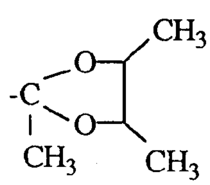 | |
| 4.123 | F | Cl | 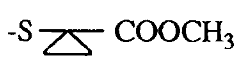 | |
| 4.124 | F | Cl | 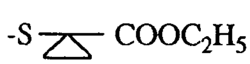 | |
| 4.125 | F | Cl | 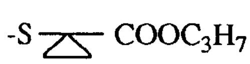 | |
| 4.126 | F | Cl | 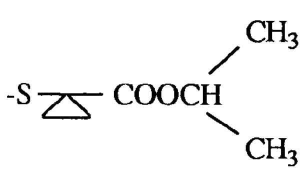 | |

| Verb. Nr. | $R_1$ | $R_2$ | A | Phys. Daten |
|---|---|---|---|---|
| 4.127 | F | Cl | -S 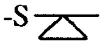 COO-$CH_2$-$CH_2$-Cl | |
| 4.128 | F | Cl | -S 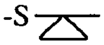 $COOC_5H_{11}$ | |
| 4.129 | F | Cl | -S 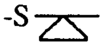 $COOCH_2$-$CH_2$-O-$CH_3$ | |
| 4.130 | F | Cl | -S 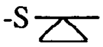 $COOCH(CH_3)$-$CH_2$-S-$CH_3$ | |
| 4.131 | F | Cl | 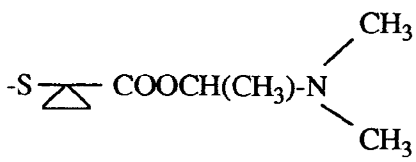 | |
| 4.132 | F | Cl | 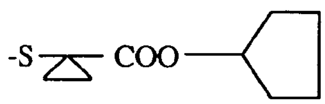 | |
| 4.133 | F | Cl | 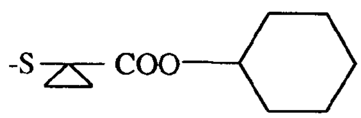 | |
| 4.134 | F | Cl | -S 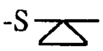 COO-$CH_2$-$CH_2$-CH=$CH_2$ | |
| 4.135 | F | Cl | 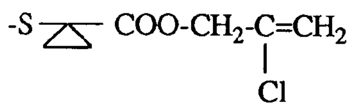 | |
| 4.136 | F | Cl | -S 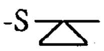 COO-$CH_2$-C≡CH | |
| 4.137 | F | Cl | -S 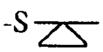 COOH | |
| 4.138 | F | Cl | -S 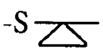 $CONH_2$ | |
| 4.139 | F | Cl | -S 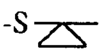 CONH-$CH_3$ | |
| 4.140 | F | Cl | 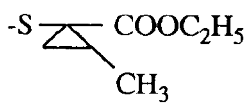 | |
| 4.141 | F | Cl | 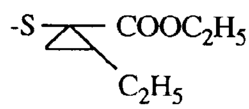 | |

| Verb. Nr. | $R_1$ | $R_2$ | A | Phys. Daten |
|---|---|---|---|---|
| 4.142 | F | Cl | 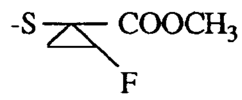 | |
| 4.143 | F | Cl | 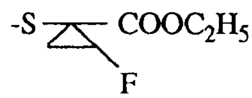 | |
| 4.144 | F | Cl | 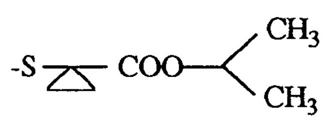 | |
| 4.145 | F | Cl | 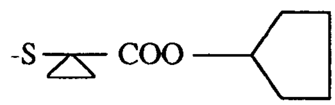 | |
| 4.146 | F | Cl | $-NH-SO_2-CH_3$ | |
| 4.147 | F | Cl | $-NH-SO_2-C_2H_5$ | |
| 4.148 | F | Cl | $-NH-SO_2-Cl$ | |
| 4.149 | F | Cl | 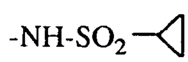 | |
| 4.150 | F | Cl | 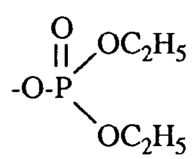 | |
| 4.151 | H | Cl | -COOH | |
| 4.152 | H | Cl | $-COOCH_3$ | |
| 4.153 | H | Cl | 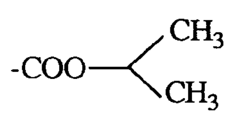 | |
| 4.154 | H | Cl | $-COO-C_5H_{11}$ | |
| 4.155 | H | Cl | $-COO-CH_2-CH_2-O-CH_3$ | |
| 4.156 | H | Cl | $-COOCH_2-S-CH_3$ | |
| 4.157 | H | Cl | $-COOCH(CH_3)-CH_2-S-CH_3$ | |
| 4.158 | H | Cl | 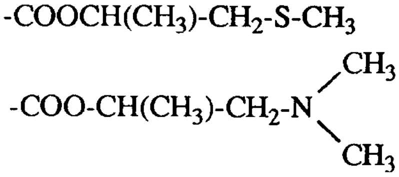 | |

| Verb. Nr. | $R_1$ | $R_2$ | A | Phys. Daten |
|---|---|---|---|---|
| 4.159 | H | Cl | 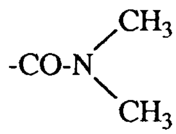 | |
| 4.160 | H | Cl | 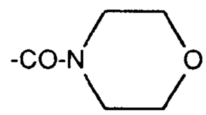 | |
| 4.161 | H | Cl | 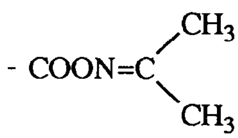 | |
| 4.162 | H | Cl | 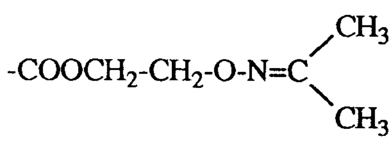 | |
| 4.163 | H | Cl | 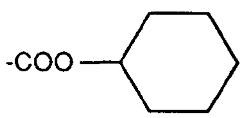 | |
| 4.164 | H | Cl | 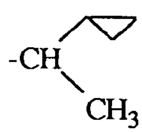 | |
| 4.165 | H | Cl | $-S-C_3H_7$ | |
| 4.166 | H | Cl | $-COOCH_2-COOCH_3$ | |
| 4.167 | H | Cl | $-COOCH(CH_3)-COOCH_3$ | |
| 4.168 | H | Cl | $-COS-CH_2-COOCH_3$ | |
| 4.169 | H | Cl | $-COS-CH(CH_3)-COOCH_3$ | |
| 4.170 | H | Cl | -OH | |
| 4.171 | H | Cl | $-OCH_3$ | |
| 4.172 | H | Cl | $-O-C_2H_5$ | |
| 4.173 | H | Cl | 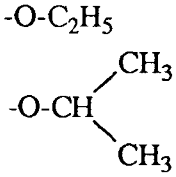 | |
| 4.174 | H | Cl | $-O-CH_2-C{\equiv}CH$ | |

| Verb. Nr. | $R_1$ | $R_2$ | A | Phys. Daten |
|---|---|---|---|---|
| 4.175 | H | Cl | $-O-CH_2-CH=CHCl$ | |
| 4.176 | H | Cl | 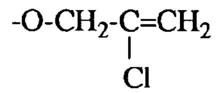 | |
| 4.177 | H | Cl | 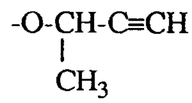 | |
| 4.178 | H | Cl | $-O-CH_2-COOCH_3$ | |
| 4.179 | H | Cl | $-O-CH_2-COOC_2H_5$ | |
| 4.180 | H | Cl | $-O-CH(CH_3)-COOCH_3$ | |
| 4.181 | H | Cl | -SH | |
| 4.182 | H | Cl | $-SCH_3$ | |
| 4.183 | H | Cl | $-SC_2H_5$ | |
| 4.184 | H | Cl | 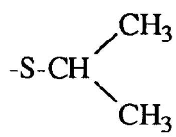 | |
| 4.185 | H | Cl | $-S-CH_2-COOCH_3$ | |
| 4.186 | H | Cl | $-S-CH(CH_3)-COOCH_3$ | |
| 4.187 | H | Cl | $-S-CH_2-COOC_2H_5$ | |
| 4.188 | H | Cl | 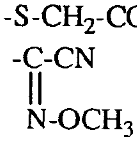 | |
| 4.189 | H | Cl | 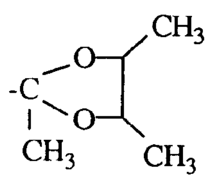 | |
| 4.190 | H | Cl | 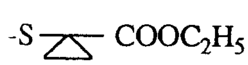 | |
| 4.191 | H | Cl | 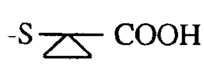 | |
| 4.192 | H | Cl | 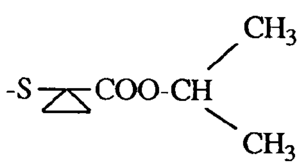 | |

| Verb. Nr. | $R_1$ | $R_2$ | A | Phys. Daten |
|---|---|---|---|---|
| 4.193 | H | Cl | 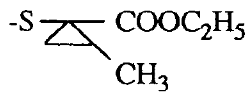 | |
| 4.194 | H | Cl | 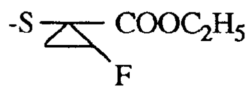 | |
| 4.195 | H | Cl | 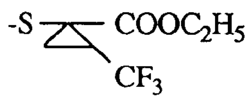 | |
| 4.196 | H | Cl | 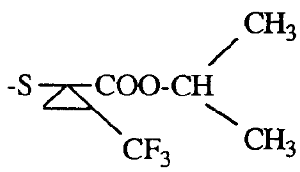 | |
| 4.197 | H | Cl | 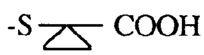 | |
| 4.198 | H | Cl | 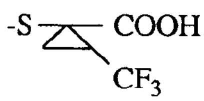 | |
| 4.199 | H | Cl | 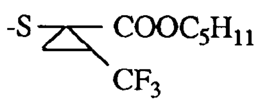 | |
| 4.200 | H | Cl | 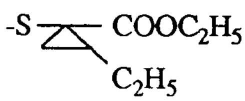 | |
| 4.201 | H | Cl | 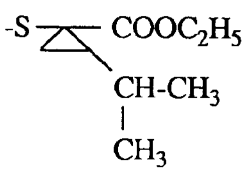 | |
| 4.202 | H | Cl | $-NH-SO_2-C_2H_5$ | |
| 4.203 | H | Cl | $-NH-SO_2-CH_2-Cl$ | |
| 4.204 | F | CN | -COOH | |
| 4.205 | F | CN | 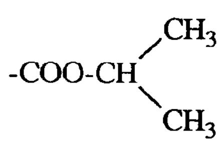 | |

| Verb. Nr. | $R_1$ | $R_2$ | A | Phys. Daten |
|---|---|---|---|---|
| 4.206 | F | CN | 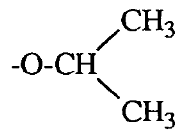 | |
| 4.207 | F | CN | $-O-CH_2-C\equiv CH$ | |
| 4.208 | F | CN | $-O-CH(CH_3)-C\equiv CH$ | |
| 4.209 | F | CN | $-S-CH_2-COOCH_3$ | |
| 4.210 | F | CN | $-S-CH(CH_3)-COOCH_3$ | |
| 4.211 | F | CN | $-O-CH_2-COOCH_3$ | |
| 4.212 | F | CN | $-O-CH_2-COOC_5H_{11}$ | |
| 4.213 | F | CN | $-O-CH(CH_3)-COOC_2H_5$ | |
| 4.214 | F | CN | 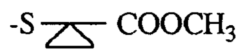 | |
| 4.215 | F | CN | 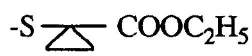 | |
| 4.216 | F | CN | 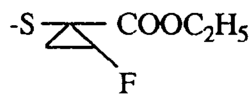 | |
| 4.217 | F | CN | 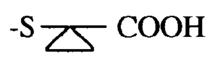 | |
| 4.218 | F | CN | 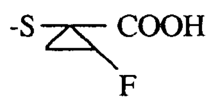 | |
| 4.219 | F | CN | 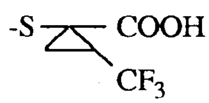 | |
| 4.220 | F | CN | 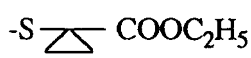 | |
| 4.221 | F | Br | -COOH | |
| 4.222 | F | Br | 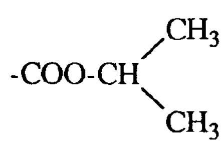 | |
| 4.223 | F | Br | -OH | |

| Verb. Nr. | $R_1$ | $R_2$ | A | Phys. Daten |
|---|---|---|---|---|
| 4.224 | F | Br | 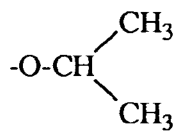 | |
| 4.225 | F | Br | $-O-CH_2-C{\equiv}CH$ | |
| 4.226 | F | Br | $-O-CH(CH_3)-C{\equiv}CH$ | |
| 4.227 | F | Br | $-O-CH_2COOCH_3$ | |
| 4.228 | F | Br | $-O-CH_2-COOC_5H_{11}$ | |
| 4.229 | F | Br | $-S-CH_2-COOCH_3$ | |
| 4.230 | F | Br | 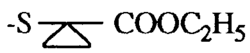 | |
| 4.231 | F | Br | 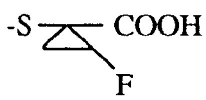 | |
| 4.232 | F | Br | 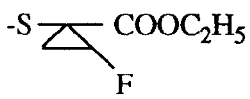 | |
| 4.233 | F | Cl | $-S-CH_2COOH$ | |

Tabelle 5: Verbindungen der Formel Ig

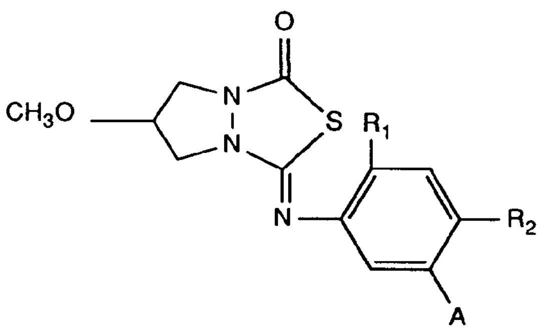

(Ig)

| Verb. Nr. | $R_1$ | $R_2$ | A | Phys. Daten |
|---|---|---|---|---|
| 5.001 | F | Cl | -H | |
| 5.002 | F | Cl | -CN | |
| 5.003 | F | Cl | $-NO_2$ | |
| 5.004 | F | Cl | -COOH | |
| 5.005 | F | Cl | $-COOCH_3$ | |
| 5.006 | F | Cl | $-COOC_2H_5$ | |
| 5.007 | F | Cl | $-COOC_3H_7$ | |
| 5.008 | F | Cl | $-COOCH(CH_3)_2$ | |
| 5.009 | F | Cl | $-COOC_4H_9$ | |
| 5.010 | F | Cl | 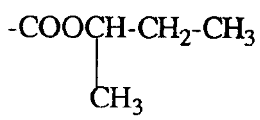 | |
| 5.011 | F | Cl | 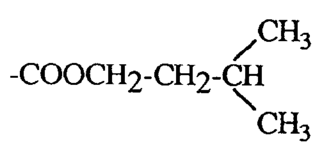 | |
| 5.012 | F | Cl | $-COOC_5H_{11}$ | |
| 5.013 | F | Cl | $-COOCH_2-CH_2-O-CH_3$ | |
| 5.014 | F | Cl | $-COOCH_2-CH_2-O-C_2H_5$ | |
| 5.015 | F | Cl | $-COOCH(CH_3)-CH_2-OCH_3$ | |
| 5.016 | F | Cl | $-COOCH_2-CH_2-S-CH_3$ | |
| 5.017 | F | Cl | $-COOCH(CH_3)-CH_2-S-CH_3$ | |

| Verb. Nr. | $R_1$ | $R_2$ | A | Phys. Daten |
|---|---|---|---|---|
| 5.018 | F | Cl | $-COOCH(CH_3)-CH_2-S-C_2H_5$ | |
| 5.019 | F | Cl | $-COOCH(CH_3)-CH_2-S-C_3H_7$ | |
| 5.020 | F | Cl | 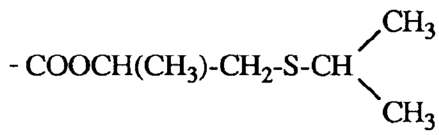 | |
| 5.021 | F | Cl | $-COOCH(CH_3)-CH_2-S-C_4H_9$ | |
| 5.022 | F | Cl | $-COOCH(CH_3)-CH_2-S-C_5H_{11}$ | |
| 5.023 | F | Cl | 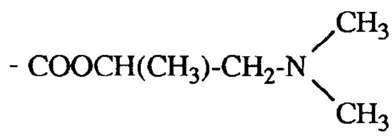 | |
| 5.024 | F | Cl | 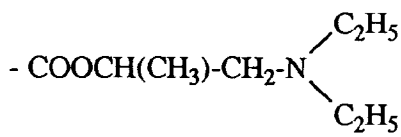 | |
| 5.025 | F | Cl | $-CONH_2$ | |
| 5.026 | F | Cl | $-CONH-CH_3$ | |
| 5.027 | F | Cl | 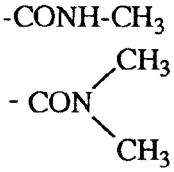 | |
| 5.028 | F | Cl | 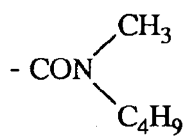 | |
| 5.029 | F | Cl | 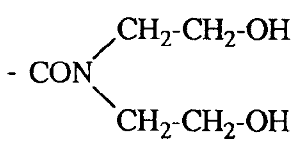 | |
| 5.030 | F | Cl | $-CONH-CH_2-CH=CH_2$ | |
| 5.031 | F | Cl | $-CON(CH_2-CH=CH_2)_2$ | |
| 5.032 | F | Cl | 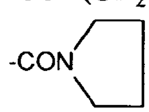 | |

| Verb. Nr. | $R_1$ | $R_2$ | A | Phys. Daten |
|---|---|---|---|---|
| 5.033 | F | Cl | 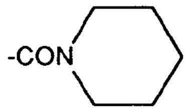 | |
| 5.034 | F | Cl | 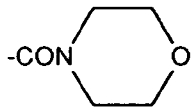 | |
| 5.035 | F | Cl | 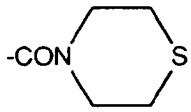 | |
| 5.036 | F | Cl | 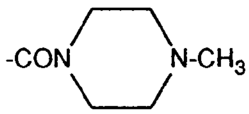 | |
| 5.037 | F | Cl | 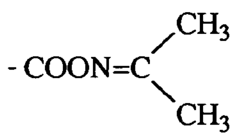 | |
| 5.038 | F | Cl | $-COOCH_2-CH_2-Cl$ | |
| 5.039 | F | Cl | $-COOCH_2-CN$ | |
| 5.040 | F | Cl | 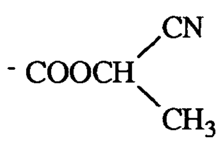 | |
| 5.041 | F | Cl | $-COOCH_2-CH=CH_2$ | |
| 5.042 | F | Cl | $-COOCH_2-CH=CHCl$ | |
| 5.043 | F | Cl | 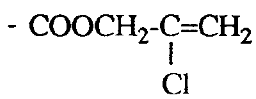 | |
| 5.044 | F | Cl | $-COOCH_2-C\equiv CH$ | |
| 5.045 | F | Cl | 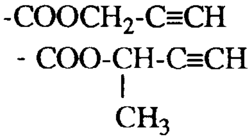 | |
| 5.046 | F | Cl | 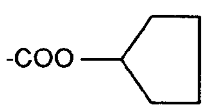 | |

| Verb. Nr. | $R_1$ | $R_2$ | A | Phys. Daten |
|---|---|---|---|---|
| 5.047 | F | Cl | 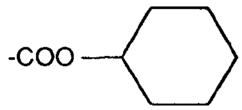 | |
| 5.048 | F | Cl | 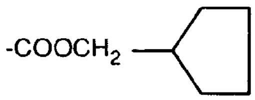 | |
| 5.049 | F | Cl | 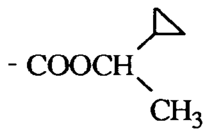 | |
| 5.050 | F | Cl | 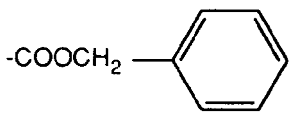 | |
| 5.051 | F | Cl | 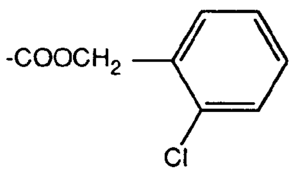 | |
| 5.052 | F | Cl | 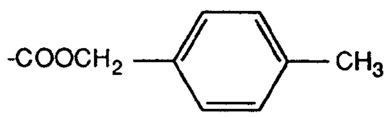 | |
| 5.053 | F | Cl | $-COSCH_3$ | |
| 5.054 | F | Cl | $-COSC_2H_5$ | |
| 5.055 | F | Cl | $-COSC_3H_7$ | |
| 5.056 | F | Cl | $-COS-CH_2-CH=CH_2$ | |
| 5.057 | F | Cl | $-COS-CH_2-COOCH_3$ | |
| 5.058 | F | Cl | $-COS-CH_2-COOC_2H_5$ | |
| 5.059 | F | Cl | $-COS-CH_2-COOC_5H_{11}$ | |
| 5.060 | F | Cl | $- COS-CH-COOCH_3$<br>$\quad\quad\quad |$<br>$\quad\quad\quad CH_3$ | |
| 5.061 | F | Cl | $- COS-CH-COOC_2H_5$<br>$\quad\quad\quad |$<br>$\quad\quad\quad CH_3$ | |

| Verb. Nr. | $R_1$ | $R_2$ | A | Phys. Daten |
|---|---|---|---|---|
| 5.062 | F | Cl | 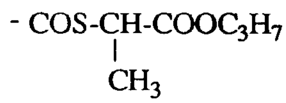 | |
| 5.063 | F | Cl | $-COS-CH_2-CH_2-COOCH_3$ | |
| 5.064 | F | Cl | $-COS-CH_2-COOCH_2-CH_2-O-CH_3$ | |
| 5.065 | F | Cl | $-COOCH_2-COOCH_3$ | |
| 5.066 | F | Cl | 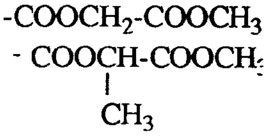 | |
| 5.067 | F | Cl | $-COOCH_2-COOC_5H_{11}$ | |
| 5.068 | F | Cl | $-COOCH_2-CH_2-Si(CH_3)_3$ | |
| 5.069 | F | Cl | -COONa | |
| 5.070 | F | Cl | 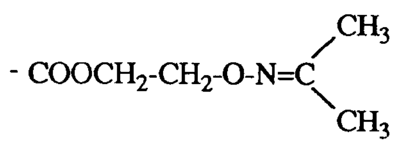 | |
| 5.071 | F | Cl | -OH | |
| 5.072 | F | Cl | $-OCH_3$ | |
| 5.073 | F | Cl | $-OC_2H_5$ | |
| 5.074 | F | Cl | $-OC_3H_7$ | |
| 5.075 | F | Cl | 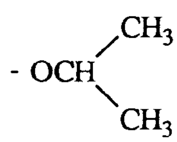 | Oel |
| 5.076 | F | Cl | $-OC_4H_9$ | |
| 5.077 | F | Cl | 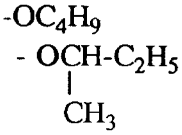 | |
| 5.078 | F | Cl | 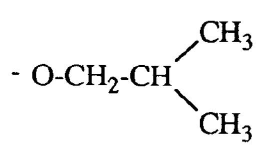 | |
| 5.079 | F | Cl | $-OCH_2CH=CH_2$ | |
| 5.080 | F | Cl | 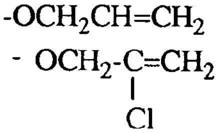 | |

| Verb. Nr. | $R_1$ | $R_2$ | A | Phys. Daten |
|---|---|---|---|---|
| 5.081 | F | Cl | $-OCH_2CH{=}CHCl$ | |
| 5.082 | F | Cl | $-OCH_2C{\equiv}CH$ | |
| 5.083 | F | Cl | 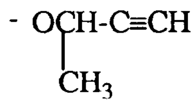 | |
| 5.084 | F | Cl | $-OCH_2-COOCH_3$ | |
| 5.085 | F | Cl | $-O-CH_2-COOC_5H_{11}$ | |
| 5.086 | F | Cl | 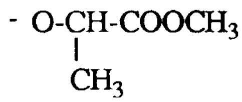 | |
| 5.087 | F | Cl | $-O-CH_2-COOC_2H_5$ | |
| 5.088 | F | Cl | 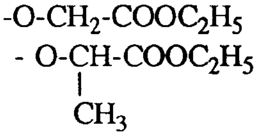 | |
| 5.089 | F | Cl | $-O-CH_2-CH_2-O-CH_3$ | |
| 5.090 | F | Cl | 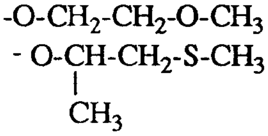 | |
| 5.091 | F | Cl | 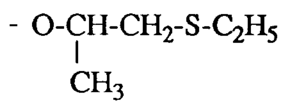 | |
| 5.092 | F | Cl | 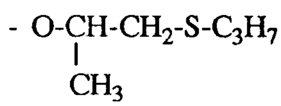 | |
| 5.093 | F | Cl | $-O-CH_2-CH_2-Cl$ | |
| 5.094 | F | Cl | $-O-CH_2-CN$ | |
| 5.095 | F | Cl | 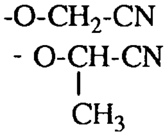 | |
| 5.096 | F | Cl | $-S-CH_3$ | |
| 5.097 | F | Cl | $-S-C_2H_5$ | |
| 5.098 | F | Cl | $-S-C_3H_7$ | |
| 5.099 | F | Cl | 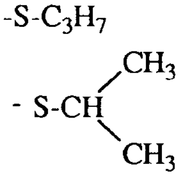 | |

| Verb. Nr. | $R_1$ | $R_2$ | A | Phys. Daten |
|---|---|---|---|---|
| 5.100 | F | Cl | $-S-CH_2-CH=CH_2$ | |
| 5.101 | F | Cl | $- S-CH_2-C=CH_2$<br>\|<br>Cl | |
| 5.102 | F | Cl | $-S-CH_2-CH=CHCl$ | |
| 5.103 | F | Cl | $-S-CH_2-C\equiv CH$ | |
| 5.104 | F | Cl | $- S-CH-C\equiv CH$<br>\|<br>$CH_3$ | |
| 5.105 | F | Cl | $-S-CH_2-COOCH_3$ | Oel |
| 5.106 | F | Cl | $-S-CH_2-COOC_2H_5$ | |
| 5.107 | F | Cl | $-S-CH_2-COOC_5H_{11}$ | |
| 5.108 | F | Cl | $- S-CH-COOCH_3$<br>\|<br>$CH_3$ | |
| 5.109 | F | Cl | $- S-CH-COOC_2H_5$<br>\|<br>$CH_3$ | |
| 5.110 | F | Cl | $-S-CH_2-COOCH_2-CH_2-O-CH_3$ | |
| 5.111 | F | Cl | 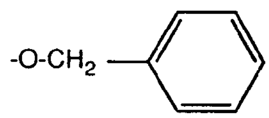 | |
| 5.112 | F | Cl | 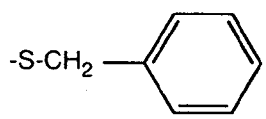 | |
| 5.113 | F | Cl | $-C-CN$<br>\|\|<br>$N-O-CH_3$ | |
| 5.114 | F | Cl | $-C-CN$<br>\|\|<br>$N-O-CH_2-COOCH_3$ | |
| 5.115 | F | Cl | $-C-CN$<br>\|\|<br>$N-O-CH_2-C\equiv CH$ | |

| Verb. Nr. | $R_1$ | $R_2$ | A | Phys. Daten |
|---|---|---|---|---|
| 5.116 | F | Cl | 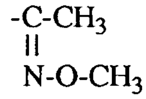 | |
| 5.117 | F | Cl | 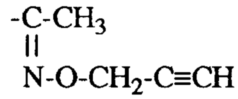 | |
| 5.118 | F | Cl | 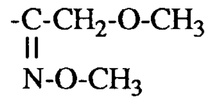 | |
| 5.119 | F | Cl | 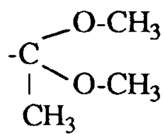 | |
| 5.120 | F | Cl | 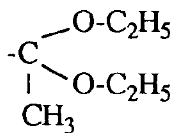 | |
| 5.121 | F | Cl | 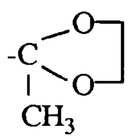 | |
| 5.122 | F | Cl | 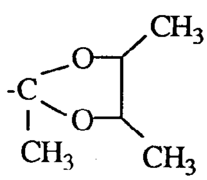 | |
| 5.123 | F | Cl | 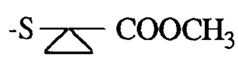 | |
| 5.124 | F | Cl | 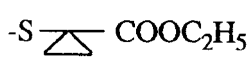 | |
| 5.125 | F | Cl | 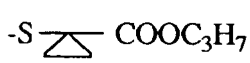 | |
| 5.126 | F | Cl | 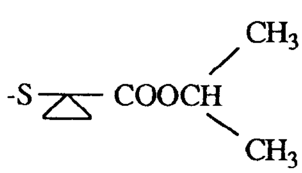 | |

| Verb. Nr. | $R_1$ | $R_2$ | A | Phys. Daten |
|---|---|---|---|---|
| 5.127 | F | Cl | 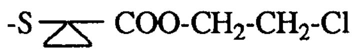 | |
| 5.128 | F | Cl | 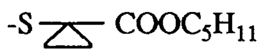 | |
| 5.129 | F | Cl | 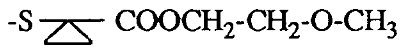 | |
| 5.130 | F | Cl | 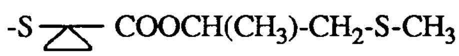 | |
| 5.131 | F | Cl | 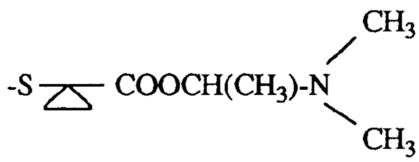 | |
| 5.132 | F | Cl | 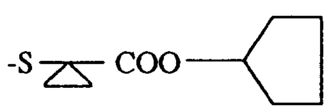 | |
| 5.133 | F | Cl | 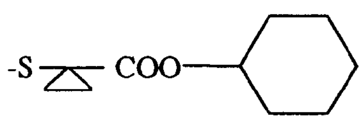 | |
| 5.134 | F | Cl | 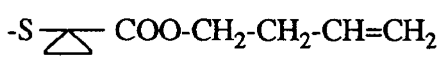 | |
| 5.135 | F | Cl | 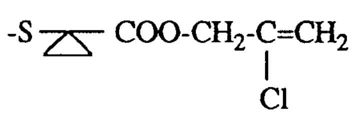 | |
| 5.136 | F | Cl | 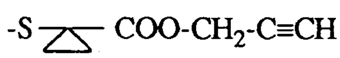 | |
| 5.137 | F | Cl | 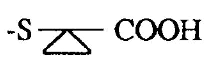 | |
| 5.138 | F | Cl | 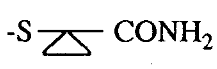 | |
| 5.139 | F | Cl | 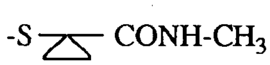 | |
| 5.140 | F | Cl | 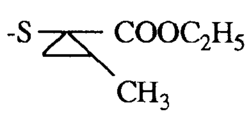 | |
| 5.141 | F | Cl | 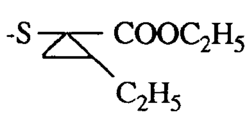 | |

| Verb. Nr. | $R_1$ | $R_2$ | A | Phys. Daten |
|---|---|---|---|---|
| 5.142 | F | Cl | 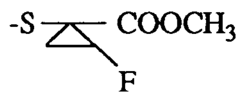 | |
| 5.143 | F | Cl | 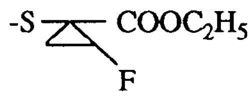 | |
| 5.144 | F | Cl | 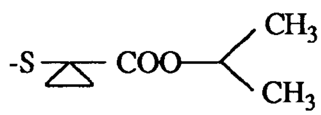 | |
| 5.145 | F | Cl | 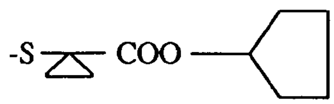 | |
| 5.146 | F | Cl | $-NH-SO_2-CH_3$ | |
| 5.147 | F | Cl | $-NH-SO_2-C_2H_5$ | |
| 5.148 | F | Cl | $-NH-SO_2-Cl$ | |
| 5.149 | F | Cl | 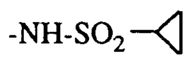 | |
| 5.150 | F | Cl | 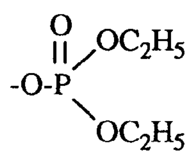 | |
| 5.151 | H | Cl | $-COOH$ | |
| 5.152 | H | Cl | $-COOCH_3$ | |
| 5.153 | H | Cl | 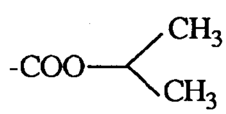 | Harz |
| 5.154 | H | Cl | $-COO-C_5H_{11}$ | |
| 5.155 | H | Cl | $-COO-CH_2-CH_2-O-CH_3$ | |
| 5.156 | H | Cl | $-COOCH_2-S-CH_3$ | |
| 5.157 | H | Cl | $-COOCH(CH_3)-CH_2-S-CH_3$ | |
| 5.158 | H | Cl | 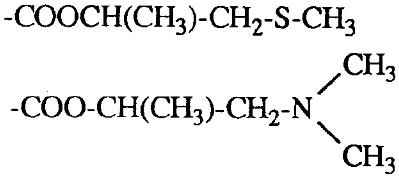 | |

| Verb. Nr. | $R_1$ | $R_2$ | A | Phys. Daten |
|---|---|---|---|---|
| 5.159 | H | Cl | 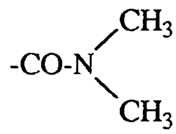 | |
| 5.160 | H | Cl | 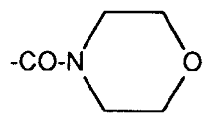 | |
| 5.161 | H | Cl | 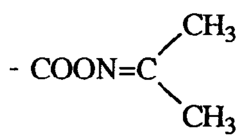 | |
| 5.162 | H | Cl | 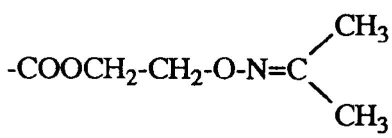 | |
| 5.163 | H | Cl | 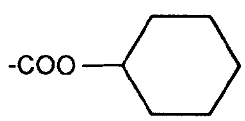 | |
| 5.164 | H | Cl | 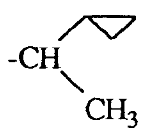 | |
| 5.165 | H | Cl | $-S-C_3H_7$ | |
| 5.166 | H | Cl | $-COOCH_2-COOCH_3$ | |
| 5.167 | H | Cl | $-COOCH(CH_3)-COOCH_3$ | |
| 5.168 | H | Cl | $-COS-CH_2-COOCH_3$ | |
| 5.169 | H | Cl | $-COS-CH(CH_3)-COOCH_3$ | |
| 5.170 | H | Cl | -OH | |
| 5.171 | H | Cl | $-OCH_3$ | |
| 5.172 | H | Cl | $-O-C_2H_5$ | |
| 5.173 | H | Cl | 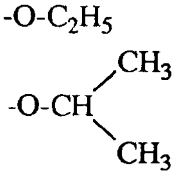 | |
| 5.174 | H | Cl | $-O-CH_2-C\equiv CH$ | |

| Verb. Nr. | $R_1$ | $R_2$ | A | Phys. Daten |
|---|---|---|---|---|
| 5.175 | H | Cl | $-O-CH_2-CH=CHCl$ | |
| 5.176 | H | Cl | 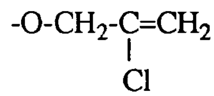 | |
| 5.177 | H | Cl | 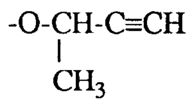 | |
| 5.178 | H | Cl | $-O-CH_2-COOCH_3$ | |
| 5.179 | H | Cl | $-O-CH_2-COOC_2H_5$ | |
| 5.180 | H | Cl | $-O-CH(CH_3)-COOCH_3$ | |
| 5.181 | H | Cl | $-SH$ | |
| 5.182 | H | Cl | $-SCH_3$ | |
| 5.183 | H | Cl | $-SC_2H_5$ | |
| 5.184 | H | Cl | 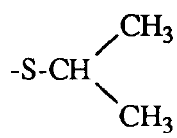 | |
| 5.185 | H | Cl | $-S-CH_2-COOCH_3$ | |
| 5.186 | H | Cl | $-S-CH(CH_3)-COOCH_3$ | |
| 5.187 | H | Cl | $-S-CH_2-COOC_2H_5$ | |
| 5.188 | H | Cl | 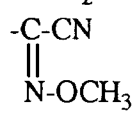 | |
| 5.189 | H | Cl | 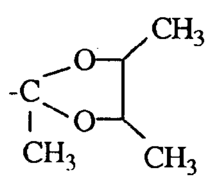 | |
| 5.190 | H | Cl | 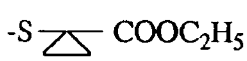 | |
| 5.191 | H | Cl | 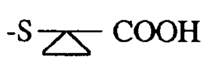 | |
| 5.192 | H | Cl | 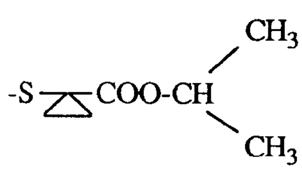 | |

| Verb. Nr. | $R_1$ | $R_2$ | A | Phys. Daten |
|---|---|---|---|---|
| 5.193 | H | Cl | 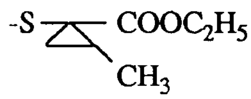 | |
| 5.194 | H | Cl | 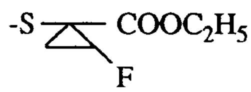 | |
| 5.195 | H | Cl | 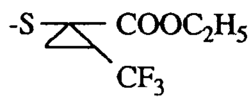 | |
| 5.196 | H | Cl | 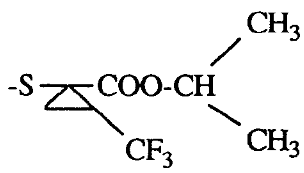 | |
| 5.197 | H | Cl | 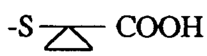 | |
| 5.198 | H | Cl | 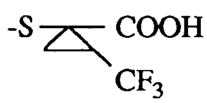 | |
| 5.199 | H | Cl | 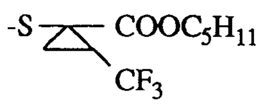 | |
| 5.200 | H | Cl | 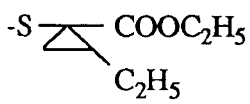 | |
| 5.201 | H | Cl | 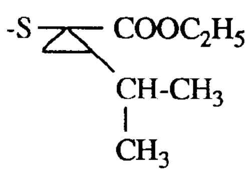 | |
| 5.202 | H | Cl | $-NH-SO_2-C_2H_5$ | |
| 5.203 | H | Cl | $-NH-SO_2-CH_2-Cl$ | |
| 5.204 | F | CN | -COOH | |
| 5.205 | F | CN | 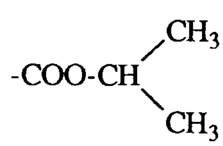 | |

| Verb. Nr. | $R_1$ | $R_2$ | A | Phys. Daten |
|---|---|---|---|---|
| 5.206 | F | CN | 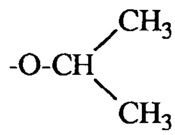 | |
| 5.207 | F | CN | $-O-CH_2-C{\equiv}CH$ | |
| 5.208 | F | CN | $-O-CH(CH_3)-C{\equiv}CH$ | |
| 5.209 | F | CN | $-S-CH_2-COOCH_3$ | |
| 5.210 | F | CN | $-S-CH(CH_3)-COOCH_3$ | |
| 5.211 | F | CN | $-O-CH_2-COOCH_3$ | |
| 5.212 | F | CN | $-O-CH_2-COOC_5H_{11}$ | |
| 5.213 | F | CN | $-O-CH(CH_3)-COOC_2H_5$ | |
| 5.214 | F | CN | 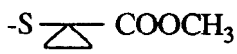 | |
| 5.215 | F | CN | 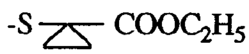 | |
| 5.216 | F | CN | 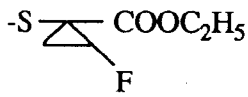 | |
| 5.217 | F | CN | 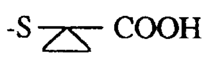 | |
| 5.218 | F | CN | 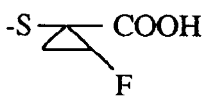 | |
| 5.219 | F | CN | 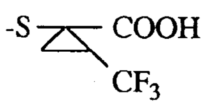 | |
| 5.220 | F | CN | 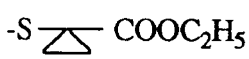 | |
| 5.221 | F | Br | -COOH | |
| 5.222 | F | Br | 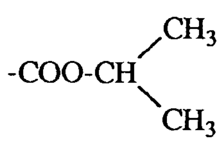 | |
| 5.223 | F | Br | -OH | |

| Verb. Nr. | $R_1$ | $R_2$ | A | Phys. Daten |
|---|---|---|---|---|
| 5.224 | F | Br | 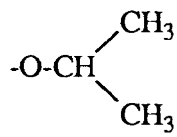 | |
| 5.225 | F | Br | $-O-CH_2-C\equiv CH$ | |
| 5.226 | F | Br | $-O-CH(CH_3)-C\equiv CH$ | |
| 5.227 | F | Br | $-O-CH_2COOCH_3$ | |
| 5.228 | F | Br | $-O-CH_2-COOC_5H_{11}$ | |
| 5.229 | F | Br | $-S-CH_2-COOCH_3$ | |
| 5.230 | F | Br | 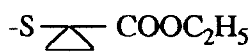 | |
| 5.231 | F | Br | 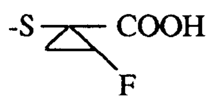 | |
| 5.232 | F | Br | 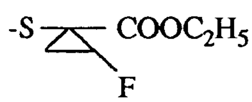 | |
| 5.233 | F | Cl | $-S-CH_2COOH$ | |

Tabelle 6: Verbindungen der Formel Ih

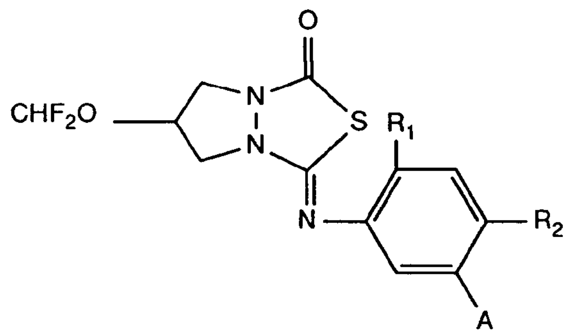

(Ih)

| Verb. Nr. | $R_1$ | $R_2$ | A | Phys. Daten |
|---|---|---|---|---|
| 6.001 | F | Cl | -H | |
| 6.002 | F | Cl | -CN | |
| 6.003 | F | Cl | $-NO_2$ | |
| 6.004 | F | Cl | -COOH | |
| 6.005 | F | Cl | $-COOCH_3$ | |
| 6.006 | F | Cl | $-COOC_2H_5$ | |
| 6.007 | F | Cl | $-COOC_3H_7$ | |
| 6.008 | F | Cl | $-COOCH(CH_3)_2$ | |
| 6.009 | F | Cl | $-COOC_4H_9$ | |
| 6.010 | F | Cl | $-COOCH-CH_2-CH_3$<br>$\vert$<br>$CH_3$ | |
| 6.011 | F | Cl | $CH_3$<br>$-COOCH_2-CH_2-CH$<br>$CH_3$ | |
| 6.012 | F | Cl | $-COOC_5H_{11}$ | |
| 6.013 | F | Cl | $-COOCH_2-CH_2-O-CH_3$ | |
| 6.014 | F | Cl | $-COOCH_2-CH_2-O-C_2H_5$ | |
| 6.015 | F | Cl | $-COOCH(CH_3)-CH_2-OCH_3$ | |
| 6.016 | F | Cl | $-COOCH_2-CH_2-S-CH_3$ | |
| 6.017 | F | Cl | $-COOCH(CH_3)-CH_2-S-CH_3$ | |

| Verb. Nr. | $R_1$ | $R_2$ | A | Phys. Daten |
|---|---|---|---|---|
| 6.018 | F | Cl | $-COOCH(CH_3)-CH_2-S-C_2H_5$ | |
| 6.019 | F | Cl | $-COOCH(CH_3)-CH_2-S-C_3H_7$ | |
| 6.020 | F | Cl | 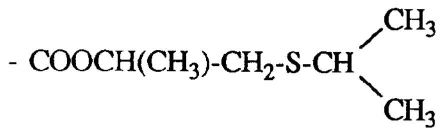 | |
| 6.021 | F | Cl | $-COOCH(CH_3)-CH_2-S-C_4H_9$ | |
| 6.022 | F | Cl | $-COOCH(CH_3)-CH_2-S-C_5H_{11}$ | |
| 6.023 | F | Cl | 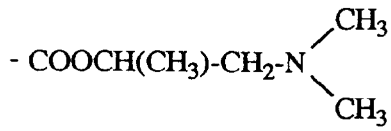 | |
| 6.024 | F | Cl | 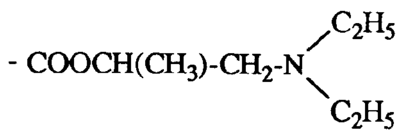 | |
| 6.025 | F | Cl | $-CONH_2$ | |
| 6.026 | F | Cl | $-CONH-CH_3$ | |
| 6.027 | F | Cl | 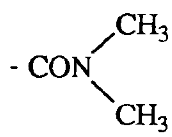 | |
| 6.028 | F | Cl | 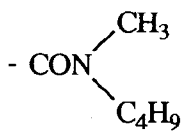 | |
| 6.029 | F | Cl | 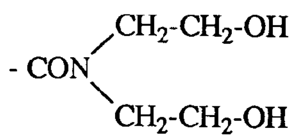 | |
| 6.030 | F | Cl | $-CONH-CH_2-CH=CH_2$ | |
| 6.031 | F | Cl | $-CON(CH_2-CH=CH_2)_2$ | |
| 6.032 | F | Cl | 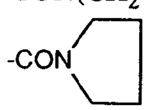 | |

| Verb. Nr. | $R_1$ | $R_2$ | A | Phys. Daten |
|---|---|---|---|---|
| 6.033 | F | Cl | 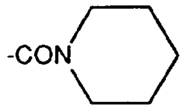 | |
| 6.034 | F | Cl | 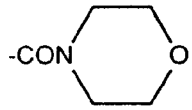 | |
| 6.035 | F | Cl | 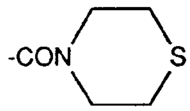 | |
| 6.036 | F | Cl | 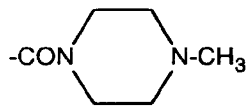 | |
| 6.037 | F | Cl | 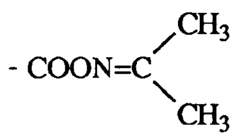 | |
| 6.038 | F | Cl | $-COOCH_2-CH_2-Cl$ | |
| 6.039 | F | Cl | $-COOCH_2-CN$ | |
| 6.040 | F | Cl | 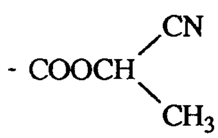 | |
| 6.041 | F | Cl | $-COOCH_2-CH=CH_2$ | |
| 6.042 | F | Cl | $-COOCH_2-CH=CHCl$ | |
| 6.043 | F | Cl | 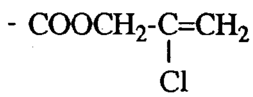 | |
| 6.044 | F | Cl | $-COOCH_2-C\equiv CH$ | |
| 6.045 | F | Cl | 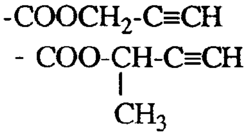 | |
| 6.046 | F | Cl | 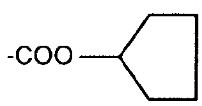 | |

| Verb. Nr. | $R_1$ | $R_2$ | A | Phys. Daten |
|---|---|---|---|---|
| 6.047 | F | Cl | -COO 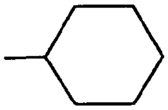 | |
| 6.048 | F | Cl | $-COOCH_2$ 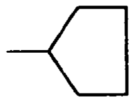 | |
| 6.049 | F | Cl | 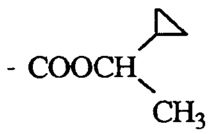 | |
| 6.050 | F | Cl | $-COOCH_2$ 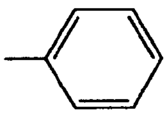 | |
| 6.051 | F | Cl | $-COOCH_2$ 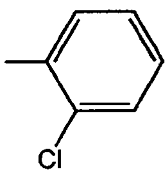 | |
| 6.052 | F | Cl | $-COOCH_2$ 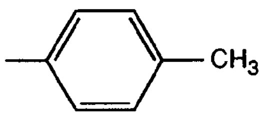 | |
| 6.053 | F | Cl | $-COSCH_3$ | |
| 6.054 | F | Cl | $-COSC_2H_5$ | |
| 6.055 | F | Cl | $-COSC_3H_7$ | |
| 6.056 | F | Cl | $-COS-CH_2-CH=CH_2$ | |
| 6.057 | F | Cl | $-COS-CH_2-COOCH_3$ | |
| 6.058 | F | Cl | $-COS-CH_2-COOC_2H_5$ | |
| 6.059 | F | Cl | $-COS-CH_2-COOC_5H_{11}$ | |
| 6.060 | F | Cl | $-$ COS-CH-$COOCH_3$<br>\|<br>$CH_3$ | |
| 6.061 | F | Cl | COS-CH-$COOC_2H_5$<br>\|<br>$CH_3$ | |

| Verb. Nr. | $R_1$ | $R_2$ | A | Phys. Daten |
|---|---|---|---|---|
| 6.062 | F | Cl | 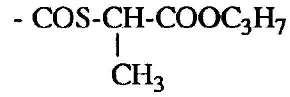 | |
| 6.063 | F | Cl | $-COS-CH_2-CH_2-COOCH_3$ | |
| 6.064 | F | Cl | $-COS-CH_2-COOCH_2-CH_2-O-CH_3$ | |
| 6.065 | F | Cl | $-COOCH_2-COOCH_3$ | |
| 6.066 | F | Cl | 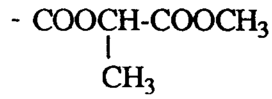 | |
| 6.067 | F | Cl | $-COOCH_2-COOC_5H_{11}$ | |
| 6.068 | F | Cl | $-COOCH_2-CH_2-Si(CH_3)_3$ | |
| 6.069 | F | Cl | -COONa | |
| 6.070 | F | Cl | 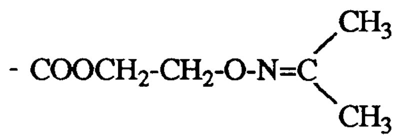 | |
| 6.071 | F | Cl | -OH | |
| 6.072 | F | Cl | $-OCH_3$ | |
| 6.073 | F | Cl | $-OC_2H_5$ | |
| 6.074 | F | Cl | $-OC_3H_7$ | |
| 6.075 | F | Cl | 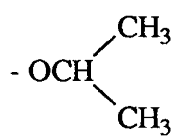 | Harz |
| 6.076 | F | Cl | $-OC_4H_9$ | |
| 6.077 | F | Cl | 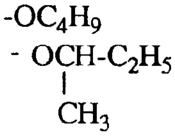 | |
| 6.078 | F | Cl | 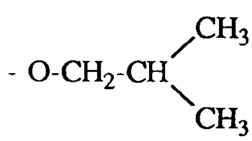 | |
| 6.079 | F | Cl | $-OCH_2CH=CH_2$ | |
| 6.080 | F | Cl | 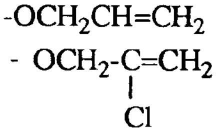 | |

| Verb. Nr. | $R_1$ | $R_2$ | A | Phys. Daten |
|---|---|---|---|---|
| 6.081 | F | Cl | $-OCH_2CH{=}CHCl$ | |
| 6.082 | F | Cl | $-OCH_2C{\equiv}CH$ | |
| 6.083 | F | Cl | 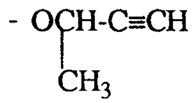 | |
| 6.084 | F | Cl | $-OCH_2-COOCH_3$ | |
| 6.085 | F | Cl | $-O-CH_2-COOC_5H_{11}$ | |
| 6.086 | F | Cl | 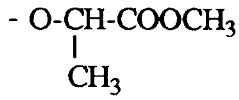 | |
| 6.087 | F | Cl | $-O-CH_2-COOC_2H_5$ | |
| 6.088 | F | Cl | 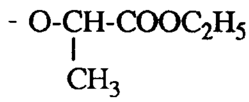 | |
| 6.089 | F | Cl | $-O-CH_2-CH_2-O-CH_3$ | |
| 6.090 | F | Cl | 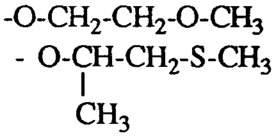 | |
| 6.091 | F | Cl | 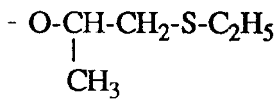 | |
| 6.092 | F | Cl | 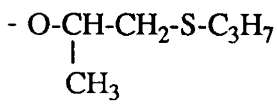 | |
| 6.093 | F | Cl | $-O-CH_2-CH_2-Cl$ | |
| 6.094 | F | Cl | $-O-CH_2-CN$ | |
| 6.095 | F | Cl | 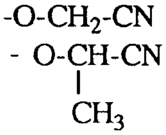 | |
| 6.096 | F | Cl | $-S-CH_3$ | |
| 6.097 | F | Cl | $-S-C_2H_5$ | |
| 6.098 | F | Cl | $-S-C_3H_7$ | |
| 6.099 | F | Cl | 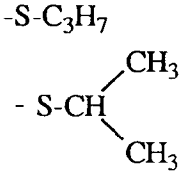 | |

| Verb. Nr. | $R_1$ | $R_2$ | A | Phys. Daten |
|---|---|---|---|---|
| 6.100 | F | Cl | $S-CH_2-CH=CH_2$ | |
| 6.101 | F | Cl | $-S-CH_2-C=CH_2$<br>\|<br>Cl | |
| 6.102 | F | Cl | $-S-CH_2-CH=CHCl$ | |
| 6.103 | F | Cl | $-S-CH_2-C{\equiv}CH$ | |
| 6.104 | F | Cl | $-S-CH-C{\equiv}CH$<br>\|<br>$CH_3$ | |
| 6.105 | F | Cl | $-S-CH_2-COOCH_3$ | |
| 6.106 | F | Cl | $-S-CH_2-COOC_2H_5$ | |
| 6.107 | F | Cl | $-S-CH_2-COOC_5H_{11}$ | |
| 6.108 | F | Cl | $-S-CH-COOCH_3$<br>\|<br>$CH_3$ | |
| 6.109 | F | Cl | $-S-CH-COOC_2H_5$<br>\|<br>$CH_3$ | |
| 6.110 | F | Cl | $-S-CH_2-COOCH_2-CH_2-O-CH_3$ | |
| 6.111 | F | Cl | 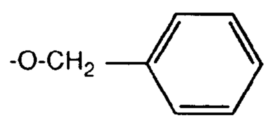 | |
| 6.112 | F | Cl | 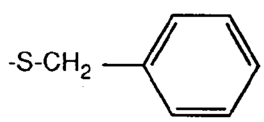 | |
| 6.113 | F | Cl | $-C-CN$<br>\|\|<br>$N-O-CH_3$ | |
| 6.114 | F | Cl | $-C-CN$<br>\|\|<br>$N-O-CH_2-COOCH_3$ | |
| 6.115 | F | Cl | $-C-CN$<br>\|\|<br>$N-O-CH_2-C{\equiv}CH$ | |

| Verb. Nr. | $R_1$ | $R_2$ | A | Phys. Daten |
|---|---|---|---|---|
| 6.116 | F | Cl | 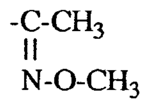 | |
| 6.117 | F | Cl | 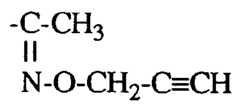 | |
| 6.118 | F | Cl | 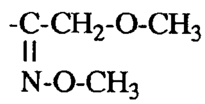 | |
| 6.119 | F | Cl | 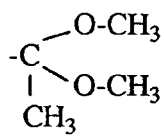 | |
| 6.120 | F | Cl | 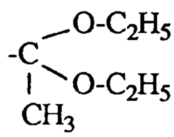 | |
| 6.121 | F | Cl | 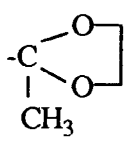 | |
| 6.122 | F | Cl | 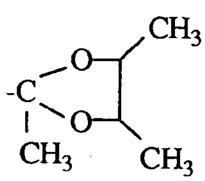 | |
| 6.123 | F | Cl | 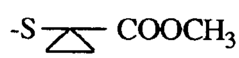 | |
| 6.124 | F | Cl | 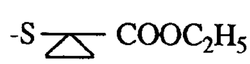 | |
| 6.125 | F | Cl | 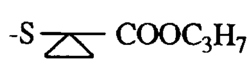 | |
| 6.126 | F | Cl | 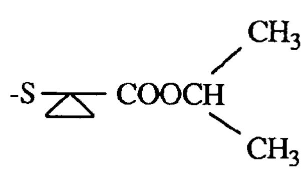 | |

| Verb. Nr. | $R_1$ | $R_2$ | A | Phys. Daten |
|---|---|---|---|---|
| 6.127 | F | Cl | 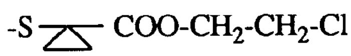 | |
| 6.128 | F | Cl | 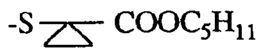 | |
| 6.129 | F | Cl | 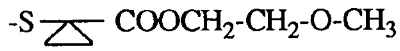 | |
| 6.130 | F | Cl | 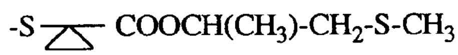 | |
| 6.131 | F | Cl | 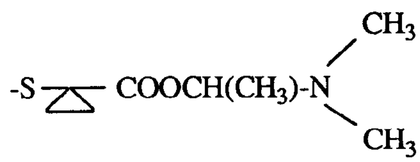 | |
| 6.132 | F | Cl | 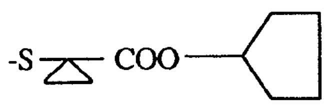 | |
| 6.133 | F | Cl | 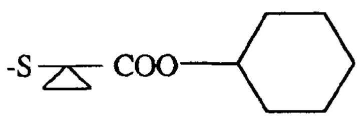 | |
| 6.134 | F | Cl | 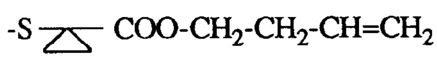 | |
| 6.135 | F | Cl | 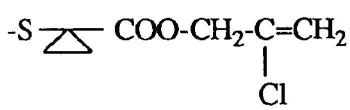 | |
| 6.136 | F | Cl | 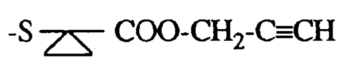 | |
| 6.137 | F | Cl | 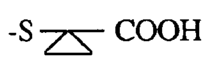 | |
| 6.138 | F | Cl | 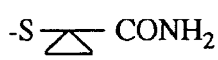 | |
| 6.139 | F | Cl | 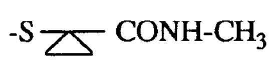 | |
| 6.140 | F | Cl | 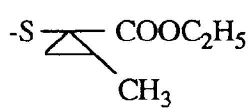 | |
| 6.141 | F | Cl | 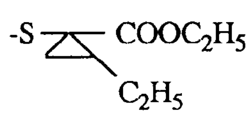 | |

| Verb. Nr. | $R_1$ | $R_2$ | A | Phys. Daten |
|---|---|---|---|---|
| 6.142 | F | Cl | 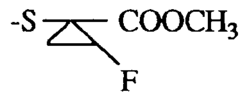 | |
| 6.143 | F | Cl | 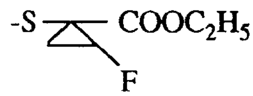 | |
| 6.144 | F | Cl | 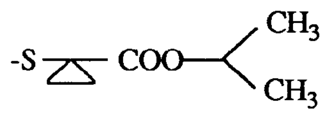 | |
| 6.145 | F | Cl | 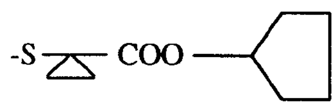 | |
| 6.146 | F | Cl | $-NH-SO_2-CH_3$ | |
| 6.147 | F | Cl | $-NH-SO_2-C_2H_5$ | |
| 6.148 | F | Cl | $-NH-SO_2-Cl$ | |
| 6.149 | F | Cl | 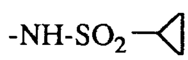 | |
| 6.150 | F | Cl | 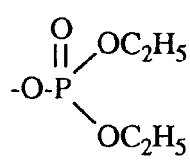 | |
| 6.151 | H | Cl | -COOH | |
| 6.152 | H | Cl | $-COOCH_3$ | |
| 6.153 | H | Cl | 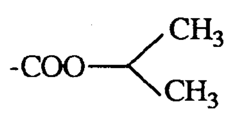 | |
| 6.154 | H | Cl | $-COO-C_5H_{11}$ | |
| 6.155 | H | Cl | $-COO-CH_2-CH_2-O-CH_3$ | |
| 6.156 | H | Cl | $-COOCH_2-S-CH_3$ | |
| 6.157 | H | Cl | $-COOCH(CH_3)-CH_2-S-CH_3$ | |
| 6.158 | H | Cl | 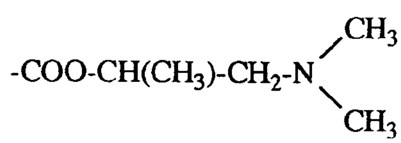 | |

| Verb. Nr. | $R_1$ | $R_2$ | A | Phys. Daten |
|---|---|---|---|---|
| 6.159 | H | Cl | 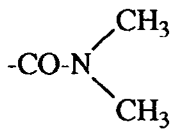 | |
| 6.160 | H | Cl | 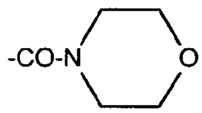 | |
| 6.161 | H | Cl | 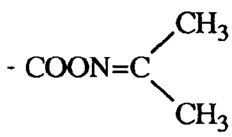 | |
| 6.162 | H | Cl | 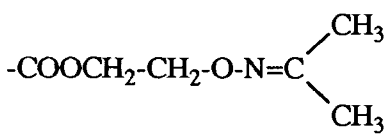 | |
| 6.163 | H | Cl | 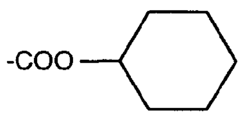 | |
| 6.164 | H | Cl | 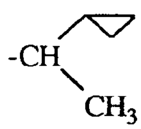 | |
| 6.165 | H | Cl | $-S-C_3H_7$ | |
| 6.166 | H | Cl | $-COOCH_2-COOCH_3$ | |
| 6.167 | H | Cl | $-COOCH(CH_3)-COOCH_3$ | |
| 6.168 | H | Cl | $-COS-CH_2-COOCH_3$ | |
| 6.169 | H | Cl | $-COS-CH(CH_3)-COOCH_3$ | |
| 6.170 | H | Cl | -OH | |
| 6.171 | H | Cl | $-OCH_3$ | |
| 6.172 | H | Cl | $-O-C_2H_5$ | |
| 6.173 | H | Cl | 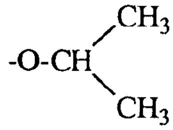 | |
| 6.174 | H | Cl | $-O-CH_2-C{\equiv}CH$ | |

| Verb. Nr. | $R_1$ | $R_2$ | A | Phys. Daten |
|---|---|---|---|---|
| 6.175 | H | Cl | $-O-CH_2-CH=CHCl$ | |
| 6.176 | H | Cl | 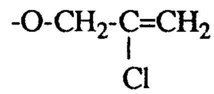 | |
| 6.177 | H | Cl | 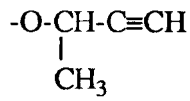 | |
| 6.178 | H | Cl | $-O-CH_2-COOCH_3$ | |
| 6.179 | H | Cl | $-O-CH_2-COOC_2H_5$ | |
| 6.180 | H | Cl | $-O-CH(CH_3)-COOCH_3$ | |
| 6.181 | H | Cl | -SH | |
| 6.182 | H | Cl | $-SCH_3$ | |
| 6.183 | H | Cl | $-SC_2H_5$ | |
| 6.184 | H | Cl | 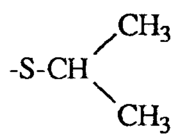 | |
| 6.185 | H | Cl | $-S-CH_2-COOCH_3$ | |
| 6.186 | H | Cl | $-S-CH(CH_3)-COOCH_3$ | |
| 6.187 | H | Cl | $-S-CH_2-COOC_2H_5$ | |
| 6.188 | H | Cl | 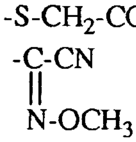 | |
| 6.189 | H | Cl | 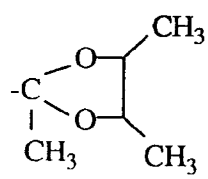 | |
| 6.190 | H | Cl | 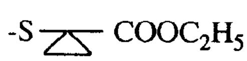 | |
| 6.191 | H | Cl | 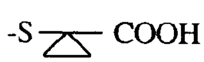 | |
| 6.192 | H | Cl | 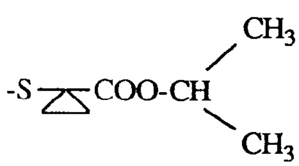 | |

| Verb. Nr. | $R_1$ | $R_2$ | A | Phys. Daten |
|---|---|---|---|---|
| 6.193 | H | Cl | 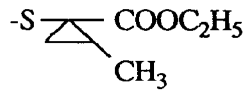 | |
| 6.194 | H | Cl | 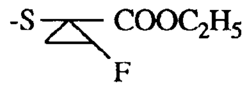 | |
| 6.195 | H | Cl | 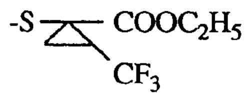 | |
| 6.196 | H | Cl | 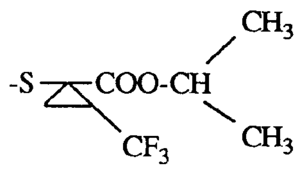 | |
| 6.197 | H | Cl | 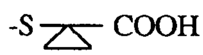 | |
| 6.198 | H | Cl | 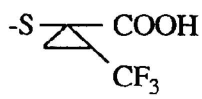 | |
| 6.199 | H | Cl | 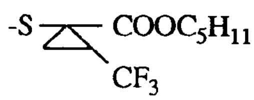 | |
| 6.200 | H | Cl | 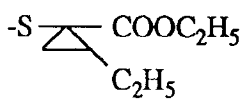 | |
| 6.201 | H | Cl | 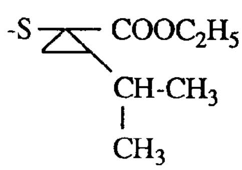 | |
| 6.202 | H | Cl | $-NH-SO_2-C_2H_5$ | |
| 6.203 | H | Cl | $-NH-SO_2-CH_2-Cl$ | |
| 6.204 | F | CN | -COOH | |
| 6.205 | F | CN | 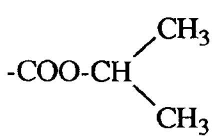 | |

| Verb. Nr. | $R_1$ | $R_2$ | A | Phys. Daten |
|---|---|---|---|---|
| 6.206 | F | CN | 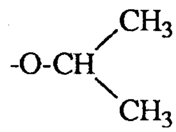 | |
| 6.207 | F | CN | $-O-CH_2-C{\equiv}CH$ | |
| 6.208 | F | CN | $-O-CH(CH_3)-C{\equiv}CH$ | |
| 6.209 | F | CN | $-S-CH_2-COOCH_3$ | |
| 6.210 | F | CN | $-S-CH(CH_3)-COOCH_3$ | |
| 6.211 | F | CN | $-O-CH_2-COOCH_3$ | |
| 6.212 | F | CN | $-O-CH_2-COOC_5H_{11}$ | |
| 6.213 | F | CN | $-O-CH(CH_3)-COOC_2H_5$ | |
| 6.214 | F | CN | 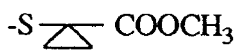 | |
| 6.215 | F | CN | 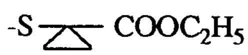 | |
| 6.216 | F | CN | 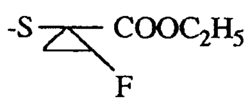 | |
| 6.217 | F | CN | 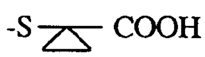 | |
| 6.218 | F | CN | 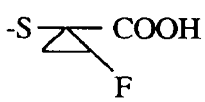 | |
| 6.219 | F | CN | 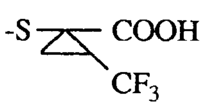 | |
| 6.220 | F | CN | 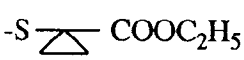 | |
| 6.221 | F | Br | -COOH | |
| 6.222 | F | Br | 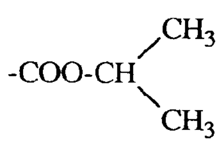 | |
| 6.223 | F | Br | -OH | |

| Verb. Nr. | $R_1$ | $R_2$ | A | Phys. Daten |
|---|---|---|---|---|
| 6.224 | F | Br | 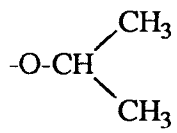 | |
| 6.225 | F | Br | $-O-CH_2-C{\equiv}CH$ | |
| 6.226 | F | Br | $-O-CH(CH_3)-C{\equiv}CH$ | |
| 6.227 | F | Br | $-O-CH_2COOCH_3$ | |
| 6.228 | F | Br | $-O-CH_2-COOC_5H_{11}$ | |
| 6.229 | F | Br | $-S-CH_2-COOCH_3$ | |
| 6.230 | F | Br | 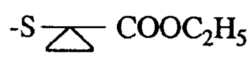 | |
| 6.231 | F | Br | 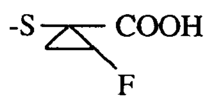 | |
| 6.232 | F | Br | 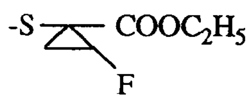 | |
| 6.233 | F | Cl | $-S-CH_2COOH$ | |

Tabelle 7: Verbindungen der Formel Ii

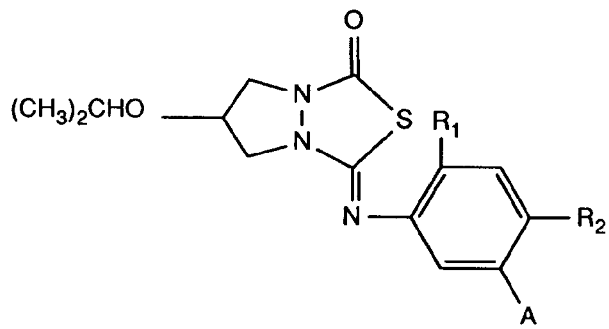

(Ii)

| Verb. Nr. | $R_1$ | $R_2$ | A | Phys. Daten |
|---|---|---|---|---|
| 7.001 | F | Cl | -H | |
| 7.002 | F | Cl | -CN | |
| 7.003 | F | Cl | $-NO_2$ | |
| 7.004 | F | Cl | -COOH | |
| 7.005 | F | Cl | $-COOCH_3$ | |
| 7.006 | F | Cl | $-COOC_2H_5$ | |
| 7.007 | F | Cl | $-COOC_3H_7$ | |
| 7.008 | F | Cl | $-COOCH(CH_3)_2$ | |
| 7.009 | F | Cl | $-COOC_4H_9$ | |
| 7.010 | F | Cl | $-COOCH-CH_2-CH_3$<br>$CH_3$ | |
| 7.011 | F | Cl | $CH_3$<br>$-COOCH_2-CH_2-CH$<br>$CH_3$ | |
| 7.012 | F | Cl | $-COOC_5H_{11}$ | |
| 7.013 | F | Cl | $-COOCH_2-CH_2-O-CH_3$ | |
| 7.014 | F | Cl | $-COOCH_2-CH_2-O-C_2H_5$ | |
| 7.015 | F | Cl | $-COOCH(CH_3)-CH_2-OCH_3$ | |
| 7.016 | F | Cl | $-COOCH_2-CH_2-S-CH_3$ | |
| 7.017 | F | Cl | $-COOCH(CH_3)-CH_2-S-CH_3$ | |

| Verb. Nr. | $R_1$ | $R_2$ | A | Phys. Daten |
|---|---|---|---|---|
| 7.018 | F | Cl | $-COOCH(CH_3)-CH_2-S-C_2H_5$ | |
| 7.019 | F | Cl | $-COOCH(CH_3)-CH_2-S-C_3H_7$ | |
| 7.020 | F | Cl | 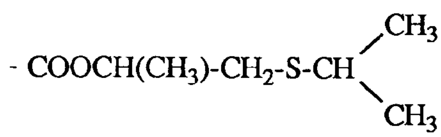 | |
| 7.021 | F | Cl | $-COOCH(CH_3)-CH_2-S-C_4H_9$ | |
| 7.022 | F | Cl | $-COOCH(CH_3)-CH_2-S-C_5H_{11}$ | |
| 7.023 | F | Cl | 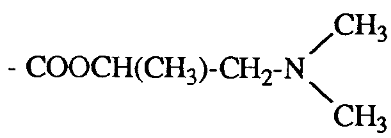 | |
| 7.024 | F | Cl | 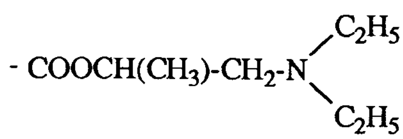 | |
| 7.025 | F | Cl | $-CONH_2$ | |
| 7.026 | F | Cl | $-CONH-CH_3$ | |
| 7.027 | F | Cl | 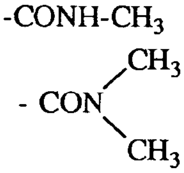 | |
| 7.028 | F | Cl | 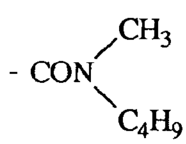 | |
| 7.029 | F | Cl | 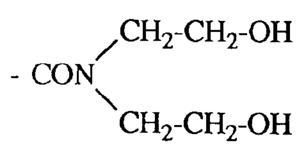 | |
| 7.030 | F | Cl | $-CONH-CH_2-CH=CH_2$ | |
| 7.031 | F | Cl | $-CON(CH_2-CH=CH_2)_2$ | |
| 7.032 | F | Cl | 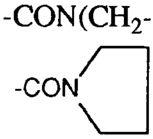 | |

| Verb. Nr. | $R_1$ | $R_2$ | A | Phys. Daten |
|---|---|---|---|---|
| 7.033 | F | Cl | 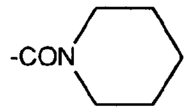 | |
| 7.034 | F | Cl | 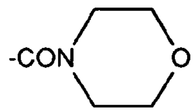 | |
| 7.035 | F | Cl | 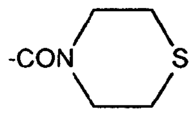 | |
| 7.036 | F | Cl | 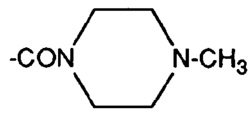 | |
| 7.037 | F | Cl | 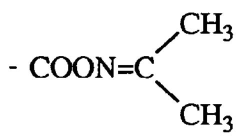 | |
| 7.038 | F | Cl | $-COOCH_2-CH_2-Cl$ | |
| 7.039 | F | Cl | $-COOCH_2-CN$ | |
| 7.040 | F | Cl | 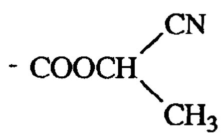 | |
| 7.041 | F | Cl | $-COOCH_2-CH=CH_2$ | |
| 7.042 | F | Cl | $-COOCH_2-CH=CHCl$ | |
| 7.043 | F | Cl | 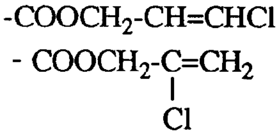 | |
| 7.044 | F | Cl | $-COOCH_2-C\equiv CH$ | |
| 7.045 | F | Cl | 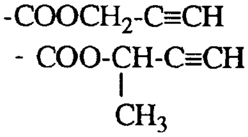 | |
| 7.046 | F | Cl | 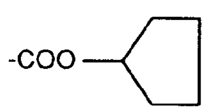 | |

| Verb. Nr. | $R_1$ | $R_2$ | A | Phys. Daten |
|---|---|---|---|---|
| 7.047 | F | Cl | 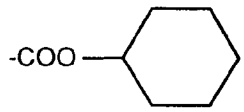 | |
| 7.048 | F | Cl | 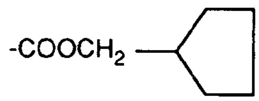 | |
| 7.049 | F | Cl | 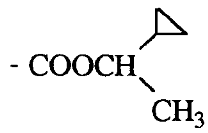 | |
| 7.050 | F | Cl | 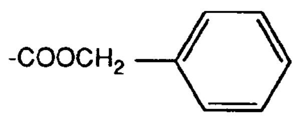 | |
| 7.051 | F | Cl | 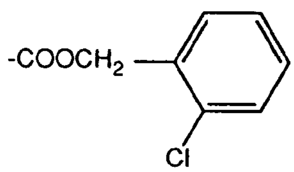 | |
| 7.052 | F | Cl | 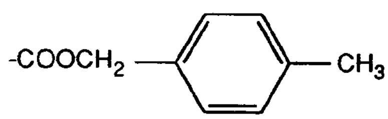 | |
| 7.053 | F | Cl | $-COSCH_3$ | |
| 7.054 | F | Cl | $-COSC_2H_5$ | |
| 7.055 | F | Cl | $-COSC_3H_7$ | |
| 7.056 | F | Cl | $-COS-CH_2-CH=CH_2$ | |
| 7.057 | F | Cl | $-COS-CH_2-COOCH_3$ | |
| 7.058 | F | Cl | $-COS-CH_2-COOC_2H_5$ | |
| 7.059 | F | Cl | $-COS-CH_2-COOC_5H_{11}$ | |
| 7.060 | F | Cl | $- COS-CH-COOCH_3$<br>\|<br>$CH_3$ | |
| 7.061 | F | Cl | $- COS-CH-COOC_2H_5$<br>\|<br>$CH_3$ | |

| Verb. Nr. | $R_1$ | $R_2$ | A | Phys. Daten |
|---|---|---|---|---|
| 7.062 | F | Cl | 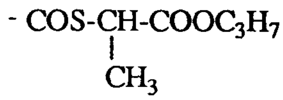 | |
| 7.063 | F | Cl | $-COS-CH_2-CH_2-COOCH_3$ | |
| 7.064 | F | Cl | $-COS-CH_2-COOCH_2-CH_2-O-CH_3$ | |
| 7.065 | F | Cl | $-COOCH_2-COOCH_3$ | |
| 7.066 | F | Cl | 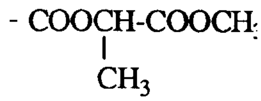 | |
| 7.067 | F | Cl | $-COOCH_2-COOC_5H_{11}$ | |
| 7.068 | F | Cl | $-COOCH_2-CH_2-Si(CH_3)_3$ | |
| 7.069 | F | Cl | -COONa | |
| 7.070 | F | Cl | 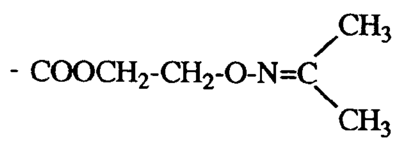 | |
| 7.071 | F | Cl | -OH | |
| 7.072 | F | Cl | $-OCH_3$ | |
| 7.073 | F | Cl | $-OC_2H_5$ | |
| 7.074 | F | Cl | $-OC_3H_7$ | |
| 7.075 | F | Cl | 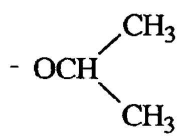 | |
| 7.076 | F | Cl | $-OC_4H_9$ | |
| 7.077 | F | Cl | 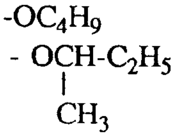 | |
| 7.078 | F | Cl | 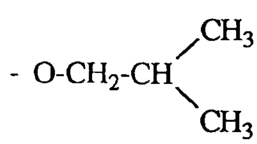 | |
| 7.079 | F | Cl | $-OCH_2CH=CH_2$ | |
| 7.080 | F | Cl | 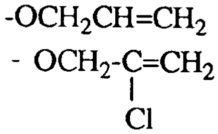 | |

| Verb. Nr. | $R_1$ | $R_2$ | A | Phys. Daten |
|---|---|---|---|---|
| 7.081 | F | Cl | $-OCH_2CH=CHCl$ | |
| 7.082 | F | Cl | $-OCH_2C\equiv CH$ | |
| 7.083 | F | Cl | $- OCH-C\equiv CH$<br>$CH_3$ | |
| 7.084 | F | Cl | $-OCH_2-COOCH_3$ | |
| 7.085 | F | Cl | $-O-CH_2-COOC_5H_{11}$ | |
| 7.086 | F | Cl | $- O-CH-COOCH_3$<br>$CH_3$ | |
| 7.087 | F | Cl | $-O-CH_2-COOC_2H_5$ | |
| 7.088 | F | Cl | $- O-CH-COOC_2H_5$<br>$CH_3$ | |
| 7.089 | F | Cl | $-O-CH_2-CH_2-O-CH_3$ | |
| 7.090 | F | Cl | $- O-CH-CH_2-S-CH_3$<br>$CH_3$ | |
| 7.091 | F | Cl | $- O-CH-CH_2-S-C_2H_5$<br>$CH_3$ | |
| 7.092 | F | Cl | $- O-CH-CH_2-S-C_3H_7$<br>$CH_3$ | |
| 7.093 | F | Cl | $-O-CH_2-CH_2-Cl$ | |
| 7.094 | F | Cl | $-O-CH_2-CN$ | |
| 7.095 | F | Cl | $- O-CH-CN$<br>$CH_3$ | |
| 7.096 | F | Cl | $-S-CH_3$ | |
| 7.097 | F | Cl | $-S-C_2H_5$ | |
| 7.098 | F | Cl | $-S-C_3H_7$ | |
| 7.099 | F | Cl | $CH_3$<br>$- S-CH$<br>$CH_3$ | |

<table>
<tr><th>Verb. Nr.</th><th>$R_1$</th><th>$R_2$</th><th>A</th><th>Phys. Daten</th></tr>
<tr><td>7.100</td><td>F</td><td>Cl</td><td>$-S-CH_2-CH=CH_2$</td><td></td></tr>
<tr><td>7.101</td><td>F</td><td>Cl</td><td>$-S-CH_2-C=CH_2$<br>|<br>Cl</td><td></td></tr>
<tr><td>7.102</td><td>F</td><td>Cl</td><td>$-S-CH_2-CH=CHCl$</td><td></td></tr>
<tr><td>7.103</td><td>F</td><td>Cl</td><td>$-S-CH_2-C{\equiv}CH$</td><td></td></tr>
<tr><td>7.104</td><td>F</td><td>Cl</td><td>$-S-CH-C{\equiv}CH$<br>|<br>$CH_3$</td><td></td></tr>
<tr><td>7.105</td><td>F</td><td>Cl</td><td>$-S-CH_2-COOCH_3$</td><td></td></tr>
<tr><td>7.106</td><td>F</td><td>Cl</td><td>$-S-CH_2-COOC_2H_5$</td><td></td></tr>
<tr><td>7.107</td><td>F</td><td>Cl</td><td>$-S-CH_2-COOC_5H_{11}$</td><td></td></tr>
<tr><td>7.108</td><td>F</td><td>Cl</td><td>$-S-CH-COOCH_3$<br>|<br>$CH_3$</td><td></td></tr>
<tr><td>7.109</td><td>F</td><td>Cl</td><td>$-S-CH-COOC_2H_5$<br>|<br>$CH_3$</td><td></td></tr>
<tr><td>7.110</td><td>F</td><td>Cl</td><td>$-S-CH_2-COOCH_2-CH_2-O-CH_3$</td><td></td></tr>
<tr><td>7.111</td><td>F</td><td>Cl</td><td>$-O-CH_2$ 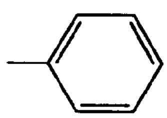</td><td></td></tr>
<tr><td>7.112</td><td>F</td><td>Cl</td><td>$-S-CH_2$ 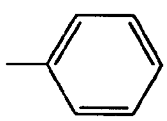</td><td></td></tr>
<tr><td>7.113</td><td>F</td><td>Cl</td><td>$-C-CN$<br>||<br>$N-O-CH_3$</td><td></td></tr>
<tr><td>7.114</td><td>F</td><td>Cl</td><td>$-C-CN$<br>||<br>$N-O-CH_2-COOCH_3$</td><td></td></tr>
<tr><td>7.115</td><td>F</td><td>Cl</td><td>$-C-CN$<br>||<br>$N-O-CH_2-C{\equiv}CH$</td><td></td></tr>
</table>

| Verb. Nr. | $R_1$ | $R_2$ | A | Phys. Daten |
|---|---|---|---|---|
| 7.116 | F | Cl | 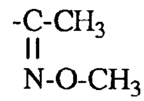 | |
| 7.117 | F | Cl | 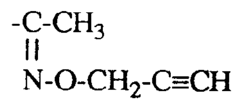 | |
| 7.118 | F | Cl | 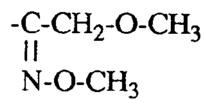 | |
| 7.119 | F | Cl | 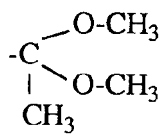 | |
| 7.120 | F | Cl | 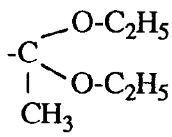 | |
| 7.121 | F | Cl | 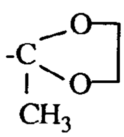 | |
| 7.122 | F | Cl | 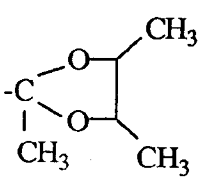 | |
| 7.123 | F | Cl | 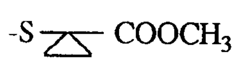 | |
| 7.124 | F | Cl | 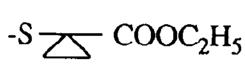 | |
| 7.125 | F | Cl | 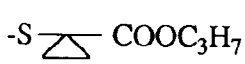 | |
| 7.126 | F | Cl | 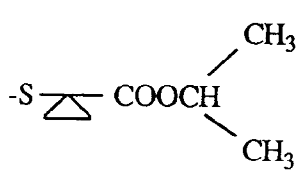 | |

| Verb. Nr. | $R_1$ | $R_2$ | A | Phys. Daten |
|---|---|---|---|---|
| 7.127 | F | Cl | 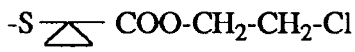 | |
| 7.128 | F | Cl | 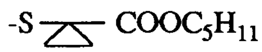 | |
| 7.129 | F | Cl | 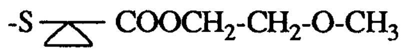 | |
| 7.130 | F | Cl | 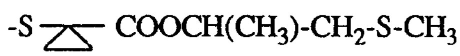 | |
| 7.131 | F | Cl | 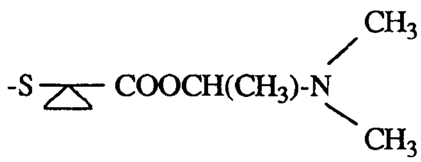 | |
| 7.132 | F | Cl | 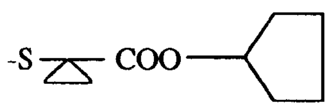 | |
| 7.133 | F | Cl | 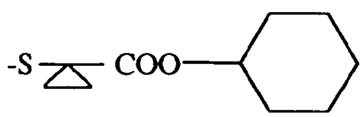 | |
| 7.134 | F | Cl | 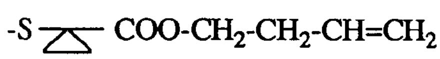 | |
| 7.135 | F | Cl | 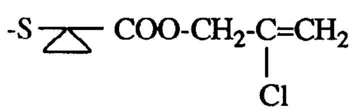 | |
| 7.136 | F | Cl | 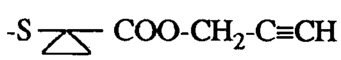 | |
| 7.137 | F | Cl | 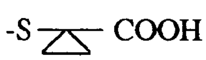 | |
| 7.138 | F | Cl | 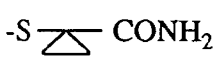 | |
| 7.139 | F | Cl | 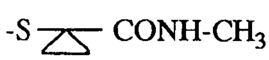 | |
| 7.140 | F | Cl | 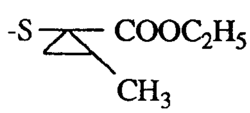 | |
| 7.141 | F | Cl | 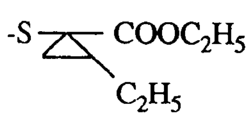 | |

| Verb. Nr. | $R_1$ | $R_2$ | A | Phys. Daten |
|---|---|---|---|---|
| 7.142 | F | Cl | 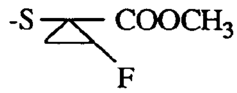 | |
| 7.143 | F | Cl | 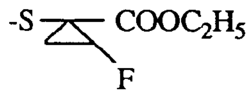 | |
| 7.144 | F | Cl | 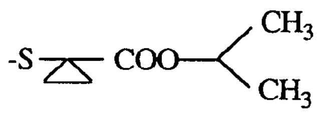 | |
| 7.145 | F | Cl | 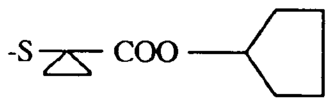 | |
| 7.146 | F | Cl | $-NH-SO_2-CH_3$ | |
| 7.147 | F | Cl | $-NH-SO_2-C_2H_5$ | |
| 7.148 | F | Cl | $-NH-SO_2-Cl$ | |
| 7.149 | F | Cl | 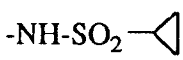 | |
| 7.150 | F | Cl | 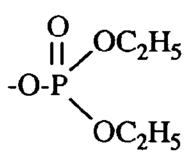 | |
| 7.151 | H | Cl | $-COOH$ | |
| 7.152 | H | Cl | $-COOCH_3$ | |
| 7.153 | H | Cl | 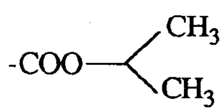 | |
| 7.154 | H | Cl | $-COO-C_5H_{11}$ | |
| 7.155 | H | Cl | $-COO-CH_2-CH_2-O-CH_3$ | |
| 7.156 | H | Cl | $-COOCH_2-S-CH_3$ | |
| 7.157 | H | Cl | $-COOCH(CH_3)-CH_2-S-CH_3$ | |
| 7.158 | H | Cl | 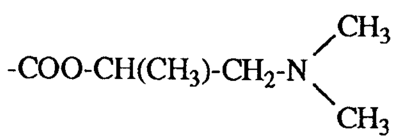 | |

| Verb. Nr. | $R_1$ | $R_2$ | A | Phys. Daten |
|---|---|---|---|---|
| 7.159 | H | Cl | 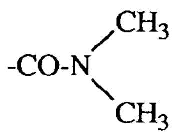 | |
| 7.160 | H | Cl | 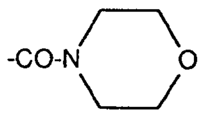 | |
| 7.161 | H | Cl | 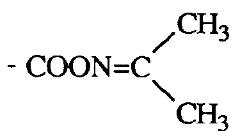 | |
| 7.162 | H | Cl | 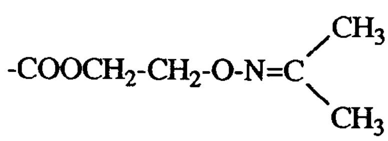 | |
| 7.163 | H | Cl | 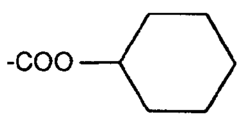 | |
| 7.164 | H | Cl | 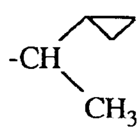 | |
| 7.165 | H | Cl | $-S-C_3H_7$ | |
| 7.166 | H | Cl | $-COOCH_2-COOCH_3$ | |
| 7.167 | H | Cl | $-COOCH(CH_3)-COOCH_3$ | |
| 7.168 | H | Cl | $-COS-CH_2-COOCH_3$ | |
| 7.169 | H | Cl | $-COS-CH(CH_3)-COOCH_3$ | |
| 7.170 | H | Cl | -OH | |
| 7.171 | H | Cl | $-OCH_3$ | |
| 7.172 | H | Cl | $-O-C_2H_5$ | |
| 7.173 | H | Cl | 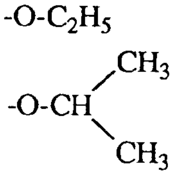 | |
| 7.174 | H | Cl | $-O-CH_2-C{\equiv}CH$ | |

| Verb. Nr. | $R_1$ | $R_2$ | A | Phys. Daten |
|---|---|---|---|---|
| 7.175 | H | Cl | $-O-CH_2-CH=CHCl$ | |
| 7.176 | H | Cl | 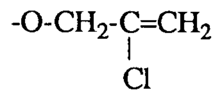 | |
| 7.177 | H | Cl | 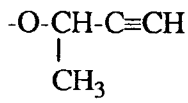 | |
| 7.178 | H | Cl | $-O-CH_2-COOCH_3$ | |
| 7.179 | H | Cl | $-O-CH_2-COOC_2H_5$ | |
| 7.180 | H | Cl | $-O-CH(CH_3)-COOCH_3$ | |
| 7.181 | H | Cl | -SH | |
| 7.182 | H | Cl | $-SCH_3$ | |
| 7.183 | H | Cl | $-SC_2H_5$ | |
| 7.184 | H | Cl | 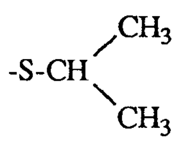 | |
| 7.185 | H | Cl | $-S-CH_2-COOCH_3$ | |
| 7.186 | H | Cl | $-S-CH(CH_3)-COOCH_3$ | |
| 7.187 | H | Cl | $-S-CH_2-COOC_2H_5$ | |
| 7.188 | H | Cl | 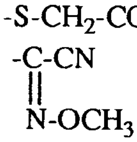 | |
| 7.189 | H | Cl | 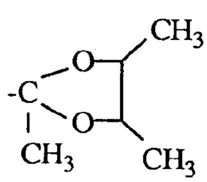 | |
| 7.190 | H | Cl | 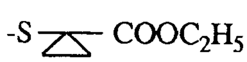 | |
| 7.191 | H | Cl | 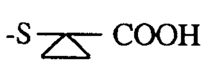 | |
| 7.192 | H | Cl | 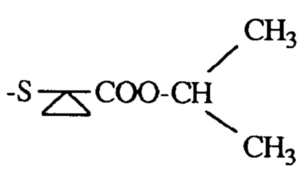 | |

| Verb. Nr. | $R_1$ | $R_2$ | A | Phys. Daten |
|---|---|---|---|---|
| 7.193 | H | Cl | 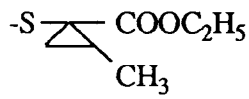 | |
| 7.194 | H | Cl | 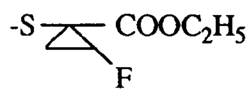 | |
| 7.195 | H | Cl | 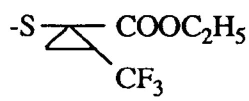 | |
| 7.196 | H | Cl | 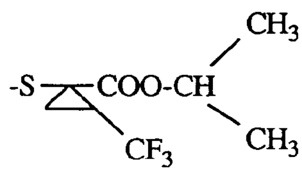 | |
| 7.197 | H | Cl | 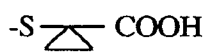 | |
| 7.198 | H | Cl | 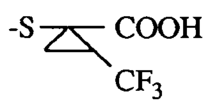 | |
| 7.199 | H | Cl | 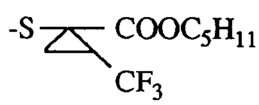 | |
| 7.200 | H | Cl | 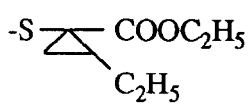 | |
| 7.201 | H | Cl | 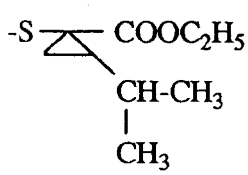 | |
| 7.202 | H | Cl | $-NH-SO_2-C_2H_5$ | |
| 7.203 | H | Cl | $-NH-SO_2-CH_2-Cl$ | |
| 7.204 | F | CN | -COOH | |
| 7.205 | F | CN | 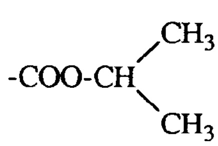 | |

| Verb. Nr. | $R_1$ | $R_2$ | A | Phys. Daten |
|---|---|---|---|---|
| 7.206 | F | CN | 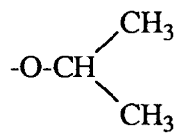 | |
| 7.207 | F | CN | $-O-CH_2-C{\equiv}CH$ | |
| 7.208 | F | CN | $-O-CH(CH_3)-C{\equiv}CH$ | |
| 7.209 | F | CN | $-S-CH_2-COOCH_3$ | |
| 7.210 | F | CN | $-S-CH(CH_3)-COOCH_3$ | |
| 7.211 | F | CN | $-O-CH_2-COOCH_3$ | |
| 7.212 | F | CN | $-O-CH_2-COOC_5H_{11}$ | |
| 7.213 | F | CN | $-O-CH(CH_3)-COOC_2H_5$ | |
| 7.214 | F | CN | 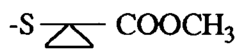 | |
| 7.215 | F | CN | 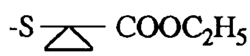 | |
| 7.216 | F | CN | 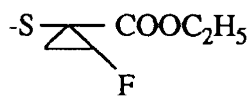 | |
| 7.217 | F | CN | 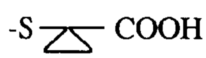 | |
| 7.218 | F | CN | 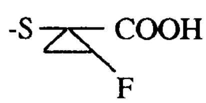 | |
| 7.219 | F | CN | 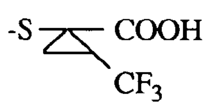 | |
| 7.220 | F | CN | 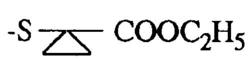 | |
| 7.221 | F | Br | -COOH | |
| 7.222 | F | Br | 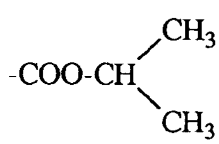 | |
| 7.223 | F | Br | -OH | |

| Verb. Nr. | $R_1$ | $R_2$ | A | Phys. Daten |
|---|---|---|---|---|
| 7.224 | F | Br | 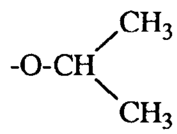 | |
| 7.225 | F | Br | $-O-CH_2-C\equiv CH$ | |
| 7.226 | F | Br | $-O-CH(CH_3)-C\equiv CH$ | |
| 7.227 | F | Br | $-O-CH_2COOCH_3$ | |
| 7.228 | F | Br | $-O-CH_2-COOC_5H_{11}$ | |
| 7.229 | F | Br | $-S-CH_2-COOCH_3$ | |
| 7.230 | F | Br | 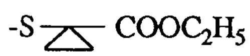 | |
| 7.231 | F | Br | 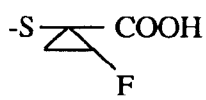 | |
| 7.232 | F | Br | 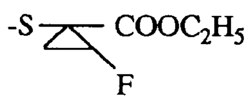 | |
| 7.233 | F | Cl | $-S-CH_2COOH$ | |

Tabelle 8: Verbindungen der Formel Ij

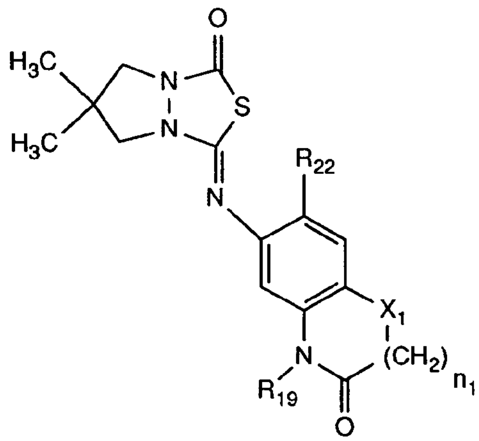

(Ij)

| Verb. Nr. | $R_{22}$ | $X_1$ | $R_{19}$ | $n_1$ | Phys. Daten |
|---|---|---|---|---|---|
| 8.001 | F | S | $-C_3H_7(i)$ | 0 | Smp. 136-137°C |
| 8.002 | H | S | $-C_3H_7(i)$ | 0 | |
| 8.003 | H | S | $-CH_2-C\equiv CH$ | 0 | |
| 8.004 | F | S | $-CH_2-C\equiv CH$ | 0 | |
| 8.005 | F | S | $-CH(CH_3)C\equiv CH$ | 0 | |
| 8.006 | H | S | $-CH(CH_3)C\equiv CH$ | 0 | |
| 8.007 | H | S | $-CH(CH_3)COOCH_3$ | 0 | |
| 8.008 | F | S | $-CH(CH_3)COOCH_3$ | 0 | |
| 8.009 | H | S | $-CH_2-COOCH_3$ | 0 | |
| 8.010 | H | S | $-CH_2-COOC_3H_7(i)$ | 0 | |
| 8.011 | F | O | $-C_3H_7(i)$ | 1 | |
| 8.012 | H | O | $-C_3H_7(i)$ | 1 | |
| 8.013 | F | O | $-CH_2-C\equiv CH$ | 1 | |
| 8.014 | H | O | $-CH_2-C\equiv CH$ | 1 | |
| 8.015 | F | O | $-CH(CH_3)C\equiv CH$ | 1 | |
| 8.016 | H | O | $-CH(CH_3)C\equiv CH$ | 1 | |
| 8.017 | H | O | $-CH_2COOCH_3$ | 1 | |
| 8.018 | F | O | $-CH_2-COOC_3H_7(i)$ | 1 | |
| 8.019 | H | O | $-CH(CH_3)COOC_3H_7(i)$ | 1 | |

Tabelle 9: Verbindungen der Formel Ik

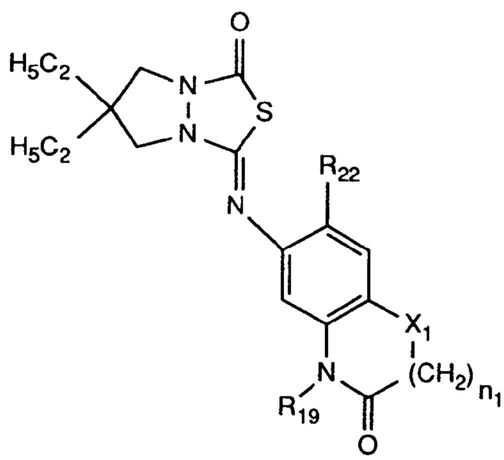

(Ik)

| Verb. Nr. | $R_{22}$ | $X_1$ | $R_{19}$ | $n_1$ | Phys. Daten |
|---|---|---|---|---|---|
| 9.001 | F | S | $-C_3H_7(i)$ | 0 | |
| 9.002 | H | S | $-C_3H_7(i)$ | 0 | |
| 9.003 | H | S | $-CH_2-C\equiv CH$ | 0 | |
| 9.004 | F | S | $-CH_2-C\equiv CH$ | 0 | |
| 9.005 | F | S | $-CH(CH_3)C\equiv CH$ | 0 | |
| 9.006 | H | S | $-CH(CH_3)C\equiv CH$ | 0 | |
| 9.007 | H | S | $-CH(CH_3)COOCH_3$ | 0 | |
| 9.008 | F | S | $-CH(CH_3)COOCH_3$ | 0 | |
| 9.009 | H | S | $-CH_2-COOCH_3$ | 0 | |
| 9.010 | H | S | $-CH_2-COOC_3H_7(i)$ | 0 | |
| 9.011 | F | O | $-C_3H_7(i)$ | 1 | |
| 9.012 | H | O | $-C_3H_7(i)$ | 1 | |
| 9.013 | F | O | $-CH_2-C\equiv CH$ | 1 | |
| 9.014 | H | O | $-CH_2-C\equiv CH$ | 1 | |
| 9.015 | F | O | $-CH(CH_3)C\equiv CH$ | 1 | |
| 9.016 | H | O | $-CH(CH_3)C\equiv CH$ | 1 | |
| 9.017 | H | O | $-CH_2COOCH_3$ | 1 | |
| 9.018 | F | O | $-CH_2-COOC_3H_7(i)$ | 1 | |
| 9.019 | H | O | $-CH(CH_3)COOC_3H_7(i)$ | 1 | |

Tabelle 10: Verbindungen der Formel II

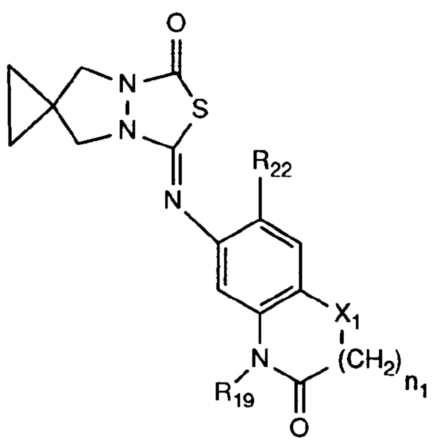

(II)

| Verb. Nr. | $R_{22}$ | $X_1$ | $R_{19}$ | $n_1$ | Phys. Daten |
|---|---|---|---|---|---|
| 10.001 | F | S | $-C_3H_7(i)$ | 0 | |
| 10.002 | H | S | $-C_3H_7(i)$ | 0 | Smp.> 73°C(Zers.) |
| 10.003 | H | S | $-CH_2-C\equiv CH$ | 0 | |
| 10.004 | F | S | $-CH_2-C\equiv CH$ | 0 | |
| 10.005 | F | S | $-CH(CH_3)C\equiv CH$ | 0 | |
| 10.006 | H | S | $-CH(CH_3)C\equiv CH$ | 0 | |
| 10.007 | H | S | $-CH(CH_3)COOCH_3$ | 0 | |
| 10.008 | F | S | $-CH(CH_3)COOC_2H_5$ | 0 | amorph |
| 10.009 | H | S | $-CH_2-COOCH_3$ | 0 | |
| 10.010 | H | S | $-CH_2-COOC_3H_7(i)$ | 0 | |
| 10.011 | F | O | $-C_3H_7(i)$ | 1 | |
| 10.012 | H | O | $-C_3H_7(i)$ | 1 | |
| 10.013 | F | O | $-CH_2-C\equiv CH$ | 1 | |
| 10.014 | H | O | $-CH_2-C\equiv CH$ | 1 | |
| 10.015 | F | O | $-CH(CH_3)C\equiv CH$ | 1 | |
| 10.016 | H | O | $-CH(CH_3)C\equiv CH$ | 1 | |
| 10.017 | H | O | $-CH_2COOCH_3$ | 1 | |
| 10.018 | F | O | $-CH_2-COOC_3H_7(i)$ | 1 | |
| 10.019 | H | O | $-CH(CH_3)COOC_3H_7(i)$ | 1 | |

Tabelle 11: Verbindungen der Formel Im

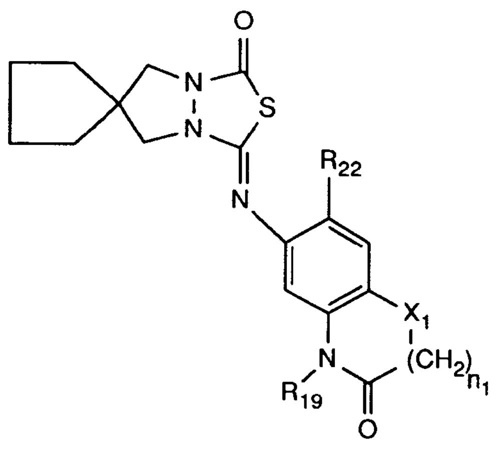

(Im)

| Verb. Nr. | $R_{22}$ | $X_1$ | $R_{19}$ | $n_1$ | Phys. Daten |
|---|---|---|---|---|---|
| 11.001 | F | S | $-C_3H_7(i)$ | 0 | |
| 11.002 | H | S | $-C_3H_7(i)$ | 0 | |
| 11.003 | H | S | $-CH_2-C{\equiv}CH$ | 0 | |
| 11.004 | F | S | $-CH_2-C{\equiv}CH$ | 0 | |
| 11.005 | F | S | $-CH(CH_3)C{\equiv}CH$ | 0 | |
| 11.006 | H | S | $-CH(CH_3)C{\equiv}CH$ | 0 | |
| 11.007 | H | S | $-CH(CH_3)COOCH_3$ | 0 | |
| 11.008 | F | S | $-CH(CH_3)COOCH_3$ | 0 | |
| 11.009 | H | S | $-CH_2-COOCH_3$ | 0 | |
| 11.010 | H | S | $-CH_2-COOC_3H_7(i)$ | 0 | |
| 11.011 | F | O | $-C_3H_7(i)$ | 1 | |
| 11.012 | H | O | $-C_3H_7(i)$ | 1 | |
| 11.013 | F | O | $-CH_2-C{\equiv}CH$ | 1 | |
| 11.014 | H | O | $-CH_2-C{\equiv}CH$ | 1 | |
| 11.015 | F | O | $-CH(CH_3)C{\equiv}CH$ | 1 | |
| 11.016 | H | O | $-CH(CH_3)C{\equiv}CH$ | 1 | |
| 11.017 | H | O | $-CH_2COOCH_3$ | 1 | |
| 11.018 | F | O | $-CH_2-COOC_3H_7(i)$ | 1 | |
| 11.019 | H | O | $-CH(CH_3)COOC_3H_7(i)$ | 1 | |

Tabelle 12: Verbindungen der Formel In

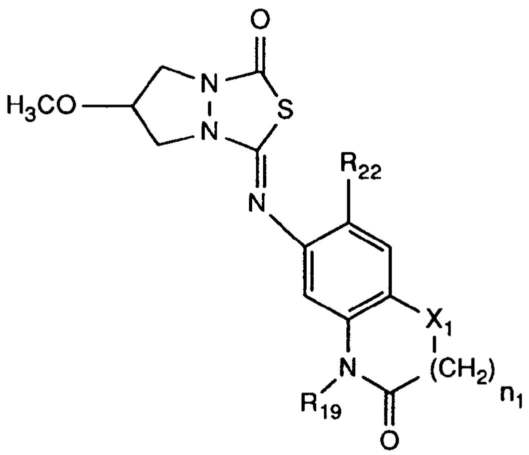

(In)

| Verb. Nr. | $R_{22}$ | $X_1$ | $R_{19}$ | $n_1$ | Phys. Daten |
|---|---|---|---|---|---|
| 12.001 | F | S | $-C_3H_7(i)$ | 0 | |
| 12.002 | H | S | $-C_3H_7(i)$ | 0 | |
| 12.003 | H | S | $-CH_2-C\equiv CH$ | 0 | |
| 12.004 | F | S | $-CH_2-C\equiv CH$ | 0 | |
| 12.005 | F | S | $-CH(CH_3)C\equiv CH$ | 0 | |
| 12.006 | H | S | $-CH(CH_3)C\equiv CH$ | 0 | |
| 12.007 | H | S | $-CH(CH_3)COOCH_3$ | 0 | |
| 12.008 | F | S | $-CH(CH_3)COOCH_3$ | 0 | |
| 12.009 | H | S | $-CH_2-COOCH_3$ | 0 | |
| 12.010 | H | S | $-CH_2-COOC_3H_7(i)$ | 0 | |
| 12.011 | F | O | $-C_3H_7(i)$ | 1 | |
| 12.012 | H | O | $-C_3H_7(i)$ | 1 | |
| 12.013 | F | O | $-CH_2-C\equiv CH$ | 1 | |
| 12.014 | H | O | $-CH_2-C\equiv CH$ | 1 | |
| 12.015 | F | O | $-CH(CH_3)C\equiv CH$ | 1 | |
| 12.016 | H | O | $-CH(CH_3)C\equiv CH$ | 1 | |
| 12.017 | H | O | $-CH_2COOCH_3$ | 1 | |
| 12.018 | F | O | $-CH_2-COOC_3H_7(i)$ | 1 | |
| 12.019 | H | O | $-CH(CH_3)COOC_3H_7(i)$ | 1 | |

Tabelle 13: Verbindungen der Formel Io

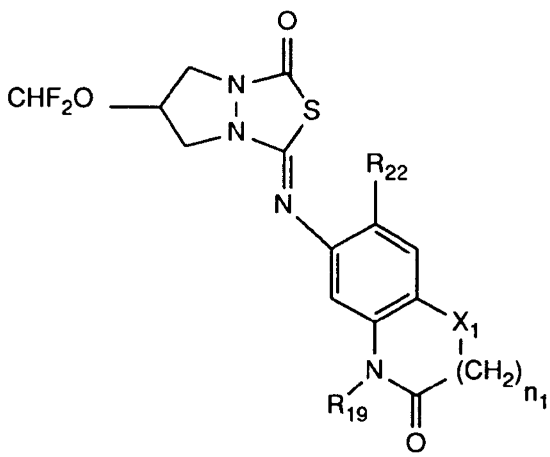

(Io)

| Verb. Nr. | $R_{22}$ | $X_1$ | $R_{19}$ | $n_1$ | Phys. Daten |
|---|---|---|---|---|---|
| 13.001 | F | S | $-C_3H_7(i)$ | 0 | |
| 13.002 | H | S | $-C_3H_7(i)$ | 0 | |
| 13.003 | H | S | $-CH_2-C\equiv CH$ | 0 | |
| 13.004 | F | S | $-CH_2-C\equiv CH$ | 0 | |
| 13.005 | F | S | $-CH(CH_3)C\equiv CH$ | 0 | |
| 13.006 | H | S | $-CH(CH_3)C\equiv CH$ | 0 | |
| 13.007 | H | S | $-CH(CH_3)COOCH_3$ | 0 | |
| 13.008 | F | S | $-CH(CH_3)COOCH_3$ | 0 | |
| 13.009 | H | S | $-CH_2-COOCH_3$ | 0 | |
| 13.010 | H | S | $-CH_2-COOC_3H_7(i)$ | 0 | |
| 13.011 | F | O | $-C_3H_7(i)$ | 1 | |
| 13.012 | H | O | $-C_3H_7(i)$ | 1 | |
| 13.013 | F | O | $-CH_2-C\equiv CH$ | 1 | |
| 13.014 | H | O | $-CH_2-C\equiv CH$ | 1 | |
| 13.015 | F | O | $-CH(CH_3)C\equiv CH$ | 1 | |
| 13.016 | H | O | $-CH(CH_3)C\equiv CH$ | 1 | |
| 13.017 | H | O | $-CH_2COOCH_3$ | 1 | |
| 13.018 | F | O | $-CH_2-COOC_3H_7(i)$ | 1 | |
| 13.019 | H | O | $-CH(CH_3)COOC_3H_7(i)$ | 1 | |

Tabelle 14: Verbindungen der Formel Ip

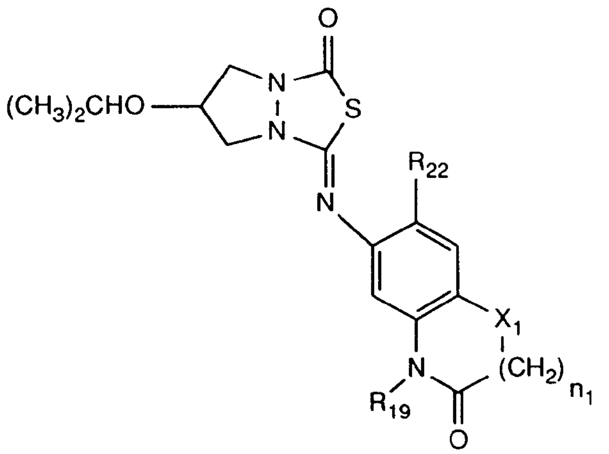

(Ip)

| Verb. Nr. | $R_{22}$ | $X_1$ | $R_{19}$ | $n_1$ | Phys. Daten |
|---|---|---|---|---|---|
| 14.001 | F | S | $-C_3H_7(i)$ | 0 | |
| 14.002 | H | S | $-C_3H_7(i)$ | 0 | |
| 14.003 | H | S | $-CH_2-C\equiv CH$ | 0 | |
| 14.004 | F | S | $-CH_2-C\equiv CH$ | 0 | |
| 14.005 | F | S | $-CH(CH_3)C\equiv CH$ | 0 | |
| 14.006 | H | S | $-CH(CH_3)C\equiv CH$ | 0 | |
| 14.007 | H | S | $-CH(CH_3)COOCH_3$ | 0 | |
| 14.008 | F | S | $-CH(CH_3)COOCH_3$ | 0 | |
| 14.009 | H | S | $-CH_2-COOCH_3$ | 0 | |
| 14.010 | H | S | $-CH_2-COOC_3H_7(i)$ | 0 | |
| 14.011 | F | O | $-C_3H_7(i)$ | 1 | |
| 14.012 | H | O | $-C_3H_7(i)$ | 1 | |
| 14.013 | F | O | $-CH_2-C\equiv CH$ | 1 | |
| 14.014 | H | O | $-CH_2-C\equiv CH$ | 1 | |
| 14.015 | F | O | $-CH(CH_3)C\equiv CH$ | 1 | |
| 14.016 | H | O | $-CH(CH_3)C\equiv CH$ | 1 | |
| 14.017 | H | O | $-CH_2COOCH_3$ | 1 | |
| 14.018 | F | O | $-CH_2-COOC_3H_7(i)$ | 1 | |
| 14.019 | H | O | $-CH(CH_3)COOC_3H_7(i)$ | 1 | |

Tabelle 15: Verbindungen der Formel Iq

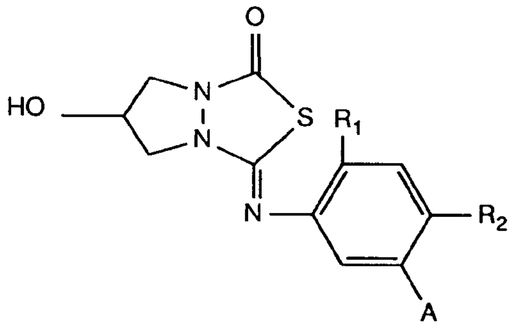

(Iq)

| Verb. Nr. | $R_1$ | $R_2$ | A | Phys. Daten |
|---|---|---|---|---|
| 15.001 | F | Cl | -H | |
| 15.002 | F | Cl | -CN | |
| 15.003 | F | Cl | $-NO_2$ | |
| 15.004 | F | Cl | -COOH | |
| 15.005 | F | Cl | $-COOCH_3$ | Harz |
| 15.006 | F | Cl | $-COOC_2H_5$ | |
| 15.007 | F | Cl | $-COOC_3H_7$ | |
| 15.008 | F | Cl | $-COOCH(CH_3)_2$ | |
| 15.009 | F | Cl | $-COOC_4H_9$ | |
| 15.010 | F | Cl | $-COOCH-CH_2-CH_3$<br>$CH_3$ | |
| 15.011 | F | Cl | $CH_3$<br>$-COOCH_2-CH_2-CH$<br>$CH_3$ | |
| 15.012 | F | Cl | $-COOC_5H_{11}$ | |
| 15.013 | F | Cl | $-COOCH_2-CH_2-O-CH_3$ | |
| 15.014 | F | Cl | $-COOCH_2-CH_2-O-C_2H_5$ | |
| 15.015 | F | Cl | $-COOCH(CH_3)-CH_2-OCH_3$ | |
| 15.016 | F | Cl | $-COOCH_2-CH_2-S-CH_3$ | |
| 15.017 | F | Cl | $-COOCH(CH_3)-CH_2-S-CH_3$ | |

| Verb. Nr. | $R_1$ | $R_2$ | A | Phys. Daten |
|---|---|---|---|---|
| 15.018 | F | Cl | $-COOCH(CH_3)-CH_2-S-C_2H_5$ | |
| 15.019 | F | Cl | $-COOCH(CH_3)-CH_2-S-C_3H_7$ | |
| 15.020 | F | Cl | 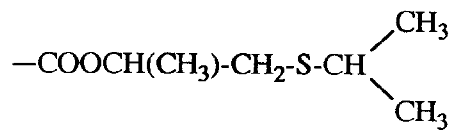 | |
| 15.021 | F | Cl | $-COOCH(CH_3)-CH_2-S-C_4H_9$ | |
| 15.022 | F | Cl | $-COOCH(CH_3)-CH_2-S-C_5H_{11}$ | |
| 15.023 | F | Cl | 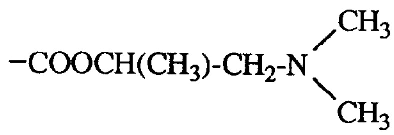 | |
| 15.024 | F | Cl | 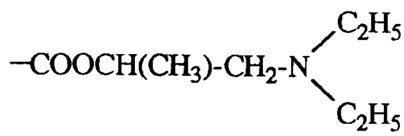 | |
| 15.025 | F | Cl | $-CONH_2$ | |
| 15.026 | F | Cl | $-CONH-CH_3$ | |
| 15.027 | F | Cl | 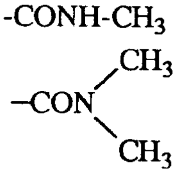 | |
| 15.028 | F | Cl | 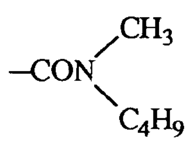 | |
| 15.029 | F | Cl | 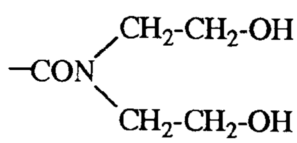 | |
| 15.030 | F | Cl | $-CONH-CH_2-CH=CH_2$ | |
| 15.031 | F | Cl | $-CON(CH_2-CH=CH_2)_2$ | |
| 15.032 | F | Cl | 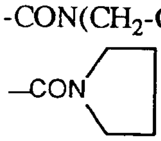 | |

| Verb. Nr. | $R_1$ | $R_2$ | A | Phys. Daten |
|---|---|---|---|---|
| 15.033 | F | Cl | 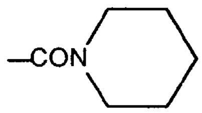 | |
| 15.034 | F | Cl | 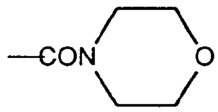 | |
| 15.035 | F | Cl | 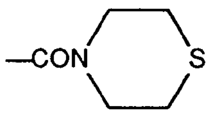 | |
| 15.036 | F | Cl | 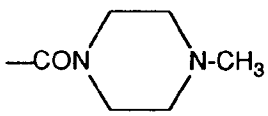 | |
| 15.037 | F | Cl | 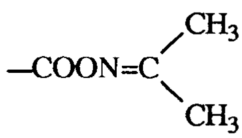 | |
| 15.038 | F | Cl | $-COOCH_2-CH_2-Cl$ | |
| 15.039 | F | Cl | $-COOCH_2-CN$ | |
| 15.040 | F | Cl | 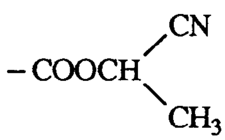 | |
| 15.041 | F | Cl | $-COOCH_2-CH=CH_2$ | |
| 15.042 | F | Cl | $-COOCH_2-CH=CHCl$ | |
| 15.043 | F | Cl | 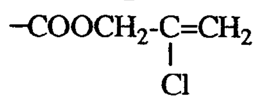 | |
| 15.044 | F | Cl | $-COOCH_2-C\equiv CH$ | |
| 15.045 | F | Cl | 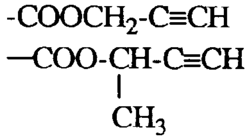 | |
| 15.046 | F | Cl | 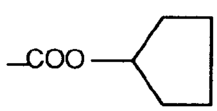 | |

| Verb. Nr. | $R_1$ | $R_2$ | A | Phys. Daten |
|---|---|---|---|---|
| 15.047 | F | Cl | 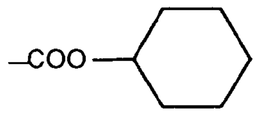 | |
| 15.048 | F | Cl | 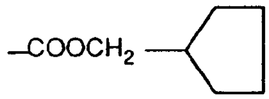 | |
| 15.049 | F | Cl | 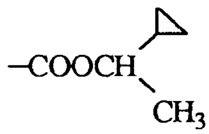 | |
| 15.050 | F | Cl | 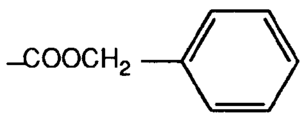 | |
| 15.051 | F | Cl | 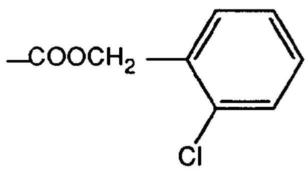 | |
| 15.052 | F | Cl | 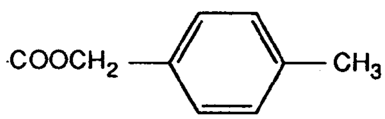 | |
| 15.053 | F | Cl | $-COSCH_3$ | |
| 15.054 | F | Cl | $-COSC_2H_5$ | |
| 15.055 | F | Cl | $-COSC_3H_7$ | |
| 15.056 | F | Cl | $-COS-CH_2-CH=CH_2$ | |
| 15.057 | F | Cl | $-COS-CH_2-COOCH_3$ | |
| 15.058 | F | Cl | $-COS-CH_2-COOC_2H_5$ | |
| 15.059 | F | Cl | $-COS-CH_2-COOC_5H_{11}$ | |
| 15.060 | F | Cl | $-COS-CH-COOCH_3$<br>$\mid$<br>$CH_3$ | |
| 15.061 | F | Cl | $-COS-CH-COOC_2H_5$<br>$\mid$<br>$CH_3$ | |

| Verb. Nr. | $R_1$ | $R_2$ | A | Phys. Daten |
|---|---|---|---|---|
| 15.062 | F | Cl | 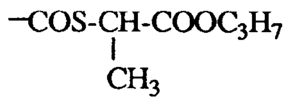 | |
| 15.063 | F | Cl | $-COS-CH_2-CH_2-COOCH_3$ | |
| 15.064 | F | Cl | $-COS-CH_2-COOCH_2-CH_2-O-CH_3$ | |
| 15.065 | F | Cl | $-COOCH_2-COOCH_3$ | |
| 15.066 | F | Cl | 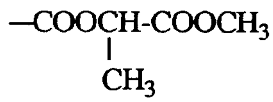 | |
| 15.067 | F | Cl | $-COOCH_2-COOC_5H_{11}$ | |
| 15.068 | F | Cl | $-COOCH_2-CH_2-Si(CH_3)_3$ | |
| 15.069 | F | Cl | -COONa | |
| 15.070 | F | Cl | 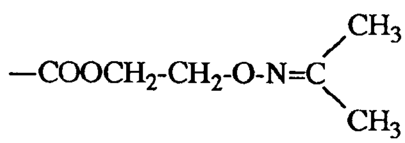 | |
| 15.071 | F | Cl | -OH | |
| 15.072 | F | Cl | $-OCH_3$ | |
| 15.073 | F | Cl | $-OC_2H_5$ | |
| 15.074 | F | Cl | $-OC_3H_7$ | |
| 15.075 | F | Cl | 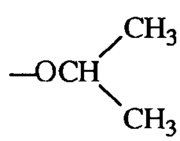 | Harz |
| 15.076 | F | Cl | $-OC_4H_9$ | |
| 15.077 | F | Cl | 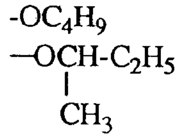 | |
| 15.078 | F | Cl | 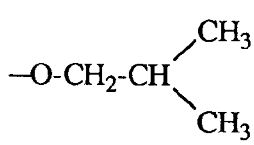 | |
| 15.079 | F | Cl | $-OCH_2CH=CH_2$ | |
| 15.080 | F | Cl | 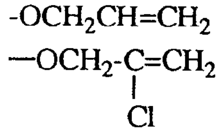 | |

| Verb. Nr. | $R_1$ | $R_2$ | A | Phys. Daten |
|---|---|---|---|---|
| 15.081 | F | Cl | $-OCH_2CH{=}CHCl$ | |
| 15.082 | F | Cl | $-OCH_2C{\equiv}CH$ | |
| 15.083 | F | Cl | $-OCH-C{\equiv}CH$<br>\|<br>$CH_3$ | |
| 15.084 | F | Cl | $-OCH_2-COOCH_3$ | |
| 15.085 | F | Cl | $-O-CH_2-COOC_5H_{11}$ | |
| 15.086 | F | Cl | $-O-CH-COOCH_3$<br>\|<br>$CH_3$ | |
| 15.087 | F | Cl | $-O-CH_2-COOC_2H_5$ | |
| 15.088 | F | Cl | $-O-CH-COOC_2H_5$<br>\|<br>$CH_3$ | |
| 15.089 | F | Cl | $-O-CH_2-CH_2-O-CH_3$ | |
| 15.090 | F | Cl | $-O-CH-CH_2-S-CH_3$<br>\|<br>$CH_3$ | |
| 15.091 | F | Cl | $-O-CH-CH_2-S-C_2H_5$<br>\|<br>$CH_3$ | |
| 15.092 | F | Cl | $-O-CH-CH_2-S-C_3H_7$<br>\|<br>$CH_3$ | |
| 15.093 | F | Cl | $-O-CH_2-CH_2-Cl$ | |
| 15.094 | F | Cl | $-O-CH_2-CN$ | |
| 15.095 | F | Cl | $-O-CH-CN$<br>\|<br>$CH_3$ | |
| 15.096 | F | Cl | $-S-CH_3$ | |
| 15.097 | F | Cl | $-S-C_2H_5$ | |
| 15.098 | F | Cl | $-S-C_3H_7$ | |
| 15.099 | F | Cl | $CH_3$<br>$-S-CH$ ⟨<br>$CH_3$ | |

| Verb. Nr. | $R_1$ | $R_2$ | A | Phys. Daten |
|---|---|---|---|---|
| 15.100 | F | Cl | $-S-CH_2-CH=CH_2$ | |
| 15.101 | F | Cl | $-S-CH_2-C=CH_2$<br>\|<br>Cl | |
| 15.102 | F | Cl | $-S-CH_2-CH=CHCl$ | |
| 15.103 | F | Cl | $-S-CH_2-C\equiv CH$ | |
| 15.104 | F | Cl | $-S-CH-C\equiv CH$<br>\|<br>$CH_3$ | |
| 15.105 | F | Cl | $-S-CH_2-COOCH_3$ | Harz |
| 15.106 | F | Cl | $-S-CH_2-COOC_2H_5$ | |
| 15.107 | F | Cl | $-S-CH_2-COOC_5H_{11}$ | |
| 15.108 | F | Cl | $-S-CH-COOCH_3$<br>\|<br>$CH_3$ | |
| 15.109 | F | Cl | $-S-CH-COOC_2H_5$<br>\|<br>$CH_3$ | |
| 15.110 | F | Cl | $-S-CH_2-COOCH_2-CH_2-O-CH_3$ | |
| 15.111 | F | Cl | 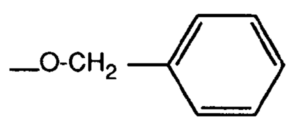 | |
| 15.112 | F | Cl | 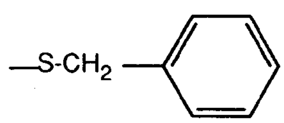 | |
| 15.113 | F | Cl | $-C-CN$<br>\|\|<br>$N-O-CH_3$ | |
| 15.114 | F | Cl | $-C-CN$<br>\|\|<br>$N-O-CH_2-COOCH_3$ | |
| 15.115 | F | Cl | $-C-CN$<br>\|\|<br>$N-O-CH_2-C\equiv CH$ | |

| Verb. Nr. | $R_1$ | $R_2$ | A | Phys. Daten |
|---|---|---|---|---|
| 15.116 | F | Cl | 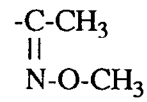 | |
| 15.117 | F | Cl | 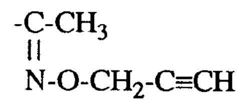 | |
| 15.118 | F | Cl | 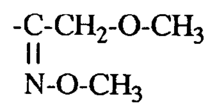 | |
| 15.119 | F | Cl | 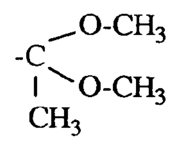 | |
| 15.120 | F | Cl | 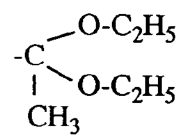 | |
| 15.121 | F | Cl | 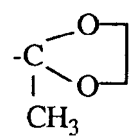 | |
| 15.122 | F | Cl | 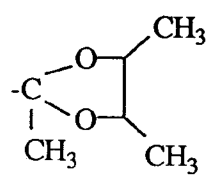 | |
| 15.123 | F | Cl | 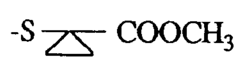 | |
| 15.124 | F | Cl | 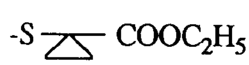 | |
| 15.125 | F | Cl | 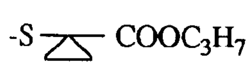 | |
| 15.126 | F | Cl | 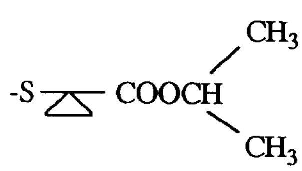 | |

| Verb. Nr. | $R_1$ | $R_2$ | A | Phys. Daten |
|---|---|---|---|---|
| 15.127 | F | Cl | 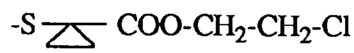 | |
| 15.128 | F | Cl | 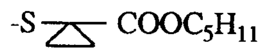 | |
| 15.129 | F | Cl | 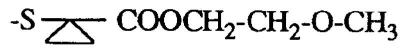 | |
| 15.130 | F | Cl | 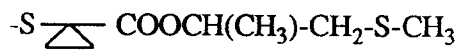 | |
| 15.131 | F | Cl | 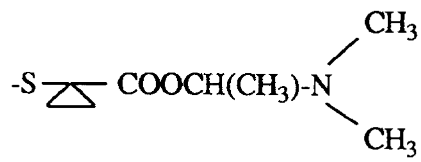 | |
| 15.132 | F | Cl | 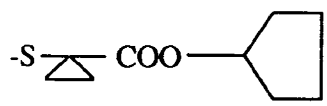 | |
| 15.133 | F | Cl | 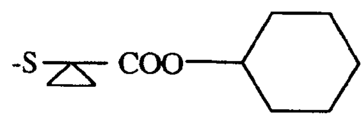 | |
| 15.134 | F | Cl | 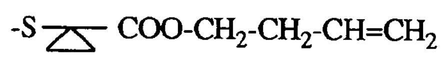 | |
| 15.135 | F | Cl | 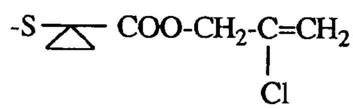 | |
| 15.136 | F | Cl | 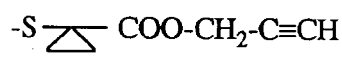 | |
| 15.137 | F | Cl | 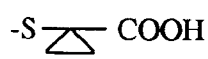 | |
| 15.138 | F | Cl | 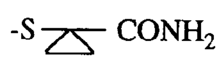 | |
| 15.139 | F | Cl | 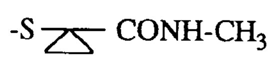 | |
| 15.140 | F | Cl | 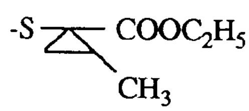 | |
| 15.141 | F | Cl | 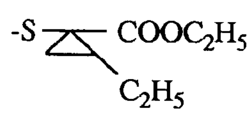 | |

| Verb. Nr. | $R_1$ | $R_2$ | A | Phys. Daten |
|---|---|---|---|---|
| 15.142 | F | Cl | 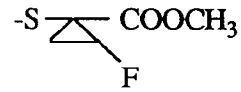 | |
| 15.143 | F | Cl | 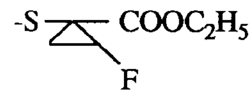 | |
| 15.144 | F | Cl | 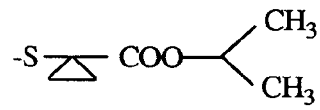 | |
| 15.145 | F | Cl | 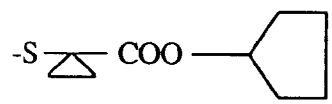 | |
| 15.146 | F | Cl | $-NH-SO_2-CH_3$ | |
| 15.147 | F | Cl | $-NH-SO_2-C_2H_5$ | |
| 15.148 | F | Cl | $-NH-SO_2-Cl$ | |
| 15.149 | F | Cl | 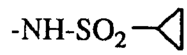 | |
| 15.150 | F | Cl | 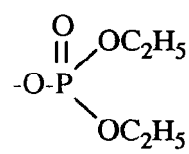 | |
| 15.151 | H | Cl | -COOH | |
| 15.152 | H | Cl | $-COOCH_3$ | Smp. 138-141°C |
| 15.153 | H | Cl | 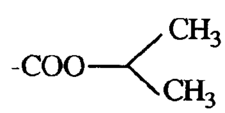 | |
| 15.154 | H | Cl | $-COO-C_5H_{11}$ | |
| 15.155 | H | Cl | $-COO-CH_2-CH_2-O-CH_3$ | |
| 15.156 | H | Cl | $-COOCH_2-S-CH_3$ | |
| 15.157 | H | Cl | $-COOCH(CH_3)-CH_2-S-CH_3$ | |
| 15.158 | H | Cl | 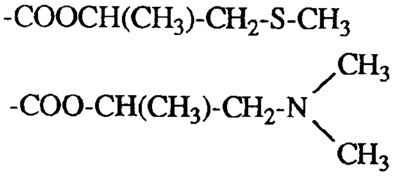 | |

| Verb. Nr. | $R_1$ | $R_2$ | A | Phys. Daten |
|---|---|---|---|---|
| 15.159 | H | Cl | 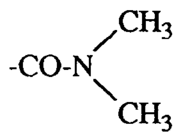 | |
| 15.160 | H | Cl | 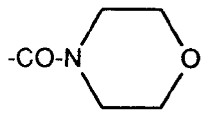 | |
| 15.161 | H | Cl | 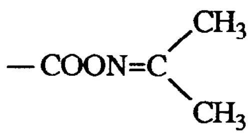 | |
| 15.162 | H | Cl | 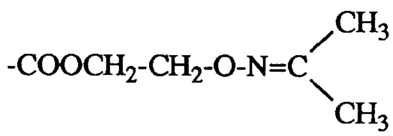 | |
| 15.163 | H | Cl | 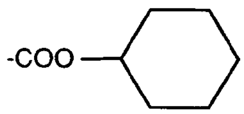 | |
| 15.164 | H | Cl | 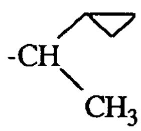 | |
| 15.165 | H | Cl | $-S-C_3H_7$ | |
| 15.166 | H | Cl | $-COOCH_2-COOCH_3$ | |
| 15.167 | H | Cl | $-COOCH(CH_3)-COOCH_3$ | |
| 15.168 | H | Cl | $-COS-CH_2-COOCH_3$ | |
| 15.169 | H | Cl | $-COS-CH(CH_3)-COOCH_3$ | |
| 15.170 | H | Cl | -OH | |
| 15.171 | H | Cl | $-OCH_3$ | |
| 15.172 | H | Cl | $-O-C_2H_5$ | |
| 15.173 | H | Cl | 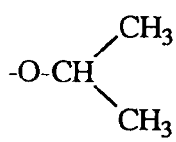 | |
| 15.174 | H | Cl | $-O-CH_2-C\equiv CH$ | |

| Verb. Nr. | $R_1$ | $R_2$ | A | Phys. Daten |
|---|---|---|---|---|
| 15.175 | H | Cl | $-O-CH_2-CH=CHCl$ | |
| 15.176 | H | Cl | 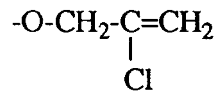 | |
| 15.177 | H | Cl | 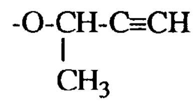 | |
| 15.178 | H | Cl | $-O-CH_2-COOCH_3$ | |
| 15.179 | H | Cl | $-O-CH_2-COOC_2H_5$ | |
| 15.180 | H | Cl | $-O-CH(CH_3)-COOCH_3$ | |
| 15.181 | H | Cl | -SH | |
| 15.182 | H | Cl | $-SCH_3$ | |
| 15.183 | H | Cl | $-SC_2H_5$ | |
| 15.184 | H | Cl | 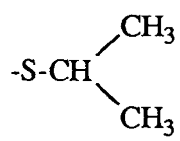 | |
| 15.185 | H | Cl | $-S-CH_2-COOCH_3$ | |
| 15.186 | H | Cl | $-S-CH(CH_3)-COOCH_3$ | |
| 15.187 | H | Cl | $-S-CH_2-COOC_2H_5$ | |
| 15.188 | H | Cl | 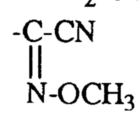 | |
| 15.189 | H | Cl | 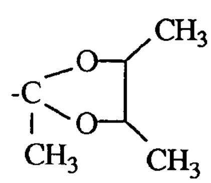 | |
| 15.190 | H | Cl | 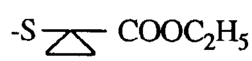 | |
| 15.191 | H | Cl | 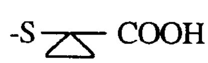 | |
| 15.192 | H | Cl | 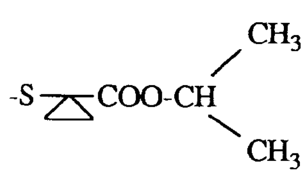 | |

| Verb. Nr. | $R_1$ | $R_2$ | A | Phys. Daten |
|---|---|---|---|---|
| 15.193 | H | Cl | 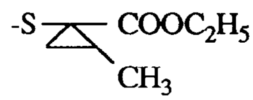 | |
| 15.194 | H | Cl | 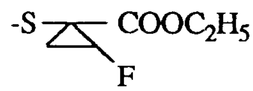 | |
| 15.195 | H | Cl | 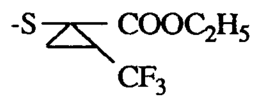 | |
| 15.196 | H | Cl | 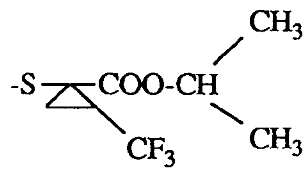 | |
| 15.197 | H | Cl | 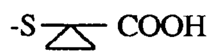 | |
| 15.198 | H | Cl | 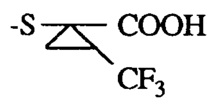 | |
| 15.199 | H | Cl | 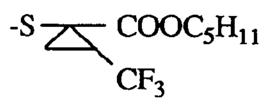 | |
| 15.200 | H | Cl | 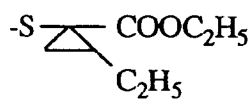 | |
| 15.201 | H | Cl | 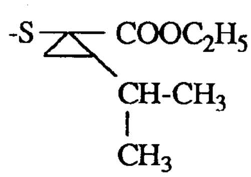 | |
| 15.202 | H | Cl | $-NH-SO_2-C_2H_5$ | |
| 15.203 | H | Cl | $-NH-SO_2-CH_2-Cl$ | |
| 15.204 | F | CN | -COOH | |
| 15.205 | F | CN | 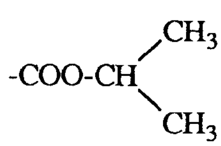 | |

| Verb. Nr. | $R_1$ | $R_2$ | A | Phys. Daten |
|---|---|---|---|---|
| 15.206 | F | CN | 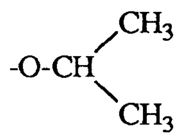 | |
| 15.207 | F | CN | $-O-CH_2-C{\equiv}CH$ | |
| 15.208 | F | CN | $-O-CH(CH_3)-C{\equiv}CH$ | |
| 15.209 | F | CN | $-S-CH_2-COOCH_3$ | |
| 15.210 | F | CN | $-S-CH(CH_3)-COOCH_3$ | |
| 15.211 | F | CN | $-O-CH_2-COOCH_3$ | |
| 15.212 | F | CN | $-O-CH_2-COOC_5H_{11}$ | |
| 15.213 | F | CN | $-O-CH(CH_3)-COOC_2H_5$ | |
| 15.214 | F | CN | 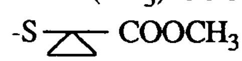 | |
| 15.215 | F | CN | 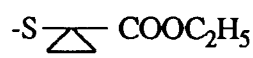 | |
| 15.216 | F | CN | 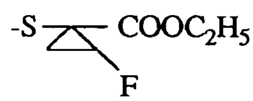 | |
| 15.217 | F | CN | 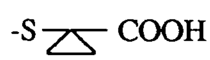 | |
| 15.218 | F | CN | 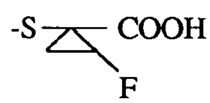 | |
| 15.219 | F | CN | 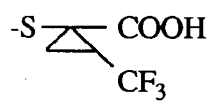 | |
| 15.220 | F | CN | 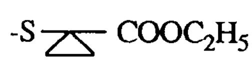 | |
| 15.221 | F | Br | -COOH | |
| 15.222 | F | Br | 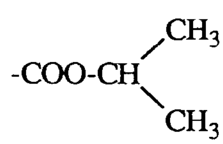 | |
| 15.223 | F | Br | -OH | |

| Verb. Nr. | $R_1$ | $R_2$ | A | Phys. Daten |
|---|---|---|---|---|
| 15.224 | F | Br | 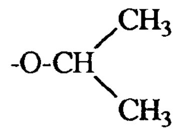 | |
| 15.225 | F | Br | $-O-CH_2-C{\equiv}CH$ | |
| 15.226 | F | Br | $-O-CH(CH_3)-C{\equiv}CH$ | |
| 15.227 | F | Br | $-O-CH_2COOCH_3$ | |
| 15.228 | F | Br | $-O-CH_2-COOC_5H_{11}$ | |
| 15.229 | F | Br | $-S-CH_2-COOCH_3$ | |
| 15.230 | F | Br | 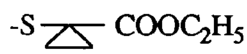 | |
| 15.231 | F | Br | 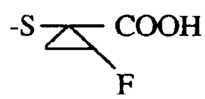 | |
| 15.232 | F | Br | 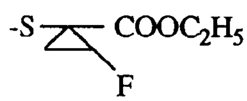 | |
| 15.233 | F | Cl | $-S-CH_2COOH$ | |

B. Formulierungsbeispiele für flüssige Wirkstoffe der Formel I

(% = Gewichtsprozent)

| 1. Emulsions-Konzentrate | a) | b) | c) |
|---|---|---|---|
| Wirkstoff gemäß Tabelle 1-15 | 25 % | 40 % | 50 % |
| Ca-Dodecylbenzolsulfonat | 5 % | 8 % | 6 % |
| Ricinusöl-polyethylenglykolether (36 Mol AeO) | 5 % | - | - |
| Tributylphenol-polyethylenglykolether (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2. Lösungen | a) | b) | c) | d) |
|---|---|---|---|---|
| Wirkstoff gemäß Tabelle 1-15 | 80 % | 10 % | 5 % | 95 % |
| Propylenglykol-monomethylether | 20 % | - | - | - |
| Polyethylenglykol MG400 | - | 70 % | - | - |
| N-Methyl-2-pyrrolidon | - | 20 % | - | - |
| Epoxidiertes Kokosnußöl | - | - | 1 % | 5 % |
| Benzin (Siedegrenzen 160-190°C) | - | - | 94 % | - |

Die Lösungen sind zur Anwendung in Form kleinster Tropfen geeignet.

| 3. Granulate | a) | b) | c) | d) |
|---|---|---|---|---|
| Wirkstoff gemäß Tabelle 1-15 | 5 % | 10 % | 8 % | 21 % |
| Kaolin | 94 % | - | 79 % | 54 % |
| Hochdisperse Kieselsäure | 1 % | - | 13 % | 7 % |
| Attapulgit | - | 90 % | - | 18 % |

Der Wirkstoff wird in Methylenchlorid gelöst, auf den Träger aufgesprüht und das Lösungsmittel anschließend im Vakuum abgedampft.

| 4. Stäubemittel | a) | b) |
|---|---|---|
| Wirkstoff gemäß Tabelle 1-5 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungsbeispiele für feste Wirkstoffe der Formel I

(% = Gewichtsprozent)

| 5. Spritzpulver | a) | b) | c) |
|---|---|---|---|
| Wirkstoff gemäß Tabelle 1-15 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 6. Emulsions-Konzentrat | |
|---|---|
| Wirkstoff gemäß 1-15 | 10 % |
| Octylphenolpolyethylenglykolether (4-5 Mol AeO) | 3 % |
| Ca-Dodecylbenzolsulfonat | 3 % |
| Ricinusölpolyglykolether (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 7. Stäubemittel | a) | b) |
|---|---|---|
| Wirkstoff gemäß Tabelle 1-15 | 5 % | 8 % |
| Talkum | 95 % | - |
| Kaolin | - | 92 % |

Man erhält anwendungsfertige Stäubemittel, indem der Wirkstoff mit den Trägern vermischt und auf einer geeigneten Mühle vermahlen wird.

| 8. Extruder-Granulat | |
|---|---|
| Wirkstoff gemäß Tabelle 1-15 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschließend im Luftstrom getrocknet.

| 9. Umhüllungs-Granulat | |
|---|---|
| Wirkstoff gemäß Tabelle 1-15 | 3 % |
| Polyäthylenglykol (MG200) | 3 % |
| Kaolin | 94 % |

Der fein gemahlene Wirkstoff wird in einem Mischer auf das mit Polyethylenglykol angefeuchtete Kaolin gleichmäßig aufgetragen. Auf diese Weise erhält man staubfreie Umhüllungs-Granulate.

| 10. Suspensions-Konzentrat | |
|---|---|
| Wirkstoff gemäß Tabelle 1-15 | 40 % |
| Propylenglykol | 10 % |
| Nonylphenolpolyethylenglykolether (15 Mol AeO) | 6 % |
| Na-Ligninsulfonat | 10 % |

| | |
|---|---|
| Carboxymethylcellulose | 1 % |
| Silikonöl in Form einer 75%-igen wäßrigen Emulsion | 1 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

## C. Biologische Beispiele:

### Beispiel B 1: Versuchsbeschreibung für die Vorauflauf-Herbizidwirkung (pre-emergent)

Monokotyle und dikotyle Testpflanzen werden in Kunststofftöpfen in Standarderde angesät. Unmittelbar nach der Saat werden die Prüfsubstanzen in wässriger Suspension, hergestellt aus einem 25 %igen Spritzpulver (Formulierungsbeispiel 5), entsprechend der Dosierung von 2 kg AS/ha aufgesprüht (500 l Wasser/ha). Anschliessend werden die Testpflanzen im Gewächshaus unter optimalen Bedingungen kultiviert. Nach 3 Wochen Testdauer wird der Versuch ausgewertet mit einer neunstufigen Notenskala (1 = vollständige Schädigung, 9 = keine Wirkung). Boniturnoten von 1 bis 4 (insbesondere 1 bis 3) bedeuten eine gute bis sehr gute Herbizidwirkung. Das gleiche Resultat wird mit Lösungen (Formulierungsbeispiel 2), dispergierbarem Granulat (Formulierungsbeispiel 3), emulgierbarem Konzentrat (Formulierungsbeispiele 1 und 6) bzw. Suspensionskonzentrat (Formulierungsbeispiel 10), Stäubemittel (Formulierungsbeispiele 4 und 7) und Extruder und Umhüllungs-Granulaten (Formulierungsbeispiele 8 und 9) erhalten.

Testpflanzen: Avena, Setaria, Sinapis, Stellaria

In diesem Versuch zeigen die Verbindungen der Tabellen 1-15 starke Herbizidwirkung.

Beispiele für die gute Herbizidwirkung sind in Tabelle B1 aufgeführt.

Tabelle B1: Pre-emergente Wirkung

| Verb.Nr. | Dosis [kg AS/ha] | Avena | Setaria | Sinapis | Stellaria |
|---|---|---|---|---|---|
| 1.075 | 2 | 3 | 1 | 1 | 1 |
| 3.005 | 2 | 1 | 1 | 1 | 1 |
| 3.075 | 2 | 2 | 1 | 1 | 1 |
| 6.075 | 2 | 4 | 1 | 1 | 5 |
| 8.001 | 2 | 4 | 1 | 1 | 1 |

### Beispiel B2: Versuchsbeschreibung für die Nachauflauf-Herbizidwirkung (post-emergent, Kontaktherbizid)

Monokotyle und dikotyle Testpflanzen werden im Gewächshaus in Kunststofftöpfe mit Standarderde angezogen und im 4- bis 6-Blatt-stadium mit einer wässrigen Suspension, hergestellt aus einem 25 %igen Spritzpulver (Formulierungsbeispiel 5), der Prüfsubstanzen besprüht, entsprechend einer Dosierung von 2 kg AS/ha (500 l Wasser/ha). Anschliessend werden die Testpflanzen im Gewächshaus unter optimalen Bedingungen weiterkultiviert. Nach ca. 18 Tagen Testdauer wird der Versuch ausgewertet mit einer neunstufigen Notenskala (1 = vollständige Schädigung, 9 = keine Wirkung). Boniturnoten von 1 bis 4 (insbesondere 1 bis 3) bedeuten eine gute bis sehr gute Herbizidwirkung. Das gleiche Resultat wird mit Lösungen (Formulierungsbeispiel 2), dispergierbarem Granulat (Formulierungsbeispiel 3), emulgierbarem Konzentrat (Formulierungsbeispiele 1 und 6) bzw. Suspensionskonzentrat (Formulierungsbeispiel 10), Stäubemittel (Formulierungsbeispiele 4 und 7) und Extruder und Umhüllungs-Granulaten (Formulierungsbeispiele 8 und 9) erhalten.

Testpflanzen: Avena, Setaria, Sinapis, Stellaria

Auch in diesem Versuch zeigen die Verbindungen der Formel I gemäß den Beispielen in den Tabellen 1 bis 15 gute Herbizidwirkung.

Beispiele für die gute herbizide Wirksamkeit der Verbindungen der Formel I gibt die Tabelle B2.

Tabelle B2: Post-emergente Wirkung

| Verb.Nr. | Dosis [kg AS/ha] | Avena | Setaria | Sinapis | Stellaria |
|---|---|---|---|---|---|
| 1.075 | 2 | 4 | 2 | 1 | 2 |
| 3.005 | 2 | 2 | 1 | 1 | 1 |
| 3.075 | 2 | 2 | 1 | 1 | 1 |
| 3.105 | 2 | 6 | 2 | 1 | 2 |
| 8.001 | 2 | 2 | 2 | 1 | 1 |

## Patentansprüche

1. Thiadiazabicyclooktane der Formel I

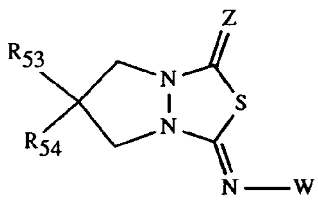

(I),

worin

| | |
|---|---|
| Z | Sauerstoff oder Schwefel; |
| $R_{53}$ | Wasserstoff oder $C_1$-$C_6$-Alkyl; |
| $R_{54}$ | $C_1$-$C_6$-Alkyl, $C_1$-$C_6$-Alkoxy, $C_3$-$C_6$-Alkenyloxy, $C_3$-$C_6$-Alkinyloxy, Hydroxy oder $C_1$-$C_6$-Haloalkoxy ist; oder |
| $R_{53}$ und $R_{54}$ | zusammen mit dem C-Atom, an das sie gebunden sind, einen unsubstituierten 3-, 4-, 5- oder 6-gliedrigen gesättigten Ring bilden; |
| W | eine Gruppe der Formel $W_1$ bis $W_{10}$ |

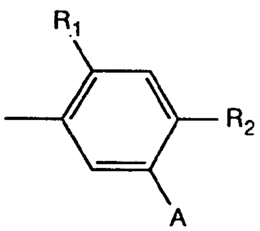

($W_1$);

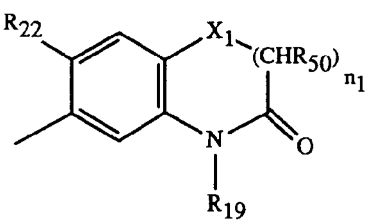

($W_2$);

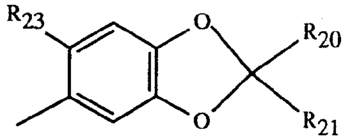 ($W_3$);

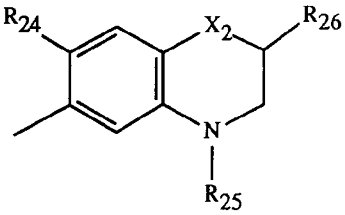 ($W_4$);

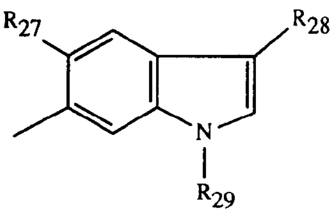 ($W_5$);

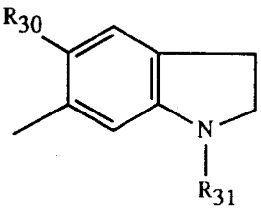 ($W_6$);

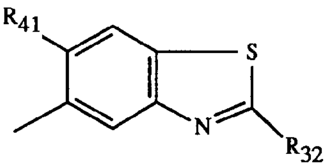 ($W_7$);

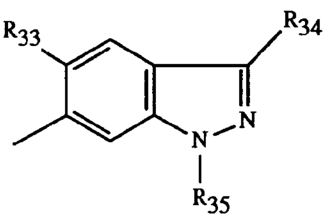 ($W_8$);

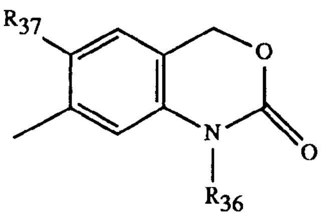 ($W_9$); oder

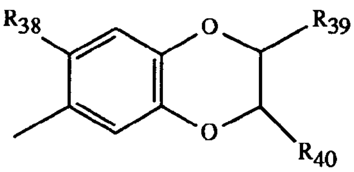 ($W_{10}$)

bedeutet;

$R_1$, $R_{22}$, $R_{23}$, $R_{24}$, $R_{27}$, $R_{30}$, $R_{33}$, $R_{37}$, $R_{38}$ und $R_{41}$ unabhängig voneinander Wasserstoff oder Halogen sind;

$R_2$ Wasserstoff, Cyano, Nitro, Halogen, $C_1$-$C_4$-Alkyl, $C_1$-$C_4$-Haloalkoxy oder $C_1$-$C_4$-Haloalkyl;

A Wasserstoff, Cyano, Nitro, $-COR_3$, $-X_3R_4$,

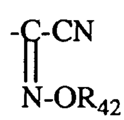 ,

$-COR_8$,

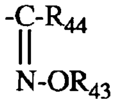 , 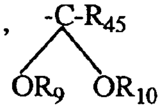 , 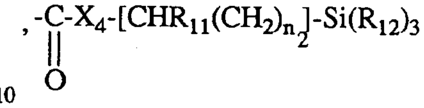 ,

$-N(R_{13})-SO_2-R_{14}$,

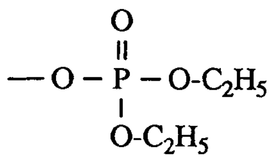 oder 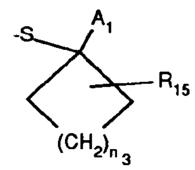;

$A_1$ Cyano oder $-COR_{16}$;

$R_3$ Halogen, $-X_4-R_5$, Amino, $C_1$-$C_4$-Alkylamino, Di-$C_1$-$C_4$-alkylamino, $C_2$-$C_4$-Haloalkylamino, Di-$C_2$-$C_4$-haloalkylamino, $C_1$-$C_4$-Alkoxyalkylamino, Di-$C_1$-$C_4$-alkoxyalkylamino, $C_3$- oder $C_4$-Alkenylamino, Diallylamino, -N-Pyrrolidino, -N-Piperidino, -N-Morpholino, -N-Thiomorpholino, -N-Piperazino, $-O-N=C(CH_3)-CH_3$ oder $-O-CH_2-CH_2-O-N=C(CH_3)-CH_3$;

$R_4$, $R_{42}$ und $R_{43}$ unabhängig voneinander Wasserstoff, $C_1$-$C_{10}$-Alkyl, $C_1$-$C_4$-Alkoxy-$C_1$-$C_4$-alkyl, $C_1$-$C_4$-Alkylthio-$C_1$-$C_4$-alkyl, Di-$C_1$-$C_4$-alkylamino-$C_1$-$C_4$-alkyl, $C_1$-$C_8$-Haloalkyl, $C_2$-$C_8$-Alkenyl, $C_2$-$C_8$-Haloalkenyl, $C_3$-$C_8$-Alkinyl, $C_3$-$C_7$-Cycloalkyl, Halogen-$C_3$-$C_7$-cycloalkyl, $C_1$-$C_8$-Alkylcarbonyl, Allylcarbonyl, $C_3$-$C_7$-Cycloalkylcarbonyl, Benzoyl, das unsubstituiert oder am Phenylring gleich oder verschieden bis zu dreifach durch Halogen, $C_1$-$C_4$-Alkyl, $C_1$-$C_4$-Haloalkyl, $C_1$-$C_4$-Haloalkoxy oder $C_1$-$C_4$-Alkoxy substituiert ist; $C_1$-$C_6$-Alkyl substituiert durch Cyano, Nitro, Carboxyl, $C_1$-$C_8$-Alkylthio-$C_1$-$C_8$-alkoxycarbonyl, Phenyl, Halogenphenyl, $C_1$-$C_4$-Alkylphenyl, $C_1$-$C_4$-Alkoxyphenyl, $C_1$-$C_4$-Haloalkylphenyl, $C_1$-$C_4$-Haloalkoxyphenyl, $C_1$-$C_6$-Alkoxycarbonyl, $C_1$-$C_4$-Alkoxy-$C_1$-$C_8$-alkoxycarbonyl, $C_3$-$C_8$-Alkenyloxycarbonyl, $C_3$-$C_8$-Alkinyloxycarbonyl, $C_1$-$C_8$-Alkylthiocarbonyl, $C_3$-$C_8$-Alkenylthiocarbonyl, $C_3$-$C_8$-Alkinylthiocarbonyl, Carbamoyl, $C_1$-$C_4$-Alkylaminocarbonyl, Di-$C_1$-$C_4$-alkylaminocarbonyl; Phenylaminocarbonyl, das unsubstituiert oder am Phenyl gleich oder verschieden bis zu dreifach durch Halogen, $C_1$-$C_4$-Alkyl, $C_1$-$C_4$-Haloalkyl, $C_1$-$C_4$-Haloalkoxy oder $C_1$-$C_4$-Alkoxy oder einfach durch Cyano oder Nitro substituiert ist; Dioxolan-2-yl, das unsubstituiert oder durch ein oder zwei $C_1$-$C_4$-Alkylreste substituiert ist; oder Dioxanyl, das unsubstituiert oder durch ein oder zwei $C_1$-$C_4$-Alkylreste substituiert ist;

$R_5$ Wasserstoff, $C_1$-$C_{10}$-Alkyl, $C_1$-$C_4$-Alkoxy-$C_1$-$C_4$-alkyl, $C_2$-$C_8$-Haloalkyl, $C_1$-$C_{10}$-Alkylthio-$C_1$-$C_4$-alkyl, Di-$C_1$-$C_4$-alkylamino-$C_1$-$C_4$-alkyl, Cyano-$C_1$-$C_8$-alkyl, $C_3$-$C_8$-Alkenyl, $C_3$-$C_8$-Haloalkenyl, $C_3$-$C_8$-Alkinyl, $C_3$-$C_7$-Cycloalkyl, $C_3$-$C_7$-Cycloalkyl-$C_1$-$C_4$-alkyl, Halogen-$C_3$-$C_7$-cycloalkyl oder Benzyl, das unsubstituiert oder am Phenylring gleich oder verschieden bis zu dreifach durch Halogen, $C_1$-$C_4$-Alkyl, $C_1$-$C_4$-Haloalkyl, $C_1$-$C_4$-Haloalkoxy oder $C_1$-$C_4$-Alkoxy substituiert ist; Alkali-, Erdalkali- oder Ammoniumionen; oder die Gruppe $-[CHR_6(CH_2)_{n_4}]-COOR_7$;

$R_6$, $R_{20}$, $R_{21}$, $R_{26}$, $R_{28}$, $R_{32}$, $R_{34}$, $R_{39}$, $R_{40}$, $R_{46}$, $R_{47}$, $R_{49}$, $R_{50}$, $R_{51}$ und $R_{52}$ unabhängig voneinander Wasserstoff oder $C_1$-$C_4$-Alkyl;

$R_7$ und $R_{48}$ unabhängig voneinander Wasserstoff, $C_1$-$C_6$-Alkyl, $C_3$-$C_8$-Alkenyl, $C_3$-$C_8$-Alkinyl, $C_1$-$C_8$-Alkoxy-$C_2$-$C_8$-alkyl, $C_1$-$C_8$-Alkylthio-$C_1$-$C_8$-alkyl oder $C_3$-$C_7$-Cycloalkyl;

$R_8$ Wasserstoff oder C1-C4-Alkyl;

$R_{44}$ und $R_{45}$ unabhängig voneinander Wasserstoff, $C_1$-$C_4$-Alkyl, $C_1$-$C_4$-Haloalkyl oder $C_1$-$C_4$-Alkoxy-$C_1$-$C_4$-alkyl;

$R_9$ und $R_{10}$ unabhängig voneinander jeweils $C_1$-$C_4$-Alkyl, $C_2$-$C_4$-Haloalkyl oder $C_2$-$C_8$-Alkoxyalkyl; oder

$R_9$ und $R_{10}$ zusammen eine Ethano-, Propano- oder eine Cyclohexan-1,2-diylbrücke, wobei diese Gruppen entweder unsubstituiert oder durch ein bis zwei Reste aus der Gruppe $C_1$-$C_4$-Alkyl, $C_1$-$C_4$-Haloalkyl oder $C_1$-$C_4$-Hydroxyalkyl substituiert sein können;

$R_{11}$ Wasserstoff, $C_1$-$C_5$-Alkyl oder $C_3$-$C_7$-Alkenyl;

$R_{12}$ $C_1$-$C_8$-Alkyl;

| | |
|---|---|
| $R_{13}$ | Wasserstoff, $C_1$-$C_5$-Alkyl, Benzyl, $C_1$-$C_4$-Haloalkyl, $C_3$-$C_8$-Alkenyl oder $C_3$-$C_8$-Alkinyl; |
| $R_{14}$ | $C_1$-$C_6$-Alkyl, $C_1$-$C_5$-Haloalkyl oder Di-$C_1$-$C_4$-alkylamino; |
| $R_{15}$ | Wasserstoff, Fluor, Chlor, Brom, $C_1$-$C_4$-Alkyl oder Trifluormethyl; |
| $R_{16}$ | Chlor, -$X_5$-$R_{17}$, Amino, $C_1$-$C_4$-Alkylamino, Di-$C_1$-$C_4$-alkylamino, $C_2$-$C_4$-Haloalkylamino, Di-$C_2$-$C_4$-haloalkylamino, $C_1$-$C_4$-Alkoxyalkylamino, Di-$C_1$-$C_4$-alkoxyalkylamino, $C_3$-$C_4$-Alkenylamino, Diallylamino, -N-Pyrrolidino, -N-Piperidino, -N-Morpholino, -N-Thiomorpholino, -N-Piperazino, oder die Gruppe -O-N=C($CH_3$)-$CH_3$, -O-$CH_2$-$CH_2$-O-N=C($CH_3$)-$CH_3$ oder -N($OR_{46}$)-$R_6$; |
| $R_{17}$ | Wasserstoff, $C_1$-$C_{10}$-Alkyl, $C_1$-$C_4$-Alkoxy-$C_1$-$C_4$-alkyl, $C_2$-$C_8$-Haloalkyl, $C_1$-$C_{10}$-Alkylthio-$C_1$-$C_4$-alkyl, Di-$C_1$-$C_4$-alkylamino-$C_1$-$C_4$-alkyl, Cyano-$C_1$-$C_8$-alkyl, $C_3$-$C_8$-Alkenyl, $C_3$-$C_8$-Haloalkenyl, $C_3$-$C_8$-Alkinyl, $C_3$-$C_7$-Cycloalkyl, $C_3$-$C_7$-Cycloalkyl-$C_1$-$C_4$-alkyl, Halogen-$C_3$-$C_7$-cycloalkyl oder Benzyl, das unsubstituiert oder am Phenylring gleich oder verschieden bis zu dreifach durch Halogen, $C_1$-$C_4$-Alkyl, $C_1$-$C_4$-Haloalkyl, $C_1$-$C_4$-Haloalkoxy oder $C_1$-$C_4$-Alkoxy substituiert ist; Alkali-, Erdalkali- oder Ammoniumionen, oder die Gruppe -[$CHR_{47}$-$(CH_2)_m$] -$COOR_{48}$ oder -[$CHR_{49}$-$(CH_2)_t$-Si$(R_{18})_3$]; |
| m | 0, 1, 2, 3 oder 4; |
| t | 0, 1, 2, 3 oder 4; |
| $R_{18}$ | $C_1$-$C_4$-Aklkyl; |
| $R_{19}$ | Wasserstoff, $C_1$-$C_6$-Alkyl, $C_2$-$C_4$-Alkenyl, oder $C_2$-$C_6$-Alkinyl; durch Halogen substituiertes $C_1$-$C_6$-Alkyl, $C_2$-$C_4$-Alkenyl, oder $C_3$-$C_6$-Alkinyl; $C_1$-$C_4$-Alkoxy-$C_1$-$C_4$-alkyl, $C_1$-$C_4$-Alkoxy-$C_1$-$C_2$-alkoxy-$C_1$-$C_2$-alkyl, 1-Phenylpropen-3-yl, durch Cyano oder $C_3$-$C_6$-Cycloalkyl substituiertes $C_1$-$C_6$-Alkyl; Carboxy-$C_1$-$C_4$-alkyl, $C_1$-$C_6$-Alkoxycarbonyl-$C_1$-$C_4$-alkyl, $C_2$-$C_6$-Haloalkoxycarbonyl-$C_1$-$C_4$-alkyl, $C_1$-$C_4$-Alkoxy-$C_1$-$C_2$-alkoxycarbonyl-$C_1$-$C_4$-alkyl, $C_1$-$C_6$-Alkoxycarbonyl-$C_1$-$C_2$-alkoxycarbonyl-$C_1$-$C_4$-alkyl, $C_3$-$C_6$-Cycloalkyl-$C_1$-$C_2$-alkoxycarbonyl-$C_1$-$C_4$-alkyl, $C_1$-$C_5$-Alkylaminocarbonyl-$C_1$-$C_4$-alkyl, Di-$C_1$-$C_4$-alkylamino-$C_1$-$C_4$-alkyl, Di-$C_1$-$C_5$-alkylaminocarbonyl-$C_1$-$C_4$-alkyl, $C_3$-$C_6$-Cycloalkyl, $C_1$-$C_4$-Alkylthio-$C_1$-$C_4$-alkyl, Benzyl oder durch Halogen substituiertes Benzyl, $C_1$-$C_4$-Alkylsulfonyl, $C_3$-$C_6$-Alkenyloxy-$C_1$-$C_4$-alkyl, $C_1$-$C_4$-Alkoxycarbonyl, $C_1$-$C_8$-Alkylcarbonyl, |

$C_1$-$C_4$-Alkyl-COO 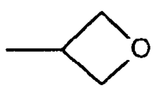 , $C_1$-$C_4$-Alkyl-COO 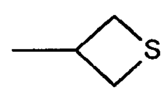 , 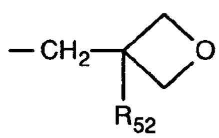 ,

| | |
|---|---|
| | $C_1$-$C_4$-Alkylthiocarbonyl-$C_1$-$C_4$-alkyl, oder die Gruppe -[$CHR_{47}$-$(CH_2)_m$]$COX_6$-$CHR_{47}$-$(CH_2)_m$-$COOR_{48}$ ; |
| $R_{25}$, $R_{29}$, $R_{31}$, $R_{35}$ und $R_{36}$ | unabhängig voneiander Wasserstoff, $C_1$-$C_4$-Alkyl, $C_1$-$C_6$-Haloalkyl, $C_3$-$C_8$-Alkenyl, $C_3$-$C_8$-Haloalkenyl, $C_3$-$C_8$-Alkinyl, $C_1$-$C_4$-Alkoxy-$C_1$-$C_8$-alkyl, Cyano-$C_1$-$C_4$-alkyl, $C_1$-$C_8$-Alkoxycarbonyl-$C_1$-$C_4$-alkyl, $C_3$-$C_7$-Cycloalkyl, $C_3$-$C_7$-Cycloalkyl-$C_1$-$C_4$-alkyl, Benzyl, durch -N-Morpholino, -N-Thiomorpholino oder -N-Piperazino substituiertes $C_1$-$C_4$-Alkyl, Di-$C_1$-$C_4$-alkylamino-$C_1$-$C_4$-alkyl, $C_1$-$C_4$-Alkylaminocarbonyl-$C_1$-$C_4$-alkyl, Di-$C_1$-$C_4$-alkylaminocarbonyl-$C_1$-$C_4$-alkyl, $C_1$-$C_4$-Alkoxycarbonyl oder $C_1$-$C_4$-Alkylcarbonyl; |
| $X_1$, $X_2$, $X_3$, $X_4$, $X_5$ und $X_6$ | unabhängig voneinander Sauerstoff oder Schwefel; |
| $n_1$, $n_2$, $n_3$ und $n_4$ | unabhängig voneinander 0, 1, 2, 3 oder 4, bedeuten; mit den Massgaben, dass mindestens einer der Substituenten $R_1$, $R_2$ oder A verschieden von Wasserstoff ist; und dass wenn $R_{63}$ Wasserstoff ist, $R_{54}$ verschieden von $C_1$-$C_4$-Alkyl ist; und ihre Salze und Stereoisomere. |

**2.** Verbindungen gemäß Anspruch 1, worin Z Sauerstoff bedeutet.

3. Verbindungen gemäß Anspruch 1 der Formel Ia

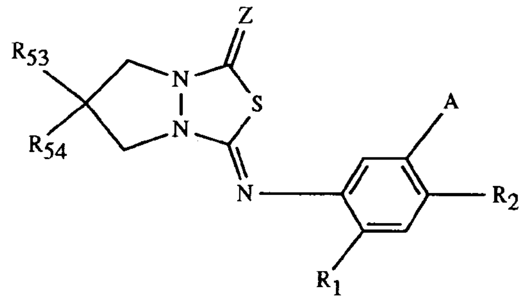

(Ia),

worin Z, A, $R_1$, $R_2$, $R_{53}$ und $R_{54}$ die in Anspruch 1 angegebene Bedeutung haben.

4. Verbindungen gemäß Anspruch 3, worin A $-X_3R_4$, $-COR_5$, $-COR_3$,

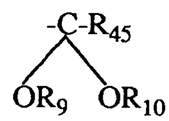

,

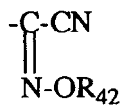

,

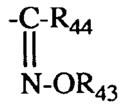

oder $-N(R_{13})-SO_2-R_{14}$ bedeutet.

5. Verbindungen gemäss Anspruch 4, worin $X_3$ Schwefel und $R_4$ durch $C_1$-$C_6$-Alkoxycarbonyl substituiertes $C_1$-$C_6$-Alkyl bedeutet.

6. Verbindungen gemäß Anspruch 3, worin $R_1$ und $R_2$ Halogen bedeuten.

7. Verbindungen gemäß Anspruch 3, worin $R_1$ Fluor und $R_2$ Chlor bedeuten.

8. Verbindungen gemäß Anspruch 1 der Formel Ib

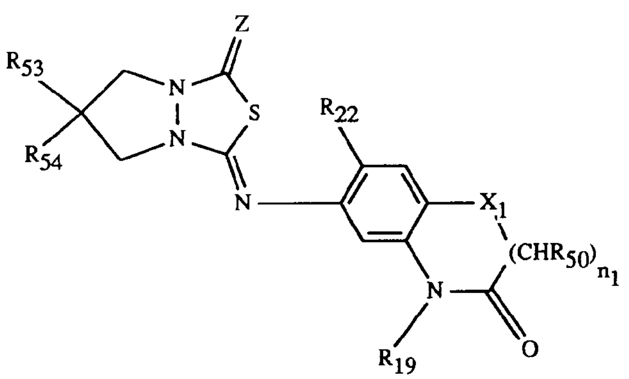

(Ib),

worin Z, $R_{19}$, $R_{22}$, $X_1$, $R_{50}$, $R_{53}$, $R_{54}$ und $n_1$ die in Anspruch 1 angegebene Bedeutung haben.

9. Verbindungen gemäß Anspruch 8, worin $R_{19}$ $C_1$-$C_4$-Alkyl, $C_3$- oder $C_4$-Alkenyl, $C_3$- oder $C_4$-Haloalkenyl, $C_3$-$C_6$-Alkinyl, $C_1$-$C_4$-Alkoxy-$C_1$-$C_4$-alkyl, Cyano-$C_1$-$C_4$-alkyl, $C_1$-$C_6$-Alkoxycarbonyl-$C_1$-$C_4$-alkyl, $C_3$-$C_6$-Cycloalkyl, $C_3$-$C_6$-Cycloalkyl-$C_1$-$C_4$-alkyl, Benzyl, Di-$C_1$-$C_4$-alkylamino-$C_1$-$C_4$-alkyl, $C_1$-$C_4$-Alkylaminocarbonyl-$C_1$-$C_4$-alkyl, Di-$C_1$-$C_4$-alkylamino-carbonyl-$C_1$-$C_4$-alkyl, $C_1$-$C_4$-Alkoxycarbonyl oder $C_1$-$C_4$-Alkylcarbonyl bedeutet.

**10.** Verbindungen gemäss Anspruch 9, worin Z Sauerstoff; $R_{19}$ $C_1$-$C_3$-Alkyl, $C_1$-$C_3$-Alkoxycarbonyl-$C_1$- oder -$C_2$-alkyl oder $C_3$- oder $C_4$-Alkinyl; $R_{22}$ Wasserstoff oder Fluor; $R_{50}$ Wasserstoff; und $n_1$ 0 oder 1 ist.

**11.** Verbindungen gemäss Anspruch 1, worin $R_{53}$ und $R_{54}$ $C_1$-$C_4$-Alkyl bedeuten.

**12.** Verbindungen gemäss Anspruch 11, worin $R_{53}$ und $R_{54}$ unabhängig voneinander Methyl oder Ethyl bedeuten.

**13.** Verbindungen gemäss Anspruch 1, worin $R_{53}$ und $R_{54}$ zusammen mit dem C-Atom, an das sie gebunden sind, einen unsubstituierten 3- oder 5-gliedrigen gesättigten Ring bilden.

**14.** Verbindungen gemäss Anspruch 1, worin $R_{53}$ Wasserstoff; und $R_{54}$ $C_1$-$C_3$-Alkoxy, $C_3$-$C_5$-Alkenyloxy, $C_3$-$C_5$-Alkinyloxy oder $C_1$-$C_3$-Haloalkoxy bedeuten.

**15.** Verbindungen gemäss Anspruch 14, worin $R_{54}$ $C_1$-$C_3$-Alkoxy oder $C_1$-$C_3$-Haloalkoxy bedeutet.

**16.** Verbindungen gemäss Anspruch 15, worin $R_{54}$ Methoxy, iso-Propoxy oder Difluormethoxy ist.

**17.** Eine Verbindung gemäss Anspruch 1 ausgewählt aus der Gruppe
8-(4-Chlor-2-fluor-5-isopropoxy-phenylimino)-3,3-dimethyl-7-thia-1,5-diazabicyclo[3.3.0]oktan-6-on;
8-(4-Chlor-2-fluor-5-isopropoxy-phenylimino)-3-methoxy-7-thia-1,5-diazabicyclo[3.3.0]oktan-6-on;
8-(4-Chlor-2-fluor-5-isopropoxy-phenylimino)-3-difluormethoxy-7-thia-1,5-diazabicyclo[3.3.0]oktan-6-on;
8-(4-Chlor-2-fluor-5-isopropoxy-phenylimino)-3,3-ethandiyl-7-thia-1,5-diazabicyclo[3.3.0]oktan-6-on.

**18.** Verfahren zur Herstellung der Verbindungen der Formel I,

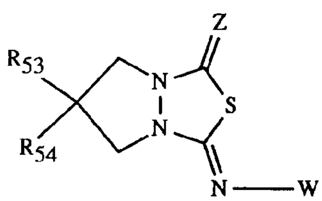

(I),

worin Z, W, $R_{53}$ und $R_{54}$ die unter Formel I in Anspruch 1 angegebene Bedeutung haben, dadurch gekennzeichnet, daß man ein Isothiocyanat der Formel II

S=C=N-W (II),

worin W die unter Formel I in Anspruch 1 angegebene Bedeutung hat, mit einer Verbindung der Formel III

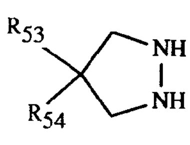

(III),

worin $R_{53}$ und $R_{54}$ die unter Formel I in Anspruch 1 angegebene Bedeutung haben, in die Verbindung der Formel IV

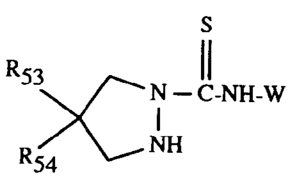

(IV)

überführt und diese anschließend mit einer Verbindung der Formel V

$CZCl_2$ (V),

worin Z für Sauerstoff oder Schwefel steht, umsetzt.

**19.** Herbizides und den Pflanzenwuchs hemmendes Mittel, dadurch gekennzeichnet, dass es eine Verbindung der Formel I oder deren Salze gemäss Anspruch 1 enthält.

**20.** Mittel gemäß Anspruch 19, dadurch gekennzeichnet, daß es 0,1 bis 95 Gewichtsprozent Wirkstoff der Formel I gemäß Anspruch 1 enthält.

**21.** Verfahren zur Bekämpfung unerwünschten Pflanzenwachstums, dadurch gekennzeichnet, daß man die gegen Unkräuter zu schützenden Kulturpflanzen und /oder die Unkräuter und Gräser mit einem Wirkstoff der Formel I gemäss Anspruch 1 oder mit einem diesen Wirkstoff enthaltenden Mittel gemäss Anspruch 19 behandelt.

**22.** Verfahren gemäß Anspruch 21, dadurch gekennzeichnet, daß man eine Verbindung der Formel I in einer Menge von 0,001 bis 2 kg pro Hektar appliziert.

**23.** Verfahren gemäß Anspruch 21, zur selektiven pre- oder post-emergenten Bekämpfung von Unkräutern und Gräser in Nutzpflanzenkulturen.

**24.** Verfahren zur Hemmung des Pflanzenwachstums, dadurch gekennzeichnet, daß man einen Wirkstoff der Formel I, gemäß Anspruch 1, oder ein diesen Wirkstoff enthaltendes Mittel in einer wirksamen Menge auf die Pflanzen oder deren Lebensraum appliziert.

**25.** Verwendung eines Mittels gemäss Anspruch 19 zur selektiven pre- oder post-emergenten Bekämpfung von Unkräutern und Gräser in Nutzpflanzenkulturen.

Europäisches Patentamt

# EUROPÄISCHER RECHERCHENBERICHT

Nummer der Anmeldung
EP 93 81 0803

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| X | PHARMAZIE.<br>Bd. 46, Nr. 8 , 1991 , BERLIN DD<br>Seiten 573 - 575<br>A. KLEMANN ET AL 'N,N-Verknüpfte Heterobicyclen aus cyclischen Hydrazin-Derivaten'<br>* Verbindung 6 *<br>--- | 1 | C07D513/04<br>A01N43/90<br>//C07D513/04, 285:00,231:00 |
| D,A | EP-A-0 238 711 (KUMIAI CHEMICAL INDUSTRY)<br>* Ansprüche 1,9 *<br>--- | 1,19 | |
| P,X | WO-A-92 21684 (CIBA-GEIGY)<br>* Ansprüche 1,24 *<br>--- | 1,19 | |
| L | CHEMICAL ABSTRACTS, vol. 120, no. 1, 3. Januar 1994, Columbus, Ohio, US;<br>abstract no. 2802h,<br>M. YAMAGUCHI ET AL 'Preparation of condensed heterocyclic derivatives useful as herbicides'<br>Seite 2802 ;<br>* Zusammenfassung * | 1,19 | **RECHERCHIERTE SACHGEBIETE (Int.Cl.5)** |
| P,X | & JP-A-05 213 970 (KUMIAI CHEMICAL INDUSTRY) 24. August 1993<br>----- | 1,19 | C07D<br>A01N |

Der vorliegende Recherchenbericht wurde für alle Patentansprüche erstellt

| Recherchenort | Abschlußdatum der Recherche | Prüfer |
|---|---|---|
| DEN HAAG | 1. März 1994 | Voyiazoglou, D |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C01)